# EUROPEAN PATENT SPECIFICATION

(11) **EP 2 658 570 B1**
(45) Date of publication and mention of the grant of the patent: **28.11.2018**
(21) Application number: 11813490.7
(22) Date of filing: 21.12.2011
(51) Int. Cl.: A61K 39/12, A61P 31/14

(54) **BOVINE VIRAL DIARRHEA VIRUS TYPE 1B VACCINE COMPOSITIONS AND METHODS**
IMPFSTOFFZUSAMMENSETZUNG GEGEN VIRUS DER VIRALEN RINDERDIARRHOE UND VERWENDUNG
COMPOSITION VACCINALE CONTRE LE VIRUS DE LA DIARRHÉE VIRALE DES BOVINS TYPE 1B ET UTILISATION

(30) Priority: 27.12.2010 US 201061427361 P
(43) Date of publication of application: 06.11.2013
(73) Proprietor: Elanco US Inc., Greenfield, IN 46140 (US)
(72) Inventor: WEISE, Dale, Wade, Indianapolis, Indiana 46206-6288 (US); HARRIS, James, Robert, Indianapolis, Indiana 46206-6288 (US)
(74) Representative: Bassinder, Emma Marie
(86) International application number: PCT/US2011/066384
(87) International publication number: WO 2012/092053

(56) References cited:
- WO-A1-2005/089793
- WO-A2-2007/094854
- WO-A2-2007/117303
- FULTON R W ET AL: "Bovine viral diarrhea virus (BVDV) 1b: Predominant BVDV subtype in calves with respiratory disease", CANADIAN JOURNAL OF VETERINARY RESEARCH, CANADIAN VETERINARY MEDICAL ASSOCIATION, OTTAWA, CA, vol. 66, no. 3, 1 July 2002 (2002-07-01), pages 181-190, XP009146463, ISSN: 0830-9000
- MISHRA N ET AL: "Genetic analysis of Indian bovine viral diarrhea virus 1 isolates in N-pro and entire gene region coding structural proteins", ACTA VIROLOGICA, vol. 50, no. 1, 2006, pages 39-44, XP009158602, ISSN: 0001-723X
- VANDERHEIJDEN NATHALIE ET AL: "Expression of the bovine viral diarrhoea virus Osloss p80 protein: Its use as ELISA antigen for cattle serum antibody detection", JOURNAL OF GENERAL VIROLOGY, vol. 74, no. 7, 1993, pages 1427-1431, XP002674353, ISSN: 0022-1317

## Description

### FIELD OF THE INVENTION

The present invention relates generally to the fields of veterinary medicine and animal vaccines. More particularly, it concerns immunogenic compositions, methods of making such compositions, and methods for preventing, treating, ameliorating, and/or managing microbial or viral infection, or a symptom thereof, and particularly respiratory infections in mammalian livestock, including multifactorial diseases such as bovine respiratory disease complex (BRDC). Disclosed are antigenic compositions and methods for their use in the formulation and administration of prophylactic and therapeutic agents for preventing, treating, and/or ameliorating one or more symptoms of pestivirus infection, and particularly those pestiviruses responsible for, or implicated in, shipping fever, BRDC, and bovine viral diarrhea in susceptible or infected animals.

### DESCRIPTION OF RELATED ART

BRDC is a multifactorial disease complex that frequently afflicts stocker/feeder calves in market channels and at destination pasture or feedlot. The primary bacterial etiologic agents of this disease are *Mannheimia haemolytica*, *Pasteurella multocida*, *Histophilus somni* (formerly *Haemophilus somnus*) and *Mycoplasma bovis.* The viral agents that have been associated with shipping fever are Bovine Herpesvirus Type 1 (BHV-1), Parainfluenza 3 (PI₃), Bovine Respiratory Syncytial Virus (BRSV) and Bovine Viral Diarrhea Virus (BVDV).

### BOVINE VIRAL DIARRHEA VIRUS (BVDV)

BVDV is now recognized as an important etiologic agent in the Bovine Respiratory Disease Complex (BRDC) (often commonly referred to as "shipping fever."). The disease, which infects cattle of all ages (including nursing calves), is characterized by rapid breathing, coughing, depression, loss of appetite, ocular and nasal discharge, and elevated temperatures. In an acute outbreak, death may follow within 24 hours of onset of symptoms. Treatment of BVDV is problematic given the absence of antiviral therapeutics, and the mortality rate from pestivirus infection is high. Moreover, the treatment of bacterial pathogens involved in BRDC is also complicated in many instances by antimicrobial resistance to therapeutic drugs.

Bovine Viral Diarrhea viruses are a disparate group of viruses that can be classified both phenotypically (*see*, *e*.*g*., Baker, 1995) (cytopathic or noncytopathic) and genotypically (*see*, *e*.*g*., Ridpath *et al*., 1994; Vilcek *et al.,* 2001; and Flores *et al*., 2002). Establishing protective immunity against BVDV in livestock has been problematic for a number of reasons. As in some other virally-mediated diseases, the levels of serum antibodies against BVDV do not necessarily correlate with protection against disease. Establishing protective immunity in nursing calves presents additional obstacles, since maternal antibodies to BVDV may deplete the injected immunogen and effectively neutralize the vaccine.

A study by Fulton *et al*. (2006) evaluated 21,743 calves entering U.S. feedlots and determined that 88 calves were persistently infected (PI) with BVDV. Of the 88 PI calves, 77.9% were infected with BVDV-lb; only 11.6% were infected with BVDV-la, and only 10.5% were infected with BVDV-2a. Unfortunately, while these data *clearly* demonstrated that BVDV-lb is the predominate subtype of PI cattle entering domestic feedlots, there are currently no USDA-licensed vaccines that are specific for BVDV-1b infection. Fulton et al. (Can J Vet Res: Vol 66, 181-109; 2002) also disclosed that BVDV1b is the predominant BVDV subtype in calves with respiratory disease.

Thus, for these and other reasons inherent in the prior art, there is a need for BVDV vaccine formulations that elicit a vigorous and multi-faceted immune response, and particularly those that elicit an immune response against the Type 1b form of BVDV.

Because of the potential for great economic loss in unvaccinated herds, there is a need for cost-effective (and preferably, single-dose) vaccines containing modified, live BVDV-lb viruses that induce prophylaxis against BVDV infections in mammalian livestock populations.

Likewise, because contemporary polyvalent vaccines against multifactorial diseases such as BRDC and related shipping fever diseases are largely ineffective at preventing infection by BVDV-1b, there is also a need for improved vaccination modalities to better effect prophylaxis and control of disease outbreak in BVDV-1b-susceptible livestock.

### BRIEF SUMMARY OF THE INVENTION

The present invention encompasses new and useful compositions, as well as methods of employing them that may advantageously improve delivery of prophylactic and/or therapeutic agents to an animal in need thereof. The invention provides vaccine compositions that offer advantages over conventional formulations, and specifically provide ways of preventing or controlling diseases caused by one or more pestiviruses, and in particular, those caused by BVDV viruses including those of the Type 1b subgenotype (*i*.*e*., BVDV-1b). The scope of the invention is according to the appended claims.

In an overall and general sense, the present invention encompasses compositions, and methods for their use in the prevention, treatment, and/or management, of viral and/or microbial infection, pathogenesis, and disease. The immunogenic compositions disclosed herein, as well as methods employing them, find particular use in the prevention, treatment, and/or amelioration of one or more symptoms of shipping fever/BRDC in susceptible mammals using the inventive vaccine compositions and methods employing them that are superior to conventional shipping fever preventatives/treatments currently available in the veterinary medical arts.

In particular embodiments, the present invention provides immunogenic compositions comprising modified, live BVDV-1b viruses, as well as pharmaceutical formulations containing them, and methods for using such vaccines in a variety of prophylactic regimens. Such compositions and methods find particular use in the prevention of viral and multifactorial diseases in animals such as mammalian livestock that are susceptible to BVDV infection.

In related embodiments, the invention also provides methods for using the disclosed vaccine compositions for eliciting a viral-specific immunological response in an animal.

In an overall and general sense, such methods generally involve providing to a selected animal one or more of the immunogenic compositions disclosed herein in amount(s) and for a time effective to elicit a viral-specific immunological response in an animal, and in particular, a BVDV-specific immunological response. In such embodiments, the animal is preferably a cloven-hoofed mammal, and more preferably, a member of the family *Bovidae.*

The invention also provides a method of preventing or controlling an outbreak of viral and/or microbial infection(s) (and pestiviral infections caused by BVDV and related species in particular) in one or more selected mammalian populations. The method generally involves providing to a susceptible or an at-risk member of such a population an effective amount of one or more of the disclosed immunogenic or vaccine compositions, for a time sufficient to delay, lessen, reduce, inhibit, and/or prevent the outbreak of such an infection in the general population. Exemplary mammalian populations include, without limitation, members of the *Bovinae* and *Caprinae*, and particularly those of the genera *Bison*, *Bos*, *Bubalus*, *Capra*, *Oreamnos*, *Ovibos*, *Ovis*, *Syncerus*, and such like. In illustrative embodiments, these methods are particularly desirable in the treatment of commercial livestock in the genera Bison, Bos, Capra, and Ovis, including, without limitation, *Bison bison*, *Bos taurus*, *Capra hircus* and *Ovis aries.*

In another aspect, the invention provides a method for stimulating the immune system of an animal to produce a protective immune response against a viral infection, and in particular, a *Bovidae*-virulent pestivirus infection. Such a method generally involves at least administering to an animal in need thereof, an immunologically- and prophylactically-effective amount of one or more of the disclosed vaccine compositions in an amount and for a time sufficient to stimulate the immune system of the animal. In the conventional practice of this method in one embodiment, administration of one or more of the disclosed immunologically- and prophylactically-active vaccine compositions preferably induces at least a first detectable amount of anti-pestivirus antibodies in the animal, and more preferably still, induces at least a first detectable amount of anti-BVDV-1b antibodies in the animal. In preferred embodiments, administration of the composition preferably immunizes the animal against initial, subsequent, and/or recurrent infection by BVDV-1b virus, and in certain embodiments, also preferably immunizes the animal against initial, subsequent, and/or recurrent infection by one or more genetically-similar or epitopically-related viruses, including for example, one or more types, strains, and subtypes of BVDV virus (including, without limitation, BVDV-1a, BVDV-2, and other genetically-similar pestiviruses.

In yet another embodiment, the invention provides a method for producing a protective immune response against pestivirus in an animal. Such a method generally includes providing to an animal in need thereof an immunologically- and prophylactically-effective amount of one or more of the disclosed BVDV-1b immunogenic compositions under conditions and for a time sufficient to produce such a protective immune response against one or more pestivirus species, subspecies, or strains, and preferably, against one or more BVDV species, strains, subtypes, or serotypes.

Likewise, the invention also discloses a method of providing a prophylactic or therapeutic composition to a first cell in a mammalian host. This method generally involves providing to a mammal in need thereof a prophylactically- or therapeutically-effective amount of one or more of the BVDV-specific immunogenic compositions disclosed herein, under conditions and for a time effective to provide the composition to at least a first cell, tissue, organ, or organ system in such a mammal.

The present invention also discloses a method for preventing, treating, and/or ameliorating one or more symptoms of BVDV infection in a mammal. Such a method generally includes providing to the mammal in need thereof an immunologically- and prophylactically-effective amount of one or more of the disclosed BVDV-lb immunogenic compositions under conditions and for a time sufficient to prevent, treat, and/or ameliorate one or more symptoms of the BVDV infection in the mammal.

Likewise, the present invention also discloses methods for preventing, treating, and/or ameliorating one or more symptoms of a multifactorial disease such as BRDC in a mammal, and particularly in those diseases in which infection by at least a first BVDV is implicated as a causal or contributory agent of the disease. Such methods generally involve administering to a selected mammal at least a first immunologically- or prophylactically-effective amount of at least a first anti-BVDV-lb vaccine composition under conditions and for a time sufficient to prevent, treat, and/or ameliorate one or more symptoms of the disease in the mammal.

In yet another aspect, the invention provides a method for preventing, treating, and/or ameliorating one or more symptoms of shipping fever in a member of the family *Bovidae.* Such a method generally includes providing to the bovid at least a first immunologically- or prophylactically-effective amount of a first composition comprising a population of modified, live BVDV-1b viruses under conditions and for a time sufficient to prevent, treat, and/or ameliorate one or more symptoms of shipping fever in the bovid.

### IMMUNOGENIC COMPOSITIONS

In order to provide broader protection against one or more pestiviruses, and in particular, one or more of the viral agents implicated in BRDC and shipping fever in members of the family *Bovidae*, the inventors have developed safe and effective vaccine formulations that include one or more BVDV-1b-specific antigens. Both monovalent and polyvalent vaccine formulations have been described, including those that contain, in the case of monovalent vaccines, one or more antigens, epitopes, or modified live viruses that elicit an immune response specific for BVDV alone, or, in cases of polyvalent vaccines, one or more antigens, epitopes, or modified live viruses that elicit specific immune responses not only for BVDV, but also for one or more additional pathogens.

In illustrative embodiments, a monovalent immunogenic composition comprising modified, live BVDV-1b has been developed to protect livestock from BVDV-1b infection. Additionally, multivalent immunogenic compositions specific for BRDC and related shipping fevers have also been provided by the present invention, including an exemplary six-way (*i.e*., "hexavalent") modified, live bovine viral formulation that includes antigenic components for specifically inducing an immune response against one or more, preferably two or more, and more preferably three or more of BHV-1, PI₃, BRSV, two subgenotypes of BVDV Type 1 (1a and 1b), and BVDV Type 2 in an animal. In a more preferred embodiment, the formulation induces an immune response against each of the foregoing, typically to varying levels.

In further embodiments, the invention provides vaccine formulations containing the aforementioned immunogens, and methods for their use in the prophylaxis, therapy, and/or amelioration of one or more symptoms of a pestivirus infection in an animal, and in particular, a BVDV-1b infection in mammalian livestock. The invention further encompasses vaccine formulations and methods employing them in the prevention, treatment, and/or amelioration of one or more symptoms of a shipping fever, such as BRDC, in bovids and other related mammalian species.

### VACCINE FORMULATIONS

The present invention provides immunogenic compositions and vaccine formulations thereof for use in the prophylaxis of one or more viral infections, as well as compositions for use in the prevention or therapy of one or more multifactorial disease complexes in a selected mammal. In particular, the invention provides for the use of one or more of the disclosed immunogenic compositions in the manufacture of vaccine medicaments for prophylaxis, prevention, or therapy, and particularly for use in the manufacture of veterinary-acceptable medicaments for preventing, managing, ameliorating, and/or treating one or more diseases, or one or more symptoms of such diseases, in mammals such as BVDV infections in livestock.

The vaccines of the present invention can be administered by any suitable route of administration for the selected mammal available to one of ordinary skill in the art, preferably by intramuscular or subcutaneous injection or *via* intranasal, oral, cutaneous, percutaneous or intracutaneous administration. Preferably, for vaccines against BVDV-1b, vaccinations are administered subcutaneously, or by intramuscular or intranasal routes, with subcutaneous delivery being most preferred. The vaccines are typically administered prior to weaning, at wearing, or at the time of entry to pasture or feedlot. As part of an ongoing adult cattle operation, one or more "booster" doses of such vaccines may also be routinely employed, including for example as part of an annual re-vaccination/maintenance schedule.

Although the pharmaceutical formulations and vaccines of the present invention may be prepared in any suitable, veterinary-acceptable formulation, in particular embodiments, the invention provides vaccines that are in the form of a ready-to-administer solution or suspension, in a concentrated stock solution suitable for dilution prior to administration, or in a reconstitutable form such as a lyophilized, freeze-dried, or frozen preparation, as is known to those of ordinary skill in the veterinary medical arts.

Both the preparation of the modified, live pestivirus compositions, and the formulation of the inventive compositions and vaccines with other immunogens (with or without one or more adjuvants), are conventional based upon the guidance herein, and include the mixing of the live, attenuated pestivirus with a pharmaceutically acceptable carrier or diluent, optionally with other immunogens and optionally with an adjuvant.

Carriers, diluents, or other inert or inactive components of the pharmaceutical formulations and vaccines may comprise one or more stabilizers, preservatives and/or buffers, as may be employed in the veterinary medical arts. Exemplary stabilizers include, without limitation, SPGA, and one or more of the following: carbohydrates (including, but not limited to, sorbitol, mannitol, starch, sucrose, dextran, glutamate or glucose), proteins (including, but not limited to, dried milk, serum albumin, casein, or proteins from other sources such as from plants or microorganisms), or such like. Suitable buffers include, without limitation, one or more alkali metal phosphates. Exemplary preservatives useful in formulation of the disclosed pharmaceutical compositions and vaccines include, without limitation, thimerosal, merthiolate, gentamicin, neomycin, nystatin, amphotericin B, tetracycline, penicillin, streptomycin, polymyxin B, and any combination thereof. Exemplary diluents include, without limitation, sterile water, one or more aqueous buffers (such as buffered saline and the like), one or more alcohols (including a polyol, *e*.*g*., glycerol or the like), and any combinations thereof.

Where desired, the vaccine compositions of the present invention may also further optionally include one or more adjuvants. Non-limiting examples of suitable compounds and compositions having adjuvant activity include aluminum hydroxide, -phosphate, or - oxide, oil-in-water, water-in-oil, water-in-oil-in-water, emulsions based on, for example, a mineral oil, such as, without limitation, Drakeol®, Bayol®, or Marcol®; a vegetable oil, such as without limitation, cottonseed oil, peanut oil, or corn oil; vitamin E acetate; saponins; a fish oil, such as, without limitation, squalene or squalane; or any combinations thereof.

BVDV-specific vaccine formulations according to the present invention may also further optionally contain one or more other immunogens specific for one or more additional viruses and/or microorganisms that are also potentially pathogenic to the animal being immunized. For instance in cattle and related bovines, additional immunogens that can be present in the composition may be derived from one or more species of bovine-pathogenic viruses, including without limitation, rotaviruses, bovine respiratory syncytial viruses, bovine herpesvirus (including type 1), bovine coronaviruses, parainfluenza viruses (including type 3), bovine paramyxoviruses, or such like, or alternatively, those that are derived from one or more bovine-pathogenic microbial species such as, without limitation, *Pasteurella multocida*, *Mannheimia haemolytica* (formerly *Pasteurella haemolytica*), *Histophilus somni* (formerly *Haemophilus somnus*), *Mycoplasma bovis*, and the like, or a combination of any of the foregoing.
In some embodiments, when relevant, as would be understood by one of ordinary skill in the art, the immunogenic compositions and vaccines of the present invention can include administration of a single dose to an animal, or alternatively, can include administration of multiple, and/or successive doses to the animal over time. Alternatively, the immunogenic compositions of the present invention may also be co-administered with one or more anti-viral or anti-microbial compounds, either alone or further in combination with the administration of one or more distinct microbial-specific immunogens or vaccine formulations.
Another important aspect of the present invention concerns methods for using the disclosed immunogenic compositions to deliver one or more therapeutic agents for treating or ameliorating one or more symptom(s) of an infection or disease in a mammal. Such methods generally involve administration to a mammal in need thereof, one or more of the disclosed immunogenic compositions, in an amount and for a time sufficient to treat, ameliorate, or lessen the severity, duration, or extent of, such a disease or infection in such a mammal.
The methods and compositions of the invention may also be used in prevention, prophylaxis, and/or vaccination of an animal that has, is suspected of having, is at risk for developing, or has been diagnosed with one or more infections and/or diseases, either before, during, or after diagnosis or the onset of one or more clinical symptoms of the disease, or the appearance of one or more symptoms thereof.

### METHODS FOR INDUCING AN IMMUNE RESPONSE

The present invention further includes methods for inducing a detectable immune response against one or more viral pathogens in a susceptible animal. One preferred method includes administering to an animal in need thereof, or potentially in need thereof based on, *e*.*g*., known risk factors for a given disease or infection, an amount of a vaccine composition as disclosed herein sufficient to induce a detectable immune response in the animal.

In some embodiments, the invention encompasses methods of vaccinating a subject against a pestiviral infection, such as BVDV, or against a multifactorial disease, such as BRDC, in which a pestiviral infection is either implicated or causal. Such methods generally involve administering to an animal in need thereof, a prophylactically- and/or therapeutically-effective amount of a pestiviral-specific vaccine composition, and one or more pharmaceutically- or veterinary-acceptable carriers, buffers, diluents, vehicles, or such like, to provide a detectable immune response in the animal against a pestiviral infection, or a disease caused by or exacerbated by such a pestiviral infection.

To that end, the present invention provides methods for using the disclosed immunogenic compositions and vaccine formulations for the prophylaxis, treatment, amelioration or management of one or more viral or microbial diseases or infections in an animal, or one or more symptoms thereof.

The present invention also provides for the use of one or more of the disclosed immunogenic compositions in the manufacture of a veterinary medicament or vaccine for the prophylaxis or prevention of disease, including, in the preparation of one or more vaccines suitable for prophylactic administration to prevent or ameliorate one or more symptoms of a pestiviral infection, including, for example, BVDV, in an animal.

The invention also provides methods for providing a therapeutic or prophylactic immunogenic compound to a first cell in a mammal, with the method generally including providing to a mammal in need thereof, an effective amount of a BVDV immunogenic composition as disclosed herein that includes a plurality of modified live viral particles as an active ingredient, for a time effective to provide the desired therapy and/or prophylaxis in the immunized or treated mammal.

### THERAPEUTIC AND PROPHYLACTIC KITS

Kits including one or more of the disclosed immunogenic compositions or pharmaceutical formulations including such; and instructions for using the kit in one or more prophylactic or therapeutic regimens also represent illustrative aspects of the present disclosure. Such kits preferably include one or more of the disclosed immunogenic compositions or vaccines, either alone, or in combination with one or more additional therapeutic compounds, pharmaceuticals, and such like, and instructions for use of the components in the prevention or treatment of disease in an animal. The kits according to the invention may be packaged for commercial distribution, and may also further optionally include one or more delivery devices (*e*.*g*., syringes, vials, injectables, or such like) for administering the composition(s) to the selected animal.

The container(s) for such kits may typically include at least one vial, test tube, flask, bottle, syringe or other container, into which the immunogenic composition(s) or vaccine formulation(s) may be placed, and, preferably, suitably dispensed into one or more aliquot(s) for administration to an animal. Where a second immunogenic composition or a first antiviral or antimicrobial compound is also desired, the kit may also contain the second immunogenic composition or the first antiviral or antimicrobial compound in a second distinct container, or in a single container with a breakable or non-breakable barrier to isolate the two components until preparing for administration. Alternatively, a plurality of distinct immunogenic composition(s) and/or distinct antiviral or antimicrobial compound(s) may be prepared in a single formulation, and may be packaged in a single container, vial, flask, syringe, catheter, cannula, bottle, test tube, ampoule, or other suitable container. The kit may also include a larger container, such as a case, that includes the containers noted above, along with other equipment and the like. Multiple doses may be contained in suitable vessel within the container, if desired, preferably along with any suitable weight or volumetric-measuring device to meter a pre-selected dose as needed.

### COMPOSITIONS FOR USE IN THE PREPARATION OF MEDICAMENTS

Another important aspect of the present invention concerns methods for using the disclosed immunogenic compositions (as well as formulations including them) in the preparation of medicaments for preventing, treating or ameliorating a disease, or the symptoms thereof, in an animal, such as a vertebrate mammal. Use of the disclosed immunogenic compositions is also contemplated in therapy and/or prophylaxis of one or more diseases of microbial or viral origin.

Such use generally involves administration to an animal in need thereof one or more of the disclosed immunogenic compositions in an amount and for a time sufficient to prevent, treat, or manage one or more diseases or symptoms thereof in the affected animal. Compositions including one or more of the disclosed immunogenic formulations also form part of the present invention, and particularly those compositions that further include at least a first pharmaceutically-acceptable excipient for use in the therapy or prophylaxis of one or more diseases of viral and/or microbial origin.

The immunogenic compositions of the present invention may be prepared as univalent (*i*.*e*., monovalent) vaccines, or alternatively, as bivalent, trivalent or even multivalent (*i*.*e*., polyvalent) vaccines. For example, a monovalent vaccine will preferably include a single immunogenic composition of the present invention (for example a population of modified, live BVDV-1b viruses) that is capable of eliciting a specific anti-BVDV immune response when introduced into the body of a selected recipient mammal, and particularly mammals that are susceptible to BVDV infection.

Similarly, a bivalent vaccine composition will preferably include at least a first BVDV-1b-specific composition, and at least a second viral- or microbial-specific immunogen, each being capable of eliciting a specific immune response in a mammal. Multivalent (including trivalent or polyvalent) vaccine compositions preferably will include three or more viral- or microbial-specific immunogens, respectively. In an embodiment illustrated herein, the inventors developed a surprising and unexpectedly advantageous hexavalent (six-way) modified, live vaccine formulation that comprises immunogens specific for BVDV-1b, BVDV Type 1a (BVDV-1a), BVDV Type 2 (BVDV-2), PI₃, bovine respiratory syncytial virus (BRSV), and BHV-1, the causal agent of infectious bovine rhinotracheitis (IBR), to control BRDC infection in mammalian populations, and particularly in domestic livestock.

### DESCRIPTION OF ILLUSTRATIVE EMBODIMENTS

Illustrative embodiments of the invention are described below. In the interest of clarity, not all features of an actual implementation are described in this specification. It will of course be appreciated that in the development of any such actual embodiment, numerous implementation-specific decisions must be made to achieve the developers' specific goals, such as compliance with system-related and business-related constraints, which will vary from one implementation to another. Moreover, it will be appreciated that such a development effort might be complex and time-consuming, but would be a routine undertaking for those of ordinary skill in the art having the benefit of this disclosure.

### "SHIPPING FEVER"

"Shipping fever" is a term given to an acute, highly-contagious, septicemic syndrome in cattle and sheep that is characterized clinically by fever, acute inflammation of the airways, nasal discharge, anorexia, depression, fibrinous pneumonia, and necrosis of the infected tissues. Most frequently encountered in feedlots following shipping, shipping fever is the major cause of death among young cattle, and is responsible for an estimated annual loss to the industry of more than half a billion dollars. In 1991 alone, shipping fever was estimated cost the U.S. cattle industry almost $624 million, due primarily to the costs of treatment, production loss, and death.

The pathogenesis of shipping fever is generally considered to involve adverse external influences predisposing the animal to an initial viral respiratory infection, which, in turn, produces conditions favorable for the proliferation of one or more secondary bacterial infections.

### BOVINE RESPIRATORY DISEASE (BRD) COMPLEX (BRDC)

The primary causative agent of shipping fever is a multifactorial condition known as bovine respiratory disease (BRD) or bovine respiratory disease complex (BRDC). BRDC, a major cause of economic loss in the beef cattle industry, is characterized by concomitant sequential or simultaneous infection by both viral and bacterial pathogens. The viral pathogens implicated in BRDC include Bovine herpes virus 1 (BHV-1), Bovine PI₃, Bovine viral diarrhea virus (BVDV), and Bovine respiratory syncytial virus (BRSV).

Following viral infection, the affected animals then develop one or more subsequent bacterial infections (*e*.*g*., *Mannheimia* [formerly *Pasteurella*] *haemolytica*, *Pasteurella multocida*, *Histophilus somni* [formerly *Haemophilus somnus*], *Actinomyces pyogenes*, and various *Mycoplasma* spp.) which often manifest in acute pneumonia.

The most common and earliest recognizable clinical sign of pneumonia is depression. Calves exhibiting depression will have drooping ears, an extended head, a bowed back and/or often isolate themselves from other cattle. As the health of the calves progressively deteriorates, they stop feeding, exhibit an increased respiratory rate, and develop a pronounced fever (typically in the 104°-108°F range).

### PESTIVIRUSES

Pestiviruses cause economically important diseases in animals worldwide. The genus *Pestivirus*, within the family Flaviviridae, comprises three species of single-stranded positive-sense RNA viruses: bovine viral diarrhea virus (BVDV), classical swine fever virus (CSFV), border disease virus (BDV). Recently, a fourth, distinct group of pestiviruses has been identified that is genetically related to BVDV, and is referred to in the literature as bovine viral diarrhea virus Type 2 (BVDV-2) (see *e.g.,* Thiel *et al.,* 1996; and Becher *et al*., 1995; each of which is specifically incorporated herein in its entirety by express reference thereto). Consequently, contemporary texts now refer to the original species of BVDV as "BVDV-1" to distinguish between the two species.

### BOVINE VIRAL DIARRHEA VIRUS (BVDV)

The type species of *Pestivirus* is BVDV Type 1 (BDVD-1), whose genome is approximately 12.5-kb in length and contains one large open reading frame (ORF) (Collett *et al*., 1988, specifically incorporated herein in its entirety by express reference thereto). The ORF codes for a large polyprotein of approximately 450 kDa that is processed co- and post-translationally by host or viral proteases. The N-terminal end of standard BVDV polyprotein results in a non-structural protein p20 (Npro), capsid protein p14 (C); envelope glycoproteins gp48 (E0), gp25 (E1), gp53 (E2); non-structural proteins p125 (NS23), p10 (NS4A), p32 (NS4B), p58 (NS5A) and p75 (NS5B) (see, *e.g.,* Tautz *et al*., 1997; Xu *et al*., 1997; Elbers *et al*., 1996; and Wiskerchen *et al*., 1991, each of which is specifically incorporated herein in its entirety by express reference thereto). BVDV-1 exists in two biotypes, cytopathic (designated "cp") or non-cytopathic ("ncp"), which differ by the production of a single 80-kDa polypeptide (non-structural protein p80, NS3) in the cytopathic variant (see, *e.g.,* Gillespie *et al.,* 1960, specifically incorporated herein in its entirety by express reference thereto).

Based on phylogenetic analysis of a number of BVDV isolates, BVDV-1 has been shown to comprise at least 13 distinct subgenotypes (designated BVDV-1a to BVDV-1l), while two subgenotypes (BVDV-2a and BVDV-2b) have been identified for BVDV-2 (see, *e.g.,* Pellerin *et al*., 1994; Ridpath *et al.,* 1994, and Xue *et al.,* 2010; each of which is specifically incorporated herein in its entirety by express reference thereto).

BVDV-1 and BVDV-2 both cause acute infections in cattle (diarrhea, fever, hemorrhagic syndrome) and, if the infection occurs during pregnancy, abortion, malformation of the fetus and persistent infection of the calves. Persistently infected animals represent the major reservoir of the virus, and such animals may come down with the fatal mucosal disease (MD).

BVDV is closely related to viruses causing border disease in sheep and classical swine fever in porcines. Infected cattle typically exhibit a generalized "mucosal disease," which is characterized by elevated temperature, diarrhea, coughing and ulcerations of the alimentary mucosa (see *e.g.,* Olafson *et al.,* 1946; and Ramsey *et al*., 1953, each of which is specifically incorporated herein in its entirety by express reference thereto).

The BVD virus is capable of crossing the placenta of pregnant cattle and may result in the birth of persistently infected (PI) calves that are immunotolerant to the virus and persistently viremic for the rest of their lives. These PI cattle, which are highly predisposed to infection with microorganisms causing pneumonia or enteric disease, provide the necessary viral reservoirs for outbreaks of MD disease in cattle (see *e*.*g*., Liess *et al.,* 1974; Barber *et al*., 1985; Malmquist, 1968; and Ross *et al.,* 1986, each of which is specifically incorporated herein in its entirety by express reference thereto).

BVDV is spread through a herd in a fecal-oral manner, and strategies for control of the virus range from stricter management practices in an effort to simply reduce economic loss, to elaborate testing procedures to identify infected animals that, while effective, typically entail an unacceptable level of cost.

In the past thirty years, nearly one hundred and fifty vaccines for BVDV, both modified live virus (MLV) and inactivated attenuated virus or virus particles, have been marketed to the cattle industry will varying degrees of success; outbreaks of BVDV are still reported on an annual basis despite the widespread availability and use of commercial vaccine formulations. Current approaches to disease management involve repeated yearly inoculation with vaccine for cattle, and additional steps are generally taken in an attempt to insure that no calves are born as PI carriers. Several different test methods have been developed for the detection of BVDV, and/or the detection of BVDV-infected animals, include reverse transcription-polymerase chain reaction (RT-PCR), enzyme-linked immunoassay (ELISA), standard virus isolation techniques, and various immunohistochemical assays.

### EXEMPLARY DEFINITIONS

The terms "about" and "approximately" as used herein, are interchangeable, and should generally be understood to refer to a range of numbers around a given number, as well as to all numbers in a recited range of numbers (e.g., "about 5 to 15" means "about 5 to about 15" unless otherwise stated). Moreover, all numerical ranges herein should be understood to include each whole integer within the range. As used herein, the term "antigen" or "immunogen" means a substance that induces a specific immune response in a host animal. The antigen may comprise a whole organism, killed, attenuated or live; a subunit or portion of an organism; a recombinant vector containing an insert with immunogenic properties; a piece or fragment of DNA capable of inducing an immune response upon presentation to a host animal; a protein, a polypeptide, a peptide, an epitope, a hapten, or any combination thereof. Alternately, the immunogen or antigen may comprise a toxin or antitoxin. An antigen generally encompasses any immunogenic substance, *i.e.,* any substance that elicits an immune response (*e*.*g*.*,* the production of specific antibody molecules) when introduced into the tissues of a susceptible animal, and that is capable of specifically binding to an antibody that is produced in response to the introduction of the antigen. An antigen is capable of being recognized by the immune system, inducing a humoral immune response, and/or inducing a cellular immune response leading to the activation of B-and/or T-lymphocytes. An antigen may include a single epitope, or two or more epitopes.

As used herein, the term "antibody" refers to a protein that binds to other molecules (antigens) *via* heavy and light chain variable domains, V_{H} and V_{L}, respectively. The term "antibody" refers to any immunoglobulin molecule, including, for example, but not limited to, IgM, IgG, IgA, IgE, IgD, and any subclass thereof or combination thereof. The term "antibody" also means a functional fragment of immunoglobulin molecules, including for example, but not limited to, Fab, Fab', (Fab')₂, Fv, Fd, scFv and sdFv fragments unless otherwise expressly stated.

As used herein, an "antigenic polypeptide" or an "immunogenic polypeptide" is a polypeptide which, when introduced into a vertebrate, reacts with the vertebrate's immune system molecules, *i.e*., is antigenic, and/or induces an immune response in the vertebrate, *i.e*., is immunogenic. Isolated antigenic and immunogenic polypeptides of the present invention in addition to those encoded by polynucleotides of the invention, may be provided as a recombinant protein, a purified subunit, a viral vector expressing the protein, or may be provided in the form of an inactivated virus vaccine, *e*.*g*., a live-attenuated virus vaccine, a heat-killed virus vaccine, *etc.*

As used herein, a "modified live vaccine" is a vaccine comprising a virus that has been altered, typically by passaging in tissue culture cells, to attenuate its ability to cause disease, but which retains its ability to protect against disease or infection when subsequently administered to an animal.

As used herein, an "adjuvant" means a composition comprised of one or more substances that enhances the immunogenicity and efficacy of a modified, live virus or antigen, when present in a vaccine composition that contains a population of such viruses, or a plurality of such antigens.

As used herein, an "infectious unit" of a virus is defined as a TCID₅₀, or the amount required for infecting or killing 50% of a tissue culture of susceptible cells.
As used herein, the term "carrier" is intended to include any solvent(s), dispersion medium, coating(s), diluent(s), buffer(s), isotonic agent(s), solution(s), suspension(s), colloid(s), inert(s) or such like, or a combination thereof that is pharmaceutically acceptable for administration to the relevant animal. The use of one or more delivery vehicles for chemical compounds in general, and immunogens in particular, is well known to those of ordinary skill in the pharmaceutical arts. Except insofar as any conventional media or agent is incompatible with the active ingredient, its use in the diagnostic, prophylactic, and therapeutic compositions is contemplated. One or more supplementary active ingredient(s) may also be incorporated into, or administered in association with, one or more of the disclosed immunogenic compositions and vaccine formulations thereof.

As used herein, the terms "immunize" or "immunization" or similar terms refer to conferring the ability to mount a detectable, or preferably, a substantial immune response against a specific antigenic epitope or immunogen. These terms do not necessarily infer complete immunity, but rather that an immune response be produced that is substantially greater than baseline, *e*.*g*., where immunogenic compositions of the invention are not administered or where a conventional vaccine is administered. For example, a mammal is considered to be immunized against one or more target immunogens, if a cellular and/or humoral immune response to the target immunogen(s) occurs (and preferably a substantial immune response) following administration of the vaccines compositions disclosed herein.
As used herein, the term "immunological response" to a composition or vaccine denotes the development of a cellular and/or antibody-mediated immune response in the host animal. Generally, an immunological response includes (but is not restricted to) one or more of the following effects: (a) the production of antibodies; (b) the production of B cells; (c) the production of helper T cells; and/or (d) the production of cytotoxic T cells, that are specifically directed to a given antigen or hapten.
As used herein, the term "immunogenic" as used herein also refers to a substance, such as an amino acid sequence, a portion of an amino acid sequence within a protein, polypeptide, or peptide, or a modified or attenuated killed or live virus that elicits an immunological response in a host animal. As used herein, the term "immunogenic protein," "immunogenic peptide," or "immunogenic polypeptide" refers to proteins, peptides, and polypeptides that are immunologically active in the sense that once administered to the host, it is able to evoke an immune response of the humoral and/or cellular type directed against the protein. The term epitope relates to a protein site able to induce an immune reaction of the humoral type (B cells) and/or cellular type (T cells).

The term "pathogen" is defined herein as any sort of infectious agent, including *e*.*g*., viruses, prions, protozoans, parasites, as well as microbes such as bacteria, yeast, molds, fungi, and the like.

As used herein, the term "individual" (also interchangeably referred to as "host," "subject," "recipient," "patient," *etc*.) refers to any animal that can receive one or more of the pharmaceutical compositions or vaccine formulations disclosed herein. Preferably, the subject is a vertebrate animal, which is intended to denote any animal species (and preferably, a mammalian species). In certain embodiments, the individual is preferably any mammalian host, including but not limited to, non-human primates, bovines, canines, caprines, cavines, corvines, epines, equines, felines, hircines, lapines, leporines, lupines, ovines, porcines, racines, vulpines, and the like, including livestock, zoological specimens, exotics, as well as companion animals, pets, and any animal under the care of a veterinary practitioner.
The phrases "veterinary-acceptable" and "pharmaceutically-acceptable" refers to molecular entities and compositions that do not produce an allergic or similar untoward reaction when administered to a mammal. As used herein, "pharmaceutically acceptable salt" refers to a salt that retains the desired biological activity of the parent compound and does not impart any undesired toxicological effects. Examples of such salts include, but are not limited to, acid addition salts formed with inorganic acids, for example, hydrochloric acid, hydrobromic acid, sulfuric acid, phosphoric acid, nitric acid, and the like; and salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, pamoic (embonic) acid, alginic acid, naphthoic acid, polyglutamic acid, naphthalenesulfonic acids, naphthalenedisulfonic acids, polygalacturonic acid; salts with polyvalent metal cations such as zinc, calcium, bismuth, barium, magnesium, aluminum, copper, cobalt, nickel, cadmium, and the like; salts formed with an organic cation formed from N,N'-dibenzylethylenediamine or ethylenediamine; and combinations thereof.
As used herein, a "protective immune response" or "therapeutic immune response" refers to a CTL and/or an HTL response to an antigen, which in some way prevents or at least partially arrests disease, or the symptoms, side effects or progression thereof. The immune response may also include an antibody response that has been facilitated by the stimulation of helper T cells.

As used herein, the term "vaccine" refers to a composition or formulation that contains an immunogenic composition of the present invention in a form that is capable of being administered to a vertebrate, and preferably to an animal such as a mammal. Typically, vaccines of the present invention will include one or more of the immunogenic compositions (including one or more modified, live virus particles or pluralities thereof) disclosed herein, formulated for administration to an animal in need thereof. Such compositions may be of any suitable formulation, including, without limitation, those prepared in an aqueous vehicle, as well as those in frozen, freeze-dried, lyophilized, or dehydrated form that are then subsequently rehydrated or suspended in a conventional pharmaceutically-acceptable vehicle (*e*.*g*., sterile saline or a similar buffered aqueous solution) prior to administration. In such forms, the vaccine compositions of the present invention can be manufactured in convenient single or multiple-dose aliquots that may readily be employed in one or more of the methods or vaccination regimens disclosed herein to prevent, manage or otherwise treat one or more conditions or one or more symptoms of viral and/or microbial infection in a susceptible animal.

Upon introduction into the animal host, the immunogenic compositions and vaccines comprising them are able to provoke an immune response, and preferably an immune response that is specific to the introduced antigen(s), such that the resulting immune response is readily detectable using conventional assays known to those of ordinary skill in the immunological arts, including, but not limited to, assays detecting the production of specific antibodies, cytokines and/or the activation of cytotoxic T cells, antigen presenting cells, helper T cells, dendritic cells and/or other cellular responses within the cells and tissues of the vaccinated animal. The vaccine compositions of the present invention may include, or be concomitantly administered in or with, one or more adjuvants alone, or in combination with one or more additional antigen(s) or such like.

The vaccines and immunogenic compositions of the present invention confer an immune response to an animal after immunization. As used herein, the term "immune response" refers to a humoral immune response and/or cellular immune response leading to the activation or proliferation of B-and/or T-lymphocytes. In some instances, however, the immune responses may be of low intensity and become detectable only when using at least one substance in accordance with the invention, while others may require repeated administration, an adjuvant, a second or further active agent, or one or more combinations thereof. The term "adjuvant" refers to an agent used to stimulate the immune system of a living organism, so that one or more functions of the immune system are increased and directed towards the immunogenic agent.

As used herein, the terms "treatment," "treat," "treated," or "treating" refer to therapy or the amelioration of one or more symptoms of a disease, or the reduction in the extent or severity of disease, or a symptom thereof, whether before or after its development afflicts a susceptible animal. When used with respect to an infectious disease, for example, the terms refer to a treatment or treatment regimen that decreases the severity of the infection or decreases or lessens or delays one or more symptoms of illness attributable to the infection, as well as increasing the ability of the infected animal to fight the infection, including *e*.*g*., the reduction and/or elimination of the infection from the body of the treated individual, or to lessen or prevent the disease from becoming worse, or from spreading to other animals that come into contact with the affected animal.

The term "immunogenically-effective amount" has its usual meaning in the art, *i*.*e.,* an amount of an immunogen that is capable of inducing an immune response that significantly engages pathogenic agents that share immunological features with the immunogen. This term can also encompass either therapeutically or prophylactically effective amounts, or both.

As used herein, the terms "prevention," "prevent," "prevented," "preventing," "vaccinating," and "vaccination," refer to the prophylaxis or to the partial or complete inhibition of infection, or to the reduction or delay in the onset, of one or more diseases, or one or more symptoms thereof, in an animal that may be predisposed to it, but has not yet been exposed to it or been diagnosed as having it. When used with respect to an infectious disease, for example, the terms refer to a prophylactic administration of one or more of the immunogenic compositions of the invention, which tends to increase the resistance of an animal to infection with one or more viral or microbial pathogens or, in other words, decreases the likelihood that the subject will become infected with the pathogen(s) or, if infected, will delay symptoms, decrease the severity of the infection, or decrease symptoms of illness attributable to the infection, or any combination thereof in the infected animal.

Each of "preventing" and "treating" include managing a particular infection, disease, condition, or symptom thereof, in an animal, as well as any beneficial modification of candidate status or the course of the disease or condition, or any symptoms thereof. The managing may address some or all of the symptoms thereof with or without actually affecting the underlying infection or any disease or condition resulting therefrom.

The term "e.g.," as used herein, is used merely by way of example, without limitation intended, and should not be construed as referring only those items explicitly enumerated in the specification.
In accordance with long standing patent law convention, the words "a" and "an" when used in this application, including the claims, denotes "one or more."

### EXAMPLES

The following examples are included to demonstrate illustrative embodiments of the invention. It should be appreciated by those of ordinary skill in the art that the techniques disclosed in the examples that follow represent techniques discovered to function well in the practice of the invention, and thus can be considered to constitute preferred modes for its practice. However, those of ordinary skill in the art should, in light of the present disclosure, appreciate that many changes can be made in the specific embodiments which are disclosed and still obtain a like or similar result without departing from the spirit and scope of the invention.

### EXAMPLE 1 - PRODUCTION OF BVDV MODIFIED, LIVE VIRUS

The present Example provides an illustrative method for large-scale production of BVDV-1b, modified, live virus (MLV).

### MATERIALS AND METHODS

### VIRUS AND MICROORGANISMS

The TGAC strain of BVDV-1b was obtained from The United States Department of Agriculture (USDA), Animal and Plant Health Inspection Service (APHIS), Center for Veterinary Biologics Laboratory (CVB-L) (Ames, IA, USA). The Master Seed (MS) was subsequently denoted as "**TVL-BVDV1b MS 04/27/2007 DW3-095**," and as noted in Applicants' copending U.S. provisional patent application no. 61/427,404, filed currently herewith, has been deposited under conditions that assure that access to the cultures will be available during the pendency of this patent application to one determined by the Commissioner of Patents and Trademarks to be entitled thereto under 37 C.F.R. § 1.14 and 35 U.S.C. § 122. The deposit is available as required by foreign patent laws in countries wherein counterparts of the subject application, or its progeny, are filed. However, it should be understood that the availability of a deposit does not constitute a license to practice the subject invention in derogation of patent rights granted by governmental action. The subject culture deposit will be stored and made available to the public in accord with the provisions of the Budapest Treaty for the Deposit of Microorganisms, *i.e*., it will be stored with all the care necessary to keep it viable and uncontaminated for a period of at least five years after the most recent request for the finishing of a sample of the deposit, and in any case, for a period of at least 30 (thirty) years after the date of deposit or for the enforceable life of any patent which may issue disclosing the deposited culture. The depositor acknowledges the duty to replace the deposit should the depository be unable to furnish a sample when requested, due to the condition of the deposit. All restrictions on the availability to the public of the subject culture deposit will be irrevocably removed upon the granting of a patent disclosing it. A deposit of **TVL BVDV1b MS 04/27/2007 DW3-095** virus was entered into the permanent collection of the Patent Depository of the American Type Culture Laboratory, located at 10801 University Blvd., Manassas, VA, 20110-2209, USA, on December 21, 2010 under the terms of the Budapest Treaty, whereupon it was assigned accession number ATCC PTA-11553 by the repository.
Identity of the MS virus (MSV) was performed using a reverse transcription-polymerase chain reaction (RT-PCR) assay to positively identify the MS virus as BVDV-lb. The protocol for differentiation of BVDV by PCR was developed by Ridpath and Bolin, 1998 (specifically incorporated herein in its entirety by express reference thereto). Positive identification was performed using an immunofluorescence assay (IFA) (McNulty *et al*., 1984). Replication of these viruses in Texas Vet Lab Bovine Kidney (TVL-BK) cells produced readily recognizable cytopathological changes.

### FREQUENCY OF IDENTIFICATION OF MICROORGANISMS

Prior to inoculation of TVL-BK cells with production seed virus, the cells are microscopically visualized to confirm that the cells are displaying the morphology previously described. Prior to harvesting each batch of production virus, TVL-BK cells are observed to confirm that they are exhibiting typical cytopathological effects (CPE) associated with the virus.

### VIRULENCE, MAINTENANCE, AND RANGE OF CULTURES OR SUBCULTURES

The consistency of the virulence, potency, and antigenicity of the BVDV-1b used in this vaccine was fostered by storage in the lyophilized or frozen condition, and restrictions on the number of passages or subcultures.

*Range:* Production virus = MSV+10 (efficacy serial passage), but may be any passage between 5 and 10. Production cell stock = MCS+20 (efficacy serial passage), but may also be any passage below 20.

### COMPOSITION AND REACTION OF MEDIUM USED IN SEED AND PRODUCTION CULTURES

Composition and reaction of the medium used in seed and production cultures were as follows: Working virus inocula (MSV+1 to MSV+8) and production virus inoculum (which was typically, but not exclusively at MSV+9), were propagated in TVL-BK cells; provided, the passage number of TVL-BK cells for virus propagation does not exceed 20 passages from MCS. The growth medium (for both working and production cell preparations) was Dulbeccos' Modified-Eagle's Medium (DMEM).

The resulting MSV may be stored lyophilized, in cryopreservation medium stored in liquid nitrogen, or in cryopreservation medium stored at -70 to -85°C. The MCS may be stored in cryopreservation medium (in liquid nitrogen), or in cryopreservation medium stored at -70 to -85°C.

### METHODS OF PREPARING SUSPENSIONS FOR SEEDING AND INOCULATION

Cryovials containing frozen TVL-BK cells were rapidly thawed. Culture flasks were filled to recommended capacity with medium and suspended cells were aseptically transferred from cryovials to the culture flask. Culture vessels were also seeded by splitting actively-propagating cultures at a ratio ranging from 1:3 to 1:6 (cm²:cm²). TVL-BK in flask or roller bottles, which were intended for subculture, were released from the culture surface by aseptically removing the growth medium and adding a 1X trypsin-EDTA solution. After incubation (5 min, at 35 to 39°C) the monolayer was dispersed. The trypsin was inactivated by adding the dispersed cells into a culture vessel with growth medium. Frozen aliquots of virus were rapidly thawed or resuspended in medium if the seed was lyophilized. TVL-BK cells (+20 or less) were inoculated with virus. Virus that was replicating in flasks or roller bottles, which was intended for subculture, was collected after the appropriate incubation time and/or CPE was observed. The viral fluids were then stored at 6 to -80°C or used immediately to inoculate other culture vessels.

Standard cell culture techniques were used to inoculate both seed and production media. TVL-BK cells at passage 20 or less were inoculated. Frozen production virus inoculum, which was typically at MSV+9, was rapidly thawed. The volume of virus inoculum ranged from 1 to 25 mL per 1600 cm² of culture area to achieve a multiplicity of infection ranging from 0.01 to 1.0 viruses per cell. The minimum inoculum titer was as follows: 10^{5.0}TCID₅₀ per mL for BVDV-1b. Inoculated cultures were incubated at 37 ± 2°C and 5 ± 1% CO₂ for 2 to 4 days.

### HARVEST

On the day of harvest, cultures were examined for virus-specific CPE and sterility. Typical minimum incubation time was 2 days after inoculation with BVDV-1b. Typical maximum incubation time was 4 days after inoculation with BVDV-1b. The virus suspensions were aseptically harvested from production vessels. Samples were collected to determine the TCID₅₀ of the harvest. The harvest material was stored at 6 to -80°C. Harvest material could be stored above freezing for up to one month, and frozen for up to six months prior to fabrication of the final product.

Harvest fluids that exhibit atypical CPE or evidence of contamination were discarded. Only harvest fluids exhibiting typical CPE and free of contamination were eligible for further production use. The minimum acceptable harvest virus titer was 10⁶ TCID₅₀/mL. Purity of harvest fluids was confirmed as follows: A 2-mL sample of harvest fluid was aseptically added to 38 mL of SCDB and incubated at 35 to 39°C along with negative (medium alone) and positive controls for 46 to 50 hours. The absence of macroscopic growth in the cultures as well as the negative control established that the harvest fluids were pure and satisfactory for further production use. Harvest fluids found not to be pure were discarded.

### EXEMPLARY FORMULATION OF A BVDV LIVE, MODIFIED VIRUS

All manipulations were conducted using aseptic technique. Suitable antibiotics, such neomycin and Nystatin (Mycostatin) were added during the assembly of a serial or subserial of vaccine at a rate of 15 µg and 15 Units /dose, respectively. The harvest fluids were diluted in DMEM as needed to adjust concentration, and stabilized by the addition of a 0.2-µm filtered sucrose solution, resulting in a final sucrose concentration of 20 ± 1%.

Virus harvest fluids were optionally concentrated by sterile ultrafiltration using a 10-kDa molecular weight cutoff filter. The degree of concentration typically did not exceed 50-fold.

Each batch of harvested viral fluids was analyzed to determine the TCID₅₀. Briefly, log dilutions (10⁻¹ through 10⁻⁸) of the harvest material were used to inoculate TVL-BK Cells on a 96-well plate. Plates were incubated at 37 ± 2°C and 5 ± 1% CO₂ for 96 ± 6 hours. Following incubation plates are read at 100× magnification, and examined for CPE. Titers were determined by the Spearman-Karber Method, as modified by Finney (1978). Final containers, which had been previously sterilized, were aseptically filled. Final containers were partially capped with a sterile inner seal stopper. Final product vials were transferred to a freeze dryer and desiccated. The drying cycle time ranged from 24 to 72 hours, with maximum product temperature of 22°C. Moisture content of the desiccated final product typically did not exceed 5%, and the minimum amount of antigenic material per dose in the final container was preferably approximately 8 × 10³ TCID₅₀ per dose.

### EXAMPLE 2 - BVDV-1B MONOVALENT VACCINE CALF CHALLENGE STUDY

The present example demonstrates the efficacy of a BVDV-1b monovalent vaccine in preventing infection in vaccinated animals.

### MATERIALS AND METHODS

### ANIMALS

Healthy four- to five-month-old mixed-breed heifer calves were purchased from a commercial source, identified by randomly-drawn ear tags, and screened serologically for susceptibility to BVDV. All calves received one dose of ceftiofur crystalline free acid (Excede®, Pfizer Animal Health, New York, NY, USA) upon arrival. The calves had free-choice access to water, hay and a pelleted feed ration.

### VACCINE

BVDV-1b Vaccine, Modified Live Virus, was prepared as described above. Briefly, BVDV-1b Master Seed Virus was propagated in the TVL-BK cell line (20^{th} pass), harvested at the 10^{th} passage, stabilized according to the outline of production, filled into bottles, and freeze-dried. The BVDV concentration was 8 × 10³ TCID₅₀ per dose as determined by the Spearman-Karber method (see *e*.*g*., Finney, 1978). Sterile water was used as a diluent to rehydrate the vaccine.

### VACCINATION AND CHALLENGE OF CALVES

Nineteen (19) BVDV-negative calves were commingled in a sorting ally. Each calf was put into one of two groups (vaccinates or nonvaccinated controls) by using a matched-set randomization scheme (Table 1) provided by CVB-Biometrics (Ames, IA, USA).

**TABLE 1. PAIR MATCHED RANDOMIZATION TABLE**

| Test Subject | Group | Calf |
|---|---|---|
| 1 | A | 53 |
| 2 | B | 12 |
| 3 | A | 54 |
| 4 | B | 2 |
| 5 | A | 50 |
| 6 | A | 74 |
| 7 | B | 32 |
| 8 | A | 77 |
| 9 | B | 3 |
| 10 | A | 67 |
| 11 | B | 22 |
| 12 | A | 72 |
| 13 | B | 71 |
| 14 | B | 1 |
| 15 | A | 69 |
| 16 | B | 58 |
| 17 | A | 39 |
| 18 | A | 65 |
| 19 | B | 68 |

| | | |
|---|---|---|
| Group A = Vaccinates; Group B= Non-Vaccinate Controls | | |

Ten of the serologically negative calves were vaccinated with one dose of BVDV-1b vaccine. Each vaccinate was given a 2-mL dose of the product by subcutaneous injection in the left side of the neck on day 0. The remainder of the calves did not receive any viral vaccines. After vaccination, the two groups of calves were maintained in separate but equivalent pens until two days prior to challenge.

Blood samples were collected on the day of arrival and tested for BVDV titers. Blood samples were also collected on the day of vaccination and the day of challenge. BVDV-1b antibody titers were determined by the constant virus-decreasing serum neutralization method (Schefers *et al*., 2008). Two days prior to challenge both vaccinate and non-vaccinate control groups were commingled for the purpose of pre-challenge clinical observation. Fourteen (14) days after vaccination, the vaccinate and control calves were challenged with a virulent isolate of BVDV-1b obtained from the lungs of a pen dead calf. The virus had been passed *in vitro* on TVL-BK cells using DMEM containing 10% equine Serum. A challenge dose of 8 × 10⁷ TCID₅₀ in 4 mL was administered to each calf, 2 mL into each nasal passage during inspiration.

Rectal temperatures were recorded daily for each calf for two days prior to challenge, the day of challenge and for 14 consecutive days post-challenge.

White blood cell (WBC) counts were determined for each calf for two days prior to challenge, the day of challenge and for 14 consecutive days post-challenge. Samples were collected in EDTA Vacutainer® tubes and counts performed using a Drew Scientific (Waterbury, CT, USA), Hemavet 950™ differential WBC counter.

Nasal swabs and buffy coats were collected from each calf two days prior to challenge, the day of challenge and for 14 consecutive days post challenge for the purpose of BVDV isolation. Nasal swabs were collected using BBL Culture Swabs with Liquid Stuart Media (Becton, Dickinson and Company, Sparks, MD, USA). The medium from the samples was sterile filtered and used to inoculate 80% confluent TVL-BK cells in culture.

Buffy coats were collected from EDTA vacutainer tubes and used to inoculate 80% confluent TVL-BK cells in culture. The buffy coat was removed after incubating for one hour. The cultures incubated for 4 to 5 days in 5 ± 2% CO₂ at 37 ± 3°C. The supernatants from all culture samples were assayed for the presence of BVDV-lb by RT-PCR using a BVDV-lb-specific primer set (BVDV-lb (first)) developed by the inventors with a suitable detection probe:

### BVDV-1b (first):

Forward primer: 5'-CACCCTATCAGGCTGTATTCATAGC-3' (SEQ ID NO: 1);
Reverse primer: 5'-TGCCCACAGCACATCTTAACC-3' (SEQ ID NO:2); and
BVDV-lb Detection probe: 5'-TCACCTGGACGACCC-3' (SEQ ID NO:3).

### BVDV-1b (second):

Second Forward primer: 5'-GTCGTCCAGGTGAAAACGGT-3' (SEQ ID NO:19);
Second Reverse primer: 5'-GTCGTCCAGGTGAAAACGGT-3' (SEQ ID NO:20); and
Second BVDV-1b Detection probe: 5'-GTCGTCCAGGTGAAAACGGT-3' (SEQ ID NO:21).

While BVDV-1b (first) provides acceptable results, BVDV-1b (second) displays better results.

All clinical observations were made independently, and the observers were blinded due to both the vaccinate, and non-vaccinate control groups being commingled with the only differentiating feature being the ear tag number. At no time throughout the study were the observers given knowledge of the vaccination status of an individual test animal until the clinical assessments were concluded.

### STATISTICAL ANALYSIS

**Leukopenia:** A pre-challenge leukocyte mean was calculated for each animal by averaging leukocyte counts for Days -2 through 0. The ratio of daily leukocyte counts relative to the pre-challenge mean was calculated (leukocyte count as a proportion of pre-challenge) for each post-challenge day 1 through 14. This proportion data was analyzed to determine if individual animals developed a leukopenia as defined by a 25% decrease in counts from baseline. The preventable fraction was calculated as described by Tanner and Morgan (1993) to determine if vaccination prevented the development of leukopenia in this study.

**Nasal Shedding:** Virus isolation data from nasal swab analysis is based on the proportion of vaccinates that BVDV was isolated from as compared to the proportion of controls that BVDV was isolated from post-challenge. The preventable fraction for BVDV-lb shedding was calculated as described by Tanner and Morgan (1993).

**Viremia:** Virus isolation data from buffy coats of blood samples was analyzed based on the proportion of vaccinates that BVDV-lb was isolated from as compared to the proportion of controls that BVDV-lb was isolated from post-challenge. The preventable fraction was calculated as described by Tanner and Morgan (1993).

**Antibody Titers:** Group comparisons of the ordinally-scaled titers were analyzed with the Mann-Whitney U Test.

**Rectal Temperature:** A pre-challenge average rectal temperature was determined for each animal. This average was used as a covariate in a repeated measures analysis of variance (ANOVA) which included terms for Group, Day and Group*Day interactions. All statistical analysis was performed using SAS Learning Edition v2.0 for Microsoft Windows® (SAS Institute, Inc., Cary, NC, USA).

### RESULTS

Leukopenia was detected in one of the ten vaccinates and eight of nine controls resulting in a preventable fraction of 87%. The raw leukocyte data is recorded in Table 2.

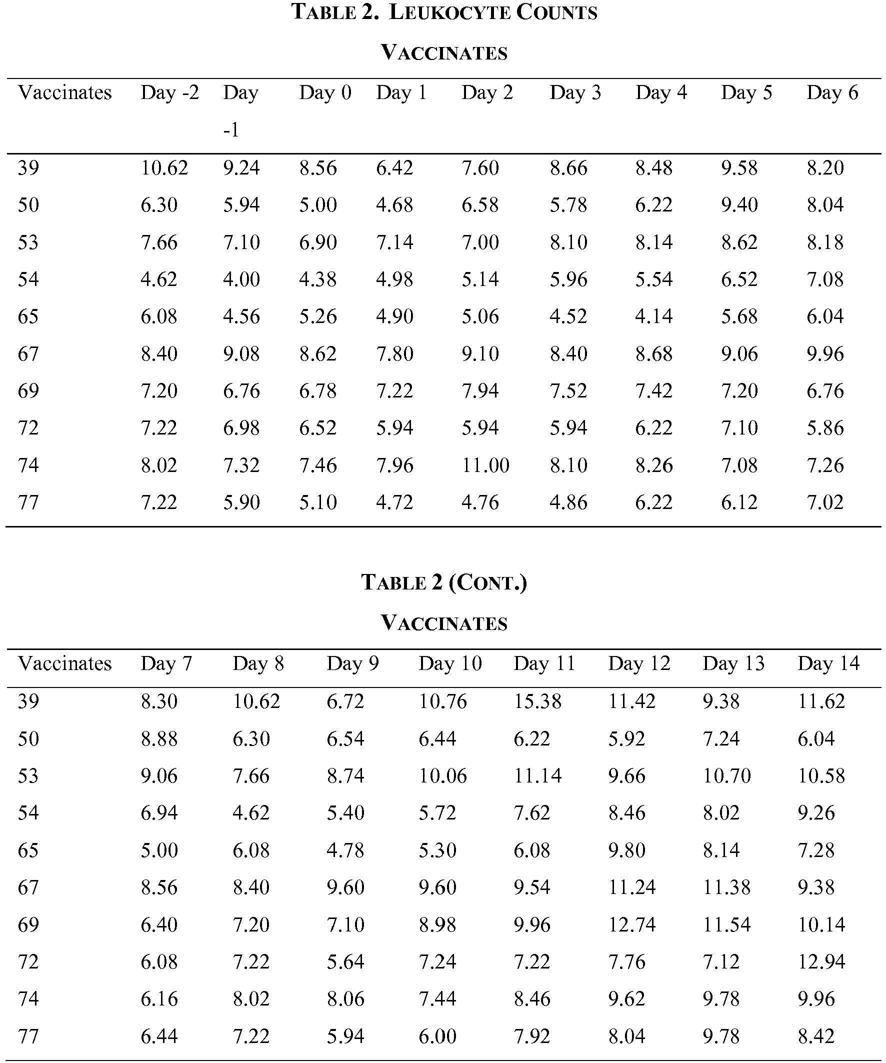

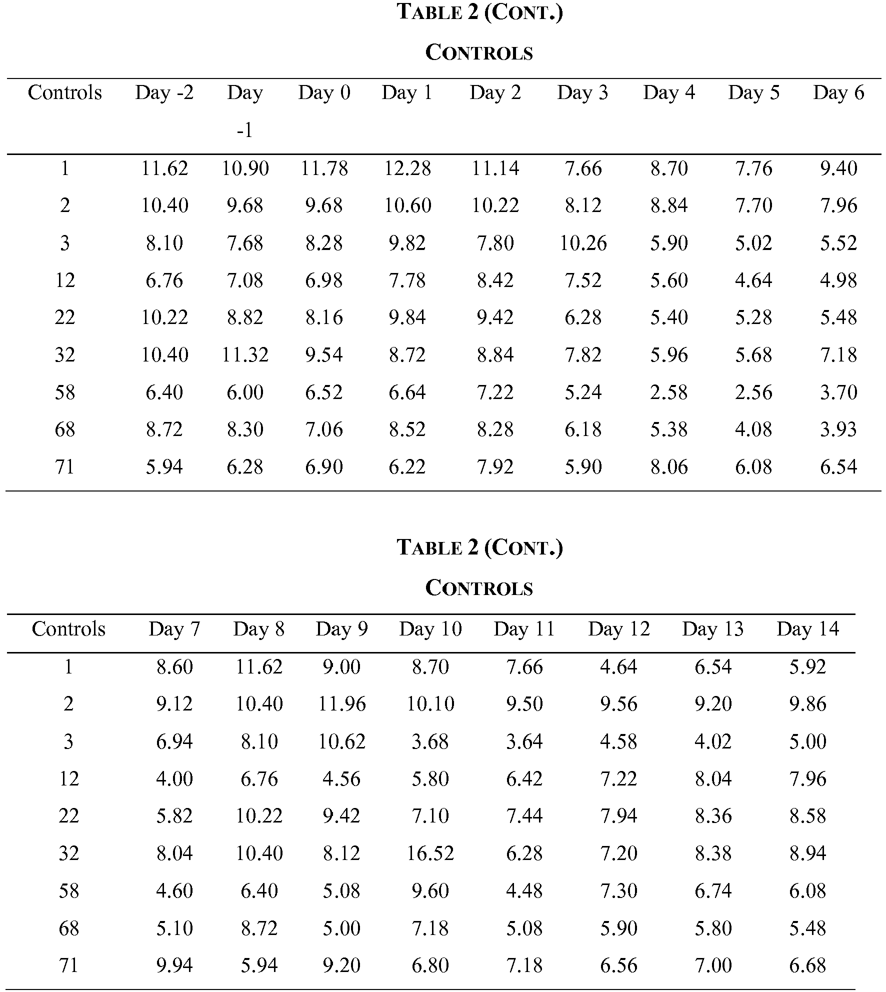

The daily proportion of the pre-challenge average for each individual calf is shown in Table 3.

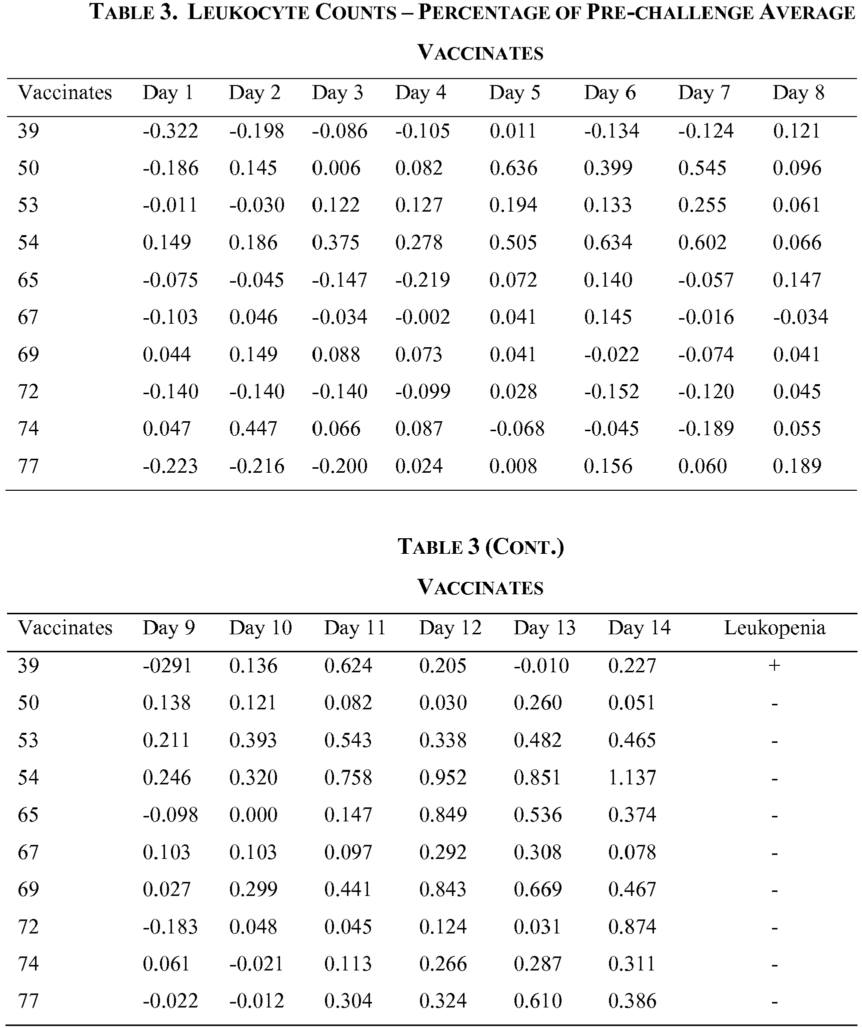

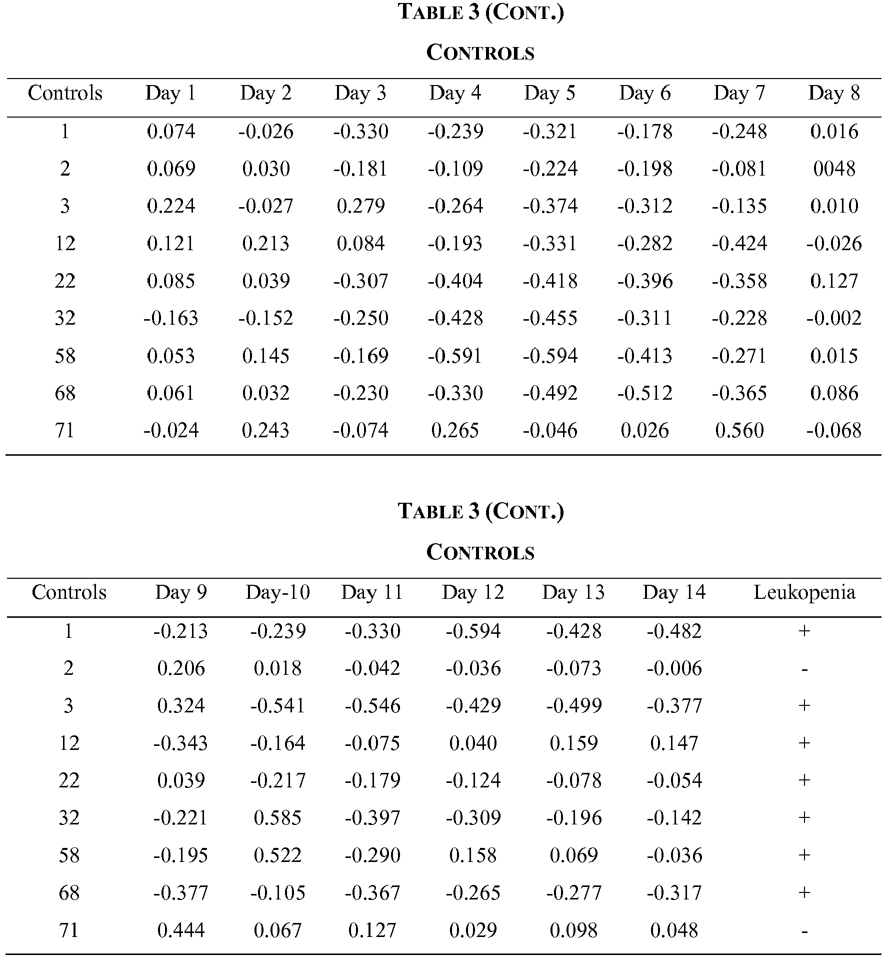

BVDV-1b was isolated from nasal secretions of one of ten vaccinates and eight of nine controls resulting in a preventable fraction of 90%. These data are shown in Table 4.

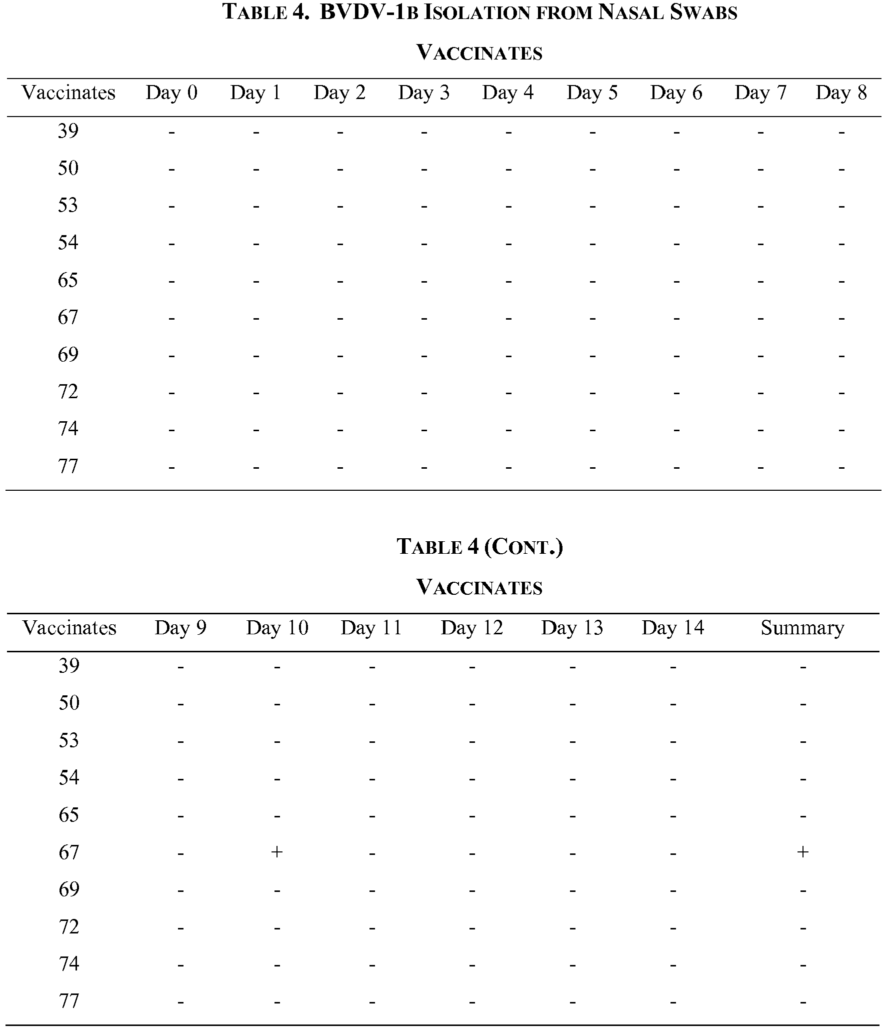

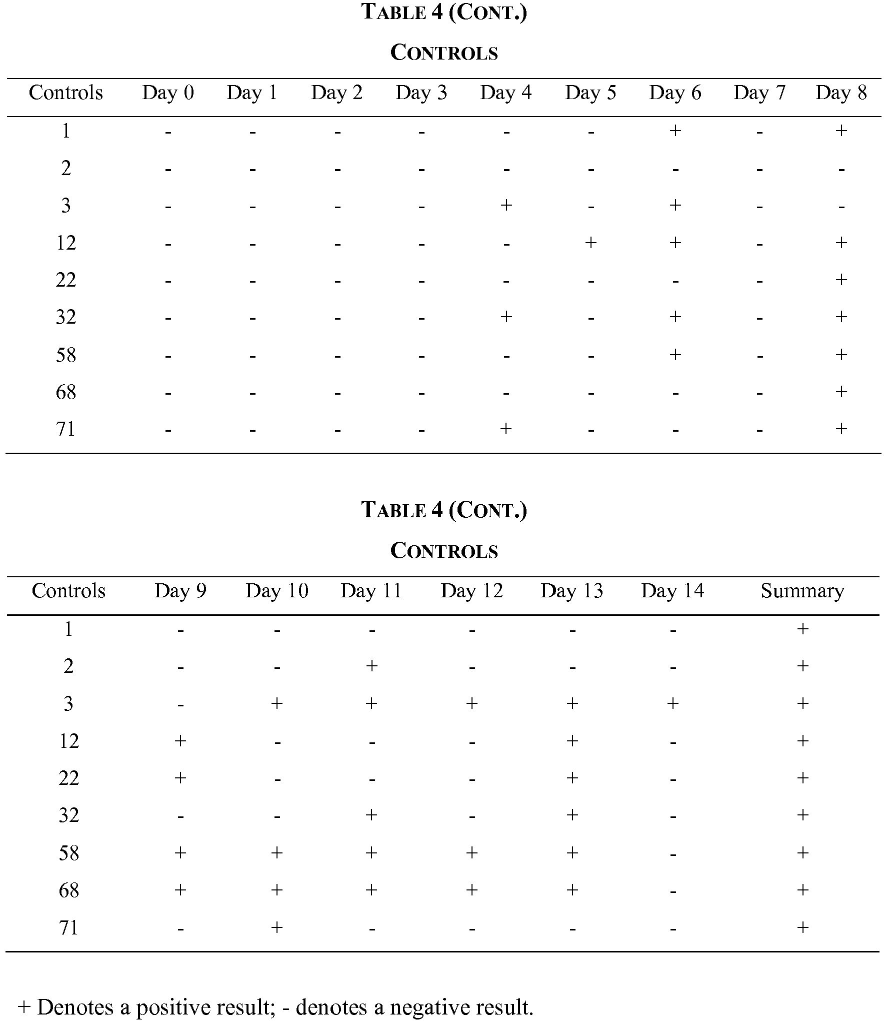

BVDV-1b was isolated from buffy coats of two vaccinate calves and all nine control calves resulting in a preventable fraction of 80%. These data are shown in Table 5.

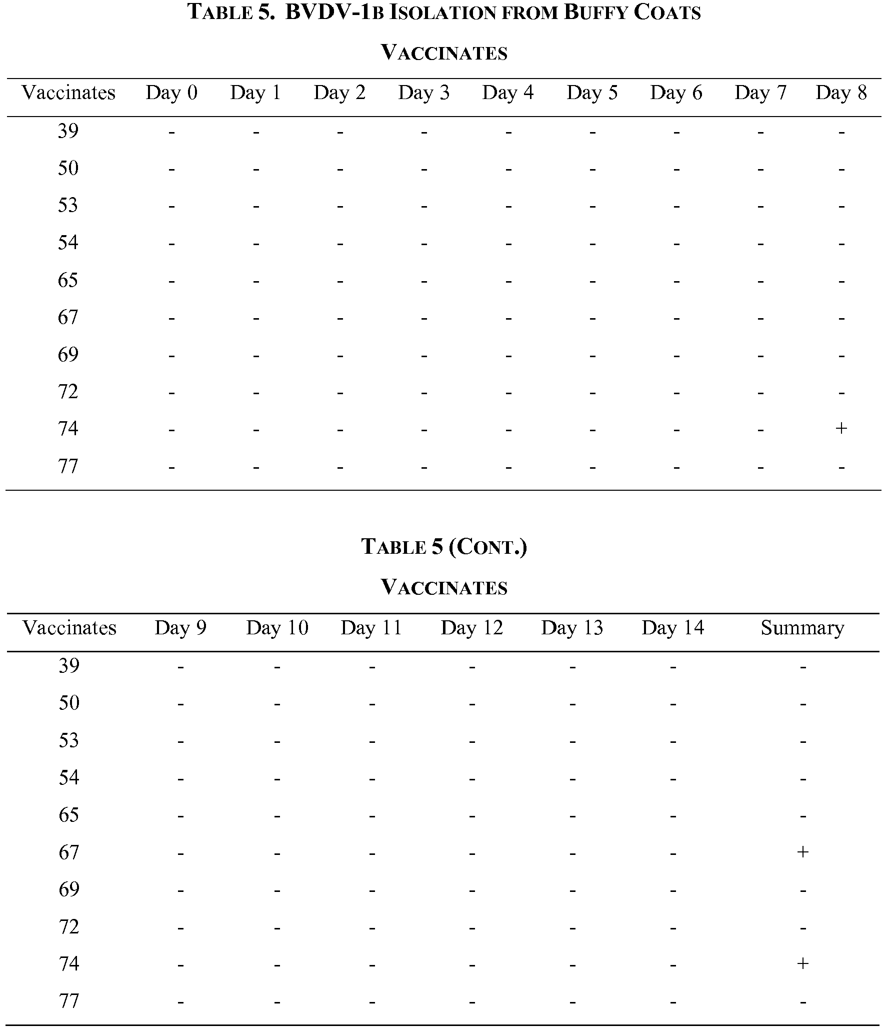

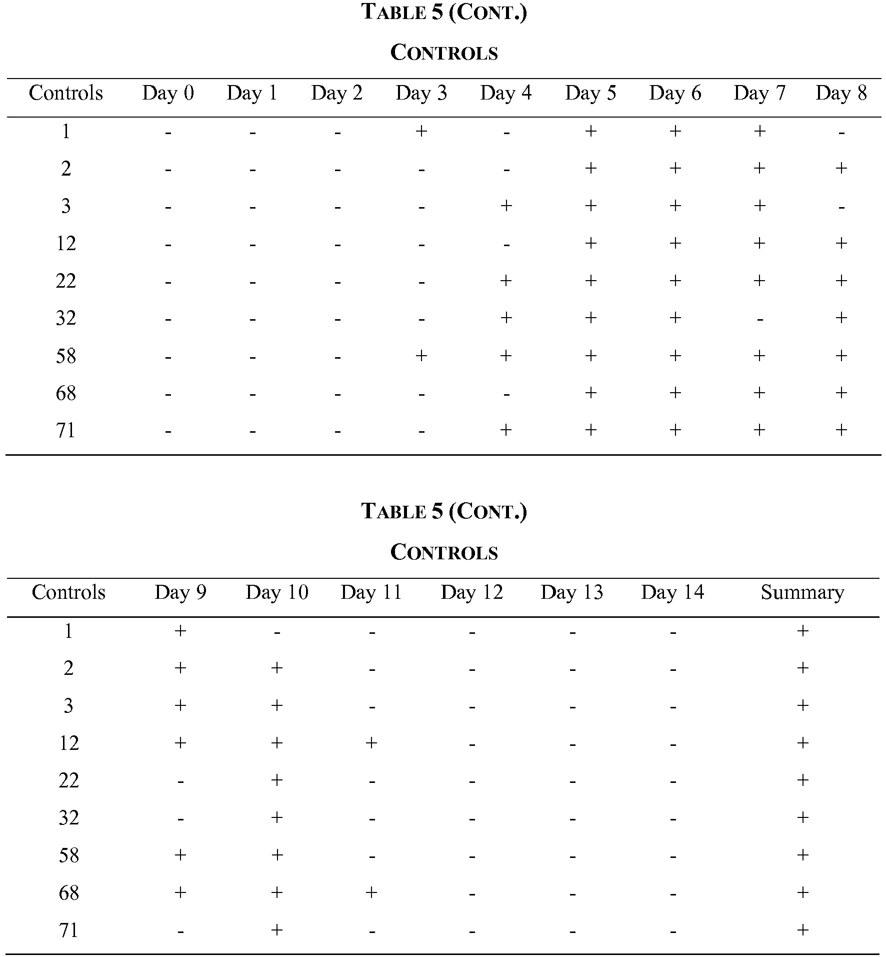

Vaccination induced a statistically-significant (*P* < 0.05) increase in BVDV-lb SN antibody titers. The vaccine did not cause a significant increase in BVDV-la or BVDV-2 SN titers. Individual SN antibody titers are presented in Table 6.

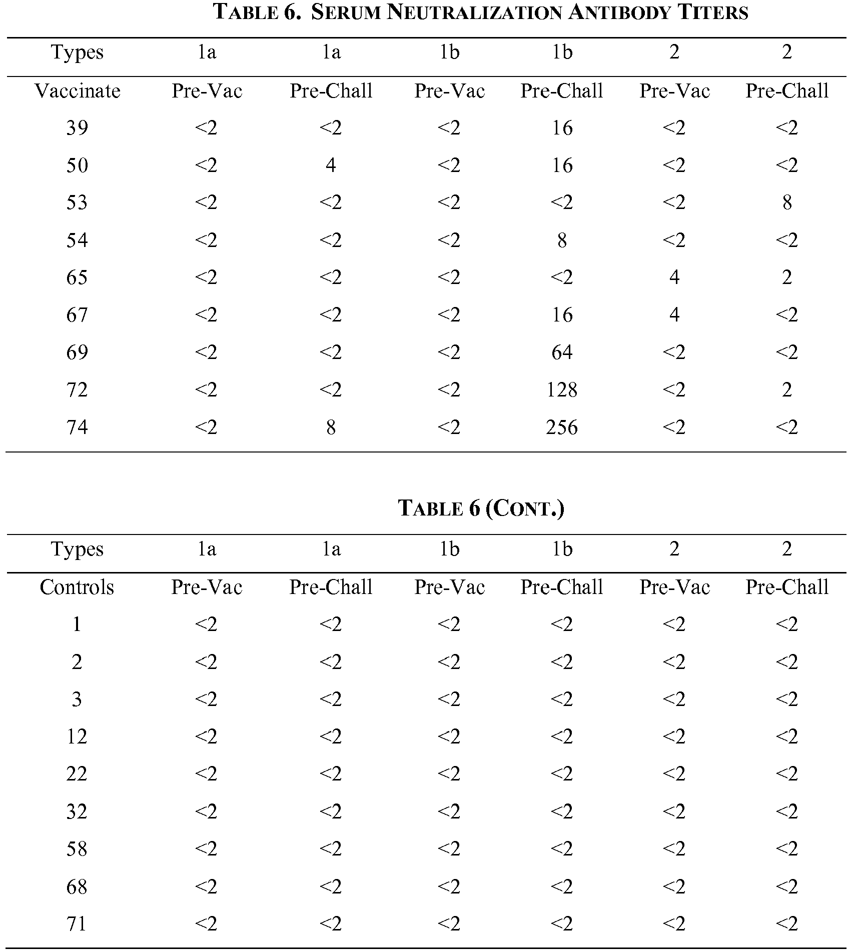

Analysis of rectal temperature data did not reveal any statistically significant effects of vaccination. Rectal temperature data is shown in Table 7.

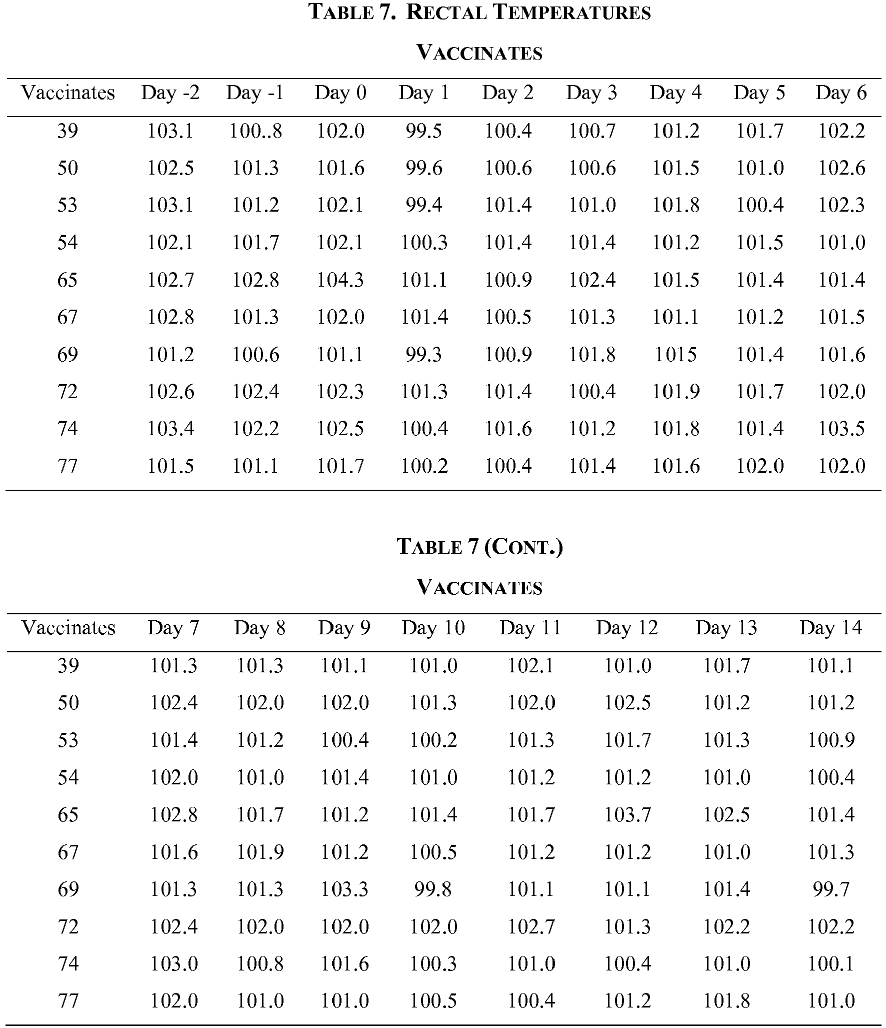

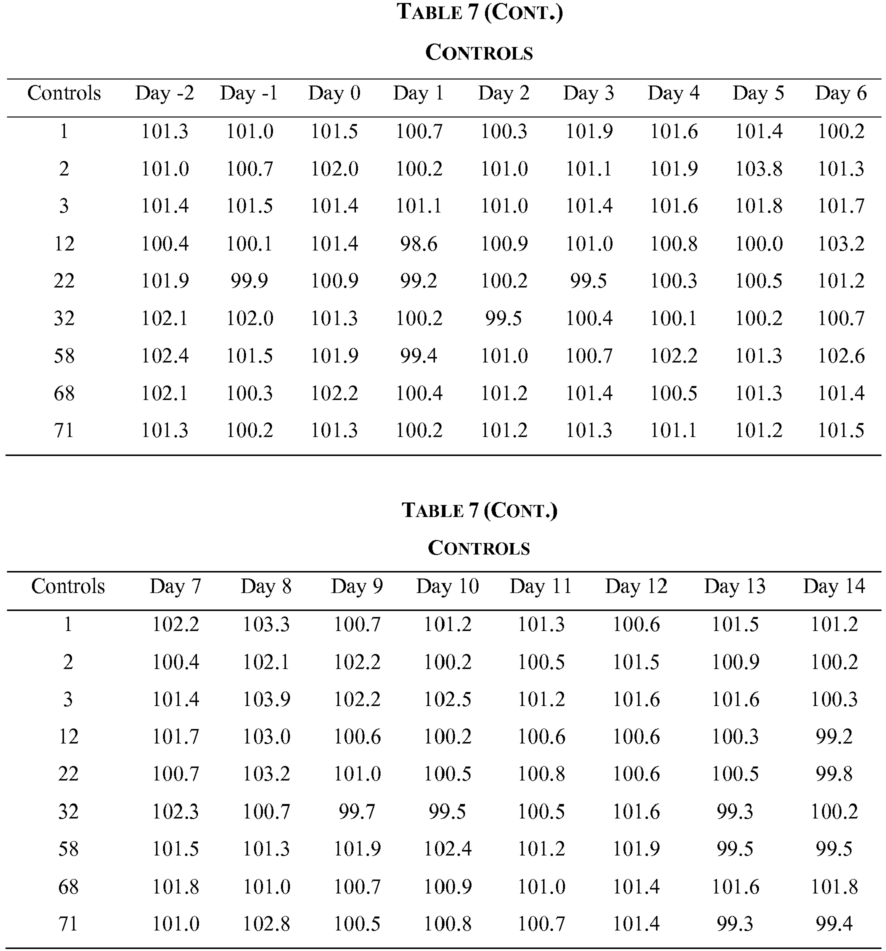

There were no clinical signs of BVDV infection observed in any of the calves during the course of this study.

Vaccination of healthy four-to-five month-old, mixed-breed heifer calves with one dose of BVBV-1b Vaccine (Modified, Live Virus) yielded an adjusted estimate of vaccine efficacy of 87% against leukopenia, 90% against nasal shedding of BVDV-lb and 80% against viremia. Serological analysis also revealed an antigenic response in the vaccinated calves.

### EXAMPLE 3 - BVDV-1B VIRAL BACKPASSAGE EVALUATION

The present examples evaluates the stability of the TVL-BVDV-1b MSV, assuring it did not revert to virulence when administered to animals.

One ampoule of the virus is thawed and used to inoculate TVL-BK cells to produce TVL-BVDV-1b +1 (5 × 10⁷ TCID₅₀/mL). This virus is used to intranasally inoculate the first set of ten colostrum-deprived dairy calves at a rate of 1 × 10⁶ TCID₅₀ per calf.

Calves in sets 2 through 5 are inoculated intranasally with pooled nasal secretions from the preceding set of calves.

Set one consists of ten calves, with the number of calves in sets two through five ranging from two to five calves depending on the number of calves that shed virus from the first set of 10 calves. Each calf remains in an isolated pen, and the primary outcomes of the study is the lack of clinical signs of BVDV in calves and the genetic stability of the MS through at least five successive *in vivo* passages.

Experimental units are excluded from the study if they have a BVDV-1a, BVDV-1b or BVDV-2 titer of ≥2 at the time of screening as determined by the constant virus decreasing serum neutralization method.

Blood samples for serology are collected from calves during the screening process, the day of inoculation (day 0) and at the conclusion of the study. Nasal secretions are collected from each individual calf in set one on days two through eight after inoculation.

Aliquots of nasal secretions from each individual calf for each of these days are assayed for virus isolation and stored frozen. The day with the greatest number of individual calves shedding BVDV-lb (*i*.*e*., maximum shedding day) is determined and all nasal secretion samples from that day are pooled and used to inoculate calves in set two. Nasal secretions are harvested from calves in subsequent groups on this predetermined maximum shedding day. These samples are then assayed for virus isolation and stored frozen until the presence of virus has been confirmed and the samples are pooled and used to inoculate the next set of calves or no further virus is isolated.

Calves are identified by ear tag number and placed on milk replacer for six to eight weeks using general husbandry and care practices standard for the breed.

Outcome variables include clinical signs of BVDV, virus shedding from nasal samples and genetic stability of the virus through five successive *in vivo* passages. All calves are observed for clinical signs of BVDV infection, and the last backpassage group is observed for 21 days after administration of the recovered virus. All clinical signs are recorded and a determination made as to the etiology of the clinical disease.

Nasal secretion specimens are taken from calves; individual aliquots of each sample are maintained for virus isolation and analysis. The samples are processed by sterile filtration through a 0.2-µm filter. Each well of a 12-well plate containing TVL-MDBK (∼80% confluent) is then inoculated with a sample. One well per plate remains uninoculated and serves as the negative control. One well of the plate is inoculated with a dilution of the MS and serves as the positive control. The plates are incubated at 3-7% CO₂ at a temperature of 35-39°C for 5 to 7 days. Samples are sub-cultured onto a secondary plate for an additional 5 to 7 days. Media from wells that exhibit typical BVDV-1b cytopathic morphology are further processed to determine the identity of the infectious agent by PCR assay.

The genetic stability of the Master Seed is analyzed by comparing the highest passage BVDV recovered from a calf to the Master Seed. The lack of shedding from all ten calves in set one of the study, or the isolation of the virus which has not reverted to virulence (as demonstrated by a lack of definitive clinical signs of BVDV) is indicative of a successful backpassage study, and genetic stability of the MS.

### EXAMPLE 4 - PREPARATION OF A HEXAVALENT BRDC VACCINE (MODIFIED, LIVE VIRUS)

The present Example describes the preparation of a multivalent (6-way) BRDC vaccine incorporating the BVDV-1b MS described above.

### MATERIALS AND METHODS

### MICROORGANISMS USED

BHV-1: The Cooper (Colorado) isolate of BHV was isolated in 1956 from lungs of a bovid that had upper respiratory tract disease (York *et al.,* 1957). This Master Seed Virus was identified as **TVL-BHV (Cooper) PO, August 5,1997 DW-1-21-W.**

BVDV-1a: The Singer strain of BVDV-la was obtained from APHIS. This Master Seed was identified as **TVL-BVD (Singer) P0, Dec 1998 DW4-29**.

BVDV-1b: The TGAC strain of BVDV-lb was obtained from APHIS. This Master Seed was identified as **TVL-BVDV 1b MS 04/27/2007 DW3-095**.

BVDV-2: The 125 strain of BVDV-2 was obtained from APHIS. This Master Seed was identified as **TVL-BVDV 2 Strain 125 P0 11/01/01 DW3-90**.

PI₃: The Reisinger SF4 strain of PI₃ was obtained from APHIS. This Master Seed was identified as **TVL**- PI₃ **(Reisinger SF-4P0, 2 April 2001 (SM-83)**.

BRSV: The N375 strain of BRSV was obtained from APHIS. This Master Seed was identified as **TVL-BRSV P0 July 20, 2001 DW3-87**.

### PROTOCOL

Prior to inoculation of TVL-BK cells with production seed virus, the cells were microscopically visualized to confirm that the cells were displaying the morphology previously described. Prior to harvesting each batch of production virus, TVL-BK cells were observed to confirm that they are exhibiting typical cytopathological effects (CPE) associated with the virus.

The consistency of the virulence, potency, and antigenicity of the BHV-1, BVDV-1a, BVDV-1b, BVDV-2, PI₃, and BRSV used in this vaccine were fostered by storage in the lyophilized or frozen condition, and restrictions on the number of passages or subcultures.

Production virus = MSV+10 (efficacy serial passage), but may be any passage between 5 and 10. Production cell stock = MCS+20 (efficacy serial passage), but may also be any passage below 20.

Working virus inocula (MSV+1 to MSV+8) and production virus inoculum (which was typically, but not exclusively, at MSV+9), were typically propagated in TVL-BK cells; provided, the passage number of TVL-BK cells for virus propagation does not exceed about 20 passages from MCS.

The growth medium for working and production cell preparations was DMEM containing 5-10% equine serum. MSV was prepared and stored as described above.

Culture vessels were seeded by splitting actively propagating cultures at a ratio ranging from 1:3 to 1:6 (cm²:cm²). TVL-BK in flask or roller bottles (which were intended for subculture) were released from the culture surface by aseptically removing the growth medium and adding 1X trypsin-EDTA solution as described in Example 1. After incubation for about 5 min at 35 to 39°C, the monolayer was dispersed. Trypsin was inactivated by adding the dispersed cells into a culture vessel with growth medium.

Frozen aliquots of virus were rapidly thawed or suspended in medium if seed is lyophilized. TVL-BK cells (+20 or less) were inoculated with virus.

Virus that were replicating in flasks or roller bottles, which was intended for subculture, were collected after the appropriate incubation time and/or CPE had been observed. The viral fluids could be stored from 6°C to -80°C or be used immediately to inoculate other culture vessels. Standard cell culture techniques were used to inoculate both seed and production media. TVL-BK cells at passage 20 or less were inoculated. Frozen production virus inoculum, which was typically at MSV+9, was rapidly thawed. The volume of virus inoculum typically ranged from 1 to 25 mL per 1600 cm² of culture area to achieve a multiplicity of infection ranging from 0.01 to 1 virus per cell.

The minimum inocula titer were as follows: 10^{5.5} TCID₅₀ per mL for BHV, 10^{5.0} TCID₅₀ per mL for BVDV-la, 10^{5.0} TCID₅₀ per mL for BVDV-lb, 10^{5.0} TCID₅₀ per mL for BVDV-2, 10^{6.0} TCID₅₀ per mL for PI₃ virus, and 10^{4.5} TCID₅₀ per mL for BRSV. Inoculated cultures were incubated at 37 ± 2°C and 5 ± 1% CO₂. BHV and PI₃ were incubated for 2 to 4 days, BVDV-la, BVDV-lb, and BVDV-2 for 4 to 6 days, and BRSV for 5 to 8 days.

### HARVEST

On the day of harvest, the culture was examined for virus-specific CPE and sterility. The minimum incubation time was 2 days after inoculation with BHV or PI₃, 4 days after inoculation with BVDV-1a, BVDV-1b or BVDV-2 and 5 days after inoculation with BRSV. The maximum incubation time was 4 days after inoculation with BHV or PI₃, 6 days after inoculation with BVDV-1a, BVDV-1b, or BVDV-2, and 8 days after inoculation with BRSV.

The virus suspensions were aseptically harvested from production vessels. Samples were collected to determine the TCID₅₀ of the harvest. The harvest material was stored at a temperature of 6°C to -80°C. Harvest material could be stored above freezing for up to one month and frozen for up to six months prior to fabrication of final product.

All manipulations were conducted using standard aseptic techniques. Neomycin and nystatin (Mycostatin®, Bristol-Myers Squibb, New York, NY, USA) were added during the assembly of a serial or sub-serial vaccine at a rate of 15 µg and 15 Units/dose, respectively.

The harvest fluids were diluted in DMEM and stabilized by the addition of a 0.2-µm filtered sucrose solution, which results in a final sucrose concentration of 10 ± 1%. Virus harvest fluids could be concentrated by sterile ultrafiltration using a 10-kDa molecular weight cutoff filter. The degree of concentration typically did not exceed 50-fold.

Batches of harvested viral fluids were analyzed to determine the TCID₅₀ (*e*.*g*., Spearman-Karber Method).

### EXAMPLE 5 - EFFICACY STUDIES FOR HEXAVALENT BRDC VACCINE (MODIFIED, LIVE VIRUS)

Examples 5 through 10 demonstrate the effectiveness of the hexavalent BRDC modified, live vaccine in preventing disease caused by each of the viruses contained therein. In this first example, the vaccine is shown to be effective in preventing disease caused by BVDV-1b.

### MATERIALS

The hexavalent vaccine (modified, live virus), was prepared in accordance with the production method described above. Briefly, BHV-1 (Cooper strain), BVDV-1a (Singer strain), BVDV-1b (TGAC Strain), BVDV-2 (125), PI₃ (Reisinger SF-4), and BRSV (N375) were propagated in the TVL-BK cell line (20^{th} passage). Individual fractions were harvested at the 10^{th} passage and blended together into the six-way product.

Delete placebos were made alongside each of the efficacy serials. For example, the BVDV-delete placebo contained the same BRSV, BHV, and PI₃ harvest batches as the corresponding efficacy serial. Culture medium was substituted for the BVDV component to maintain equivalent volume between efficacy serial and BVDV delete placebo.

Similar delete placebos were constructed for each component of the hexavalent vaccine MLV.

### EXPERIMENTAL METHODS

For each study, commercial stocker/feeder calves approximately 3-4 months of age were acquired, randomized, and identified by ear tag. These calves were de-wormed and vaccinated against pasteurellosis, mycoplasmosis, and blackleg. Calves in the vaccinate and control groups were maintained in separate, non-adjacent but equivalent pens until two days prior to challenge (Day -2) at which time they were commingled in one pen.

All calves were challenged on Day 0 with a virulent isolate of the virus of interest. For example, virulent BVDV-1b (obtained from the lungs of a pen-dead calf at Poky Feeders [Scott City, KS, USA]) was passed *in vitro* on TVL-BK cells using DMEM with 10% equine serum. A challenge dose of approximately 8 × 10⁷ TCID₅₀ in 4 mL was administered to each calf, 2 mL into each nasal passage during inspiration. Calves had free-choice access to water, coastal Bermuda hay and a mixed feed ration containing a coccidiostat, but no antibiotic.

### RANDOMIZATION

A matched randomization scheme was used to allocate calves into two treatment groups (vaccinate or placebo-vaccinated control) using a randomization table provided by CVB-Biometrics (See Table 1.) A sequence of three calves entering the chute was randomized into the two treatment groups in a 2:1 ratio. Ear tags, randomly drawn from a bucket at the time of initial blood draw for serological evaluation, identify the calves. The two groups of calves were maintained in separate but equivalent pens prior to challenge. Calves were commingled in one pen, two days prior to challenge (day -2).

### BLINDING

Blinded personnel did not have knowledge of the identity of the controls or vaccinates. All field and laboratory personnel, with the exception of the record keeper, were blinded to the study.

### OUTCOME

The selected primary outcome was leukopenia after challenge. Leukopenia was defined as a 25% decrease from baseline WBC counts. Other outcomes further supporting the efficacy of the vaccine included nasal shedding, viremia, pyrexia, antibody production and clinical signs of viral infection.

### ESTIMATOR

Individual white blood cell (WBC) counts were converted into a percentage of the pre-challenge average and measured on a continuous interval scale. These converted WBC count percentages were determined daily for at least 14 days post-challenge and serve as the primary criteria for evaluation. The detection of leukopenia served as the primary criteria for determining resistance (unaffected) or susceptibility (affected) to challenge.

The affected and unaffected group was compared to determine if vaccination prevented leukopenia after a viral challenge exposure. Clinical signs of respiratory disease and the detection of virus from nasal/buffy coat samples served as secondary criteria for determining susceptibility to challenge. The duration of leukopenia and duration of shedding (number of days virus was detected from an individual calf) was analyzed to determine if the vaccination had a mitigating effect on these parameters. All individual daily rectal temperatures were analyzed using the pre-challenge average temperature (baseline) as a covariate. Experimental units were excluded from the study if they had a viral titer of ≥2 at the initiation of the study.

### SAMPLING FRAMES AND METHODS

Blood samples were collected from calves immediately prior to vaccination (day -28 to - 21) with efficacy serial or the corresponding delete placebo. Blood samples were also collected immediately prior to challenge (Day 0) and again 14 days post challenge (Day 14). Blood samples for differential WBC counts and virus isolation were collected on day -2 through at least day 14. Samples were collected in EDTA Vacutainer® (Becton-Dickinson and Co., Franklin Lakes, NJ, USA), tubes and counts performed using a Drew Scientific, Hemavet 950 differential WBC counter. Nasal swabs were taken for virus isolation on day -2 through at least day 14.

### OBSERVATIONS AND DATA COLLECTION

Nasal samples and blood samples were collected and calves observed for clinical signs of viral infection on Days 2 through 14. Rectal temperatures were determined for each calf on Day -2 through Day 14.

The WBC count data was analyzed to determine reductions in the post-challenge count. A pre-challenge mean was calculated for each animal by averaging WBC counts for

Day -2 through Day 0. The ratio of daily WBC counts relative to the pre-challenge mean was calculated (WBC count as a proportion of pre-challenge) for each post-challenge day 1-14 or longer at the discretion of the observers. If the proportion decreased by 25%, the individual was classified as leukopenic. Nasal swabs were taken from each calf on the day of challenge and for 14 consecutive days post-challenge by swabbing both nares with a BBL Culture Swab® with Liquid Stuart Media. One the day of collection, the samples were processed by sterile filtration through a 0.2-µm filter. Each well of a 12-well plate containing TVL-MDBK (∼80% confluent) was then inoculated with a sample. One well per plate remained uninoculated and served as the negative control. One well of the plate was inoculated with a dilution of the challenge virus and served as the positive control.

The plates were incubated at 3-7% CO₂ at a temperature of 35-39°C for 2 to 4 days. Media from each well was harvested and stored at -18°C to -22°C. Media from all wells was assayed by RT-PCR (Ridpath and Bolin 1998) to determine if a particular virus was present in the culture. Individuals that were culture-positive for the virus were classified as "shedding".

Buffy coats from blood samples were used to inoculate each well of a 12-well plate containing TVL-MDBK (∼80% confluent). One well per plate remained uninoculated and served as the negative control. One well of the plate was inoculated with a dilution of the challenge virus and served as the positive control. The plates were incubated at 3-7% CO₂ at a temperature of 35-39°C for 2 to 4 days. Media from each well was harvested and stored at -18 to -22°C. Media from all wells was assayed by RT-PCR (Ridpath and Bolin, 1998) to determine if virus was present in the culture. Individuals that cultured positive for a particular virus were classified as "viremic".

Daily rectal temperatures for each animal were recorded between Day --2 and Day 14 (2 days' prior to challenge, the day of challenge and 14 days' post-challenge) of the study.

A pre-challenge mean was calculated for each animal by averaging temperature values for Day -2 through Day 0. The daily temperatures were analyzed using baseline as a covariate.

**BVDV-1b Antibody Titers:** In the case of BVDV-lb efficacy, serum neutralization antibody titers against BVDV-1b were determined by the constant virus decreasing serum method for each animal on the day of initial vaccination (day -21), the day of challenge (day 0), and at the conclusion of the study. Antibody titers were determined using a modification of Special Outline A-17, Titration of Bovine Viral Diarrhea Virus Type 1 Neutralizing Antibody. The modified assay used a cytopathic BVDV-1b isolate as the indicator virus instead of a BVDV-1a isolate. Animals that develop an antibody titer ≥8 were considered as sero-converted to BVDV-1b.

The differences in the duration of leukopenia, shedding, and viremia between the vaccinate and control group were estimated (mitigated fraction). Rectal temperatures were analyzed using the pre-challenge average temperature (baseline) as a covariate. The selected confidence interval was 95%.

**Leukopenia:** Analysis of the leukopenia data was based on the proportion of vaccinates that were leukopenic as compared to the proportion of placebo-vaccinated controls that were leukopenic after viral challenge. This was evaluated by determining the preventable fraction as described by Tanner and Morgan (1993).

**Shedding and Viremia:** Analyses of the shedding and viremia data are based on the proportion of vaccinates that shed virus or are viremic as compared to the proportion of placebo vaccinated controls that shed or are viremic after viral challenge. These were evaluated by determining the preventable fraction as described by Tanner and Morgan (1993).

The median difference in duration of leukopenia between the two groups was estimated and the mitigated fraction was calculated according to Fergen (2004). The median difference in duration of shedding and the median difference in duration of viremia between the two groups are estimated and the mitigated fractions calculated according to Fergen (2004).

**Clinical Observations:** Analysis of rectal temperatures for days 1-14 was conducted with a repeated measures analysis of variance (ANOVA) including terms for Group, Day and Group*Day interactions using the baseline as a covariate. Where Group*Day interactions were significant (*p* < 0.5), the vaccinate group was compared to the control through the simple effect of Group for each time point. These simple effect comparisons were obtained from the Group*Day interactions.

**Statistical Analyses:** Group comparisons of the antibody titers were analyzed with the Mann-Whitney U Test. All statistical analyses were performed using SAS Learning Edition v2.0 for MS-Windows® as described above.

### EXAMPLE 5A - EFFICACY STUDIES FOR HEXAVALAENT BRDC VACCINE

The results of this study demonstrate that the modified live Bovine Viral Diarrhea Virus - Type 1b (BVDV1b), Bovine Rhinotracheitis - Virus Diarrhea - Parainfluenza₃ - Respiratory Syncytial Virus Vaccine, Modified Live Virus, is antigenic and efficacious as an aid in the prevention of disease caused by BVDV1b. One dose of the vaccine was administered to twenty-one BVDV sero-negative calves. Ten sero-negative control calves were vaccinated with one doses of a product matched-placebo vaccine. All calves were challenged intra-nasally with BVDV1b. Vaccination resulted in an increased BVDV1b antibody titer and either prevented or reduced BVDV1b induced viremia, nasal shedding, pyrexia, clinical signs of disease and leukopenia. Consequently, a minimum BVDV1b-TCID₅₀ of 2 X 10³ per dose has been established for the BVDV1b fraction of this vaccine. This vaccine contains BHV-1, PI3, BRSV, two subgenotypes of BVDV Type 1 (1a and 1b) and BVDV Type 2.

**Vaccination, Challenge and Sample Collection:** Thirty (31) BVDV negative calves were commingled in a sorting ally. Each calf was gate cut as they were randomly herded, three calves at a time, through the alley into one of two groups (Group A - vaccinates or Group B - placebo vaccinated controls) by using a matched-set randomization scheme.

Twenty-one calves were vaccinated with one dose of Bovine Rhinotracheitis - Virus Diarrhea - Parainfluenza₃ - Respiratory Syncytial Vaccine, Modified Live Virus. Each vaccinate was given a 2 ml dose of the product by subcutaneous injection in the left side of the neck on day 0. Ten calves were vaccinated with a product matched placebo vaccine (BVDV delete). Two days prior to challenge both vaccinate and control groups were commingled for the purpose of pre-challenge clinical observation and to acquire baseline body temperature and WBC readings. The vaccinate and control calves were challenged 21 days following vaccination with a virulent isolate of BVDV1b obtained from the lungs of a pen dead calf. The virus had been passed *in vitro* on TVL-BK cells using Dubelcos Modified Eagle's Medium with 10% Equine Sera. A challenge dose of 4X10⁷ TCID₅₀ in 4 mls was administered to each calf, 2 mls into each nasal passage during inspiration.

Blood samples for determining antibody titers were collected prior to vaccination, the day of challenge (21 days post-vaccination), and 14 days post-challenge. Bovine Viral Diarrhea Virus antibody titers were determined by the constant virus-decreasing serum neutralization method.

Nasal swabs for virus isolation were collected from calves by swabbing both nares with a BBL Culture Swab® in Liquid Stuart Media on the day of challenge and for 14 consecutive days post-challenge.

Blood samples for buffy coat virus isolation were collected from each calf by venapuncture into an EDTA vacutainer tube on the day of challenge and for 14 consecutive days post-challenge.

Rectal temperatures were recorded daily for each calf for two consecutive days prior to challenge, the day of challenge and for 14 consecutive days post-challenge.

Clinical observations for each calf were made and recorded. The observers were blinded due to both the vaccinate and the control groups being commingled with the only differentiating feature being the ear tag number. At no time throughout the study were the observers given knowledge of the vaccination status of an individual test animal. Laboratory personnel did not have knowledge of the vaccination status of calves.

Blood samples for determining differential WBC counts were collected for two consecutive days prior to challenge, the day of challenge, and 14 consecutive days post-challenge. Samples were collected in EDTA vacutainer tubes and counts performed using a Drew Scientific, Hemavet 950 differential WBC counter.

**BVDV Detection:** Cell culture-enhanced PCR techniques were utilized to detect BVDV1b in collected samples. Briefly, the nasal samples were processed by sterile filtration (0.2µm) on to TVL-BK cells in culture. Buffy coats were placed directly onto TVL-BK cells in culture for 45 - 75 minutes prior to being washed off the cells. The cultures incubated for 4 days at 37°C ± 2 °C and 5 ± 1% CO₂. The supernatant from all culture samples were individually assayed for the presence/absence of BVDV1b using conventional RT qPCR methodology and the following probe/primer set:
Forward Primer : CACCCTATCAGGCTGTATTCATAGC (SEQ ID NO:1)
Reverse Primer: TGCCCACAGCACATCTTAACC (SEQ ID NO:2)
TaqMan MGB Probe: TCACCTGGACGACCC (SEQ ID NO:3)

This method was developed based on *Differentiation of types 1a*, *1b and 2 bovine viral diarrhea virus (BVDV) by PCR*, Julia F. Ridpath and Steven R. Bolin, Molecular and Cellular probes, Journal of Virological Methods, 130 (2005) 145-148). This method is also based on the Center for Veterinary Biologics and National Veterinary Services Laboratories Test Protocol - Genotyping Bovine Viral Diarrhea Virus, Number BPPR02010.01. Both of these methods of differentiating genotypes and subgenotypes of BVDV exploit the genetic differences that exists in the 5' Untranslated Region (UTR) of the genome. This probe/primer set was specifically designed to amplify a section of the 5' UTR in which a uniquely BVDV1b sequence can be detected by the highly specific probe. The specificity of the probe/primer set was confirmed in house and did not cross react with BHV,PI3, BRSV, BVDV la, or BVDV2. The RT qPCR assays were performed using Taqman® based assays on an Applied Biosystems 7500.

**Statistical Analysis:** Antibody Titers: Group comparisons of the ordinally scaled titers were analyzed with the Mann-Whitney U Test.

**Viremia:** Virus isolation data from buffy coats of blood samples was analyzed based on the proportion of vaccinates that BVDV1b was isolated from as compared to the proportion of controls that BVDV1b was isolated from post-challenge. The preventable fraction for BVDV1b viremia was calculated as described by Tanner and Morgan (1993).

**Nasal Shedding:** Virus isolation data from nasal swab analysis is based on the proportion of vaccinates that BVDV1b was isolated from as compared to the proportion of controls that BVDV1b was isolated from post-challenge. The preventable fraction for BVDV1b shedding was calculated as described by Tanner and Morgan (1993).

**Rectal Temperature:** A pre-challenge average rectal temperature was determined for each animal. This average was used as a covariate in a repeated measures analysis of variance (ANOVA) which included terms for Group, Day and Group*Day interactions.

**Clinical Signs of Disease:** Clinical signs of disease were recorded daily by each observer independently. Any calf exhibiting signs for at least one post-challenge observation day was considered "affected." The preventable fraction for clinical signs was calculated as described by Tanner and Morgan (1993).

**Leukopenia:** A pre-challenge leukocyte mean was calculated for each animal by averaging leukocyte counts for Days -2 through 0. The ratio of daily leukocyte counts relative to the pre-challenge mean was calculated (leukocyte count as a proportion of pre-challenge) for each post-challenge day 1-14. This proportion data was analyzed to determine if individual animals developed a leukopenia as defined by a 40% decrease in counts from baseline. The preventable fraction was calculated as described by Tanner and Morgan (1993) to determine if vaccination prevented the development of leukopenia in this study.

All statistical analysis was performed using SPSS version 16 for Windows.

### RESULTS

**Serum neutralization antibody titers:** All calves in this study had a pre-vaccination BVDV (BVDV1a, BVDV1b and BVDV2) serum neutralization antibody titer of <1:2. By the day of challenge (Day 21), the vaccinate group had an average BVDV1b titer of 1:83 which was significantly (p≤.05) greater than that of the control group < 1:2. The BVDV1b antibody titers increased 14 days post-challenge in both the control and vaccinate calves indicating that the calves had been challenged with BVDV1b.

**BVDV1b Isolation from Buffy Coats:** All ten control calves in this study were viremic during the post-challenge phase of this study. However, seven of the control calves were viremic during the critical time frame that BVDV post-challenge viremia is typically observed. Three control calves (1, 14, 30) were viremic on the day of challenge. These same three calves along with calf 26 were viremic the day following challenge. This observation along with the serological, nasal swab isolation and rectal temperature data suggest these calves were naturally infected late in the vaccination phase of the study, became viremic, and began shedding early in the challenge phase of this study. One of these four calves (calf 30) became viremic and shed virus during the typical post-challenge viremia/shedding period. None of the 21 vaccinates were viremic during the post challenge period. A prevented fraction was calculated using a ratio of 3/10 controls naturally protected and 21/21 vaccinates protected. This results in a Preventable Fraction of 1.0.

**BVDV1b Isolation from Nasal Swabs:** All ten control calves in this study shed BVDV1b during the post-challenge phase of this study. However, seven of the control calves were shedding during the critical time frame that BVDV post-challenge shedding is typically observed. Two control calves (26 and 30) were shedding on the day of challenge. Three control calve (1, 14 and 30) were shedding the day following challenge.

This observation along with serological, buffy coat isolation and rectal temperature data suggest these calves were naturally infected late in the vaccination phase of the study, became viremic, and began shedding early in the challenge phase of this study. One of these four calves (calf 30) became viremic and shed virus during the typical post-challenge viremia/shedding period. Only 1 of the 21 vaccinates shed BVDV1b during the post challenge period. A prevented fraction was calculated using a ratio of 3/10 controls naturally protected and 20/21 vaccinates protected. This results in a Preventable Fraction of 0.93.

**Rectal Temperatures:** The analysis of the data indicated a significant Group*Day interaction (p=.034). Analysis by day indicated that temperatures in the control group were significantly (p<0.05) greater than that of the vaccinate group on days 1,4,7 and 8 post-challenge. On day 12 the rectal temperatures of the control group had dropped and were significantly (p<0.05) less than that of the vaccinate group.

**Clinical Signs of BVDV1b:** Eight of ten (8/10 Affected) calves in the control group demonstrated clinical signs that could be contributed to BVDV1b infection. Clinical signs included diarrhea, copious amounts of clear nasal discharge, rapid respiration and ocular discharge. None (0/21 Affected) of the calves in the vaccinate group demonstrated any of these clinical signs during the post-challenge phase of the study. A prevented fraction was calculated using a ratio of 2/10 controls naturally protected and 21/21 vaccinates protected. This results in a Preventable Fraction of 1.0. No adverse post-vaccination reactions were observed in any of the calves.

**Leukopenia:** Leukopenia was detected in four of the 21 vaccinates and five of ten controls resulting in a preventable fraction of 62%.

### CONCLUSIONS

The preceding demonstrates the antigenicity and efficacy of the BVDV1b fraction of Bovine Rhinotracheitis - Virus Diarrhea - Parainfluenza₃ - Respiratory Syncytial Virus Vaccine, Modified Live Virus as an aid in the prevention of disease caused by BVDV1b.

The antigenicity and the efficacy of the BVDV1b fraction of this combined product is attested to by the following:
1) A significant increase in antibody titers to BVDV1b in the vaccinate group as compared to the control group. All calves in the vaccinate group developed BVDV1b titers ≥ 1:8 after administration of one, 2ml, dose, meeting the requirements defined in 9CFR 113.311 (c) (5).
2) Virus isolation studies revealed that the administration of one dose resulted in a significant decrease in post-challenge BVDV1b induced viremia and nasal shedding.
3) Vaccinated calves exhibited significantly less fever when compared to control calves and showed no clinical signs of disease.
4) Vaccination also decreased the probability of developing a BVDV1b induced leukopenia.

This study was marginally affected by the fact that at least three of the calves in the control group became infected with BVDV1b shortly prior to challenge. Due to the fact that all control calves, including those that were infected, were still sero-negative at the time of challenge, it was concluded that the natural exposure occurred late in the vaccination phase of the study. There was no clinical evidence of natural exposure in the vaccinate group probably due to being maintained separately from the control group until 2 days prior to challenge. Natural exposure is an inherent possibility when conducting challenge studies in a typical stocker/feeder calf environment. According to Tanner and Morgan, the estimate of the vaccine's efficacy as determined by the preventable fraction considers the relationship between the observed protection and vaccine effectiveness for several levels of natural immunity.

The study's findings are both statistically significant and clinically relevant, therefore, demonstrating the efficacy of the BVDV1b fraction contained in the vaccine.

### EXAMPLE 6 - BRSV EFFICACY PROTOCOL

This example demonstrates that the bovine respiratory syncytial virus (BRSV) fraction of the hexavalent MLV vaccine aids in the prevention of disease caused by BRSV.

### MATERIALS AND METHODS

A BRSV delete placebo is prepared and used alongside the efficacy serial in a manner analogous to that described in Example 5 for BVDV-lb. This BRSV delete placebo contains the same BVDV-la, BVDV-lb, BVDV-2, BHV and PI₃ harvest batches as the efficacy serial, with culture media substituting for the BRSV component to maintain equivalent volume between efficacy serial and BRSV delete placebo. Sterile diluent is used to rehydrate both the hexavalent MLV vaccine and the BRSV delete placebo as described herein.

### EXPERIMENTAL METHODS

Commercial stocker/feeder calves approximately 3-4 months from a relatively homogenous population having approximately the same origin, type, weight and age are used. Calves are de-wormed and vaccinated against pasteurellosis, mycoplasmosis, and blackleg. Equal numbers of vaccinate and control calves are used in the protocol; the two groups of calves are maintained in separate, non-adjacent but equivalent pens prior to challenge, and commingled in one pen, two days prior to challenge (day -2). Calves have free-choice access to water, coastal Bermuda hay and a mixed feed ration containing a coccidiostat, but no antibiotic.

### RANDOMIZATION

A matched randomization scheme is used to allocate calves into two treatment groups (vaccinate or placebo-vaccinated control) using a CVB-Biometrics randomization table, in which a sequence of three calves entering the chute is randomized into the two treatment groups in a 2:1 ratio. Ear tags, randomly drawn from a bucket at the time of initial blood draw for serological evaluation, identify the calves.

### BLINDING

All personnel are blinded to the study (with the exception of the record keeper), and do not have knowledge of the identity of the controls or vaccinates.

### OUTCOME

The primary outcome is the prevention of nasal shedding of BRSV virus after challenge, which is determined by the presence or absence of BRSV in nasal samples collected daily from the nares of all calves during the protocol. Other secondary outcomes may include one or more decreased clinical signs of BRSV infection, reduction of pyrexia, and/or reduction of the duration of shedding in the calves shedding BRSV.

Shedding of BRSV by the vaccinate and control groups is determined daily for 14 days post-challenge, with detection of BRSV serving as the primary criterion for determining resistance (unaffected) or susceptibility (affected) to challenge. The affected and unaffected groups are compared to determine if vaccination prevented the shedding of BRSV after a BRSV challenge exposure. Clinical signs of respiratory disease serve as a secondary criterion for determining susceptibility or resistance to challenge. The duration of shedding (number of days BRSV was detected from an individual calf) is analyzed to determine if the vaccination had a mitigating effect on this parameter. All individual daily rectal temperatures are analyzed against the pre-challenge average temperature (baseline) covariate. Experimental units are excluded if they have a BRSV SN titer of >2 at the initiation of the study.

### SAMPLING FRAMES AND METHOD

Blood samples are collected from calves immediately prior to vaccination (day -28 to day -21) with efficacy serial or BRSV-deleted placebo, and again immediately prior to challenge (Day 0) and again 14 days' post-challenge (Day 14).

### OBSERVATIONS AND DATA COLLECTION

Nasal swabs are taken from each calf on the day of challenge and for 14 consecutive days post-challenge by swabbing both nares with a BBL Culture Swab® with Liquid Stuart Media. The media from the samples is assayed for the presence of BRSV by real-time PCR using a probe/primer set specifically for detecting BRSV.
Forward primer: 5'-GCAATGCTGCAGGACTAGGTATAAT-3' (SEQ ID NO:4);
Reverse primer: 5'-ACACTGTAATTGATGACCCCATTCT-3' (SEQ ID NO:5); and
BRSV TaqMan® MGB probe: 5'-AAGACTTGTATGATGCTGCCAA-3' (SEQ ID NO:6).

Duration data is analyzed to demonstrate that vaccination had a mitigating effect on the duration of time BRSV was detected in nasal samples.

**Clinical Observations:** Daily rectal temperatures for each animal are recorded between Day -2 and Day 14 (2 days prior to challenge, the day of challenge and 14 days post-challenge), with a pre-challenge mean being calculated for each animal by averaging temperature values for Day -2 through Day 0. The daily temperatures are then analyzed using baseline as a covariate.

Serum-neutralization antibody titers are determined by the constant virus decreasing serum method for each animal on the day of vaccination, Day 0 and Day 14 of the study (Schefers *et al.,* 2008).

Clinical signs of BRSV in beef calves have previously been described by Baker (1986). Clinical signs include, but are not limited to, nasal and lacrimal discharge, increased respiratory rate, elevated rectal temperature, mild depression, decreased feed intake, hypersalivation and dyspnea. Clinical signs of BRSV infection are less obvious in traditional challenge models because the virus used to challenge has been attenuated by propagation *in vitro,* and the only important parameter in evaluating these models is virus shedding (Wren, 2001). Consequently, clinical signs are recorded two days prior to challenge, the day of challenge and for fourteen days post challenge. The observation of clinical signs of BRSV is taken into consideration during data analysis, but not considered as a primary outcome because these signs can be indicative of many respiratory diseases commonly found in commercial cattle.

The proportion of vaccinate and placebo vaccinated controls that are classified as either affected or unaffected is estimated (prevented fraction), as is the difference in the duration of shedding between the vaccinate and control group (mitigated fraction).

The efficacy of the BRSV virus fraction of the hexavalent MLV vaccine may readily be evaluated by determining the preventable fraction as described by Tanner and Morgan (1993). This analysis is based on the proportion of vaccinates that are resistant to the BRSV challenge as compared to the proportion of placebo vaccinated controls that are resistant.

**Statistical Analyses:** The median difference in duration of shedding between the two groups can be estimated and the mitigated fraction calculated according to the method of Fergen (2004). Group comparisons of the antibody titers may be analyzed with the Mann-Whitney U Test. All statistical analyses were performed using SAS Learning Edition 2.0 for Microsoft Windows® as described above.

### Example 6A - BRSV Efficacy Study

The results of this study demonstrate that the modified live Bovine Respiratory Syncytial Virus (BRSV) fraction of Bovine Rhinotracheitis - Virus Diarrhea - Parainfluenza₃ - Respiratory Syncytial Virus Vaccine, Modified Live Virus, is antigenic and efficacious as an aid in the prevention of shedding of Bovine Respiratory Syncytial Virus in stocker/feeder calves. One dose of the vaccine was administered to twenty-two BRSV sero-negative calves. Eleven sero-negative control calves were vaccinated with one dose of a product matched placebo vaccine. All calves were challenged intra-nasally with BRSV. Vaccination resulted in an increased BRSV antibody titer and prevented BRSV shedding after challenge. Consequently, a minimum BRSV-TCID₅₀ of 7X10² per dose has been established for the BRSV fraction of this vaccine.

### MATERIALS AND METHODS

**Vaccination and Challenge of Calves**: Thirty-three (33) BRSV negative calves were commingled in a sorting ally. Each calf was gate cut as they were randomly herded, three calves at a time, through the alley into one of two groups (Group A - vaccinates or Group B - placebo vaccinated controls) by using a matched-set randomization scheme provided by CVB-Biometrics.

Twenty-two of the serologically negative calves were vaccinated with one dose of Bovine Rhinotracheitis - Virus Diarrhea - Parainfluenza₃ - Respiratory Syncytial Vaccine, Modified Live Virus Vaccine. Each vaccinate was given a 2 ml dose of the product by subcutaneous injection in the left side of the neck on day 0. Eleven of the calves were vaccinated with a product matched placebo vaccine (BRSV delete). Nasal swabs were collected, calves were bled, and screened serologically to confirm susceptibility to BRSV by a constant virus decreasing serum neutralization assay. Two days prior to challenge both vaccinate and control groups were commingled for the purpose of pre-challenge clinical observation. The vaccinate and control calves were challenged 32 days post vaccination with 4 ml (2ml per nostril) of TVL-BRSV, N375 isolate. The challenge virus was titered immediately prior to challenge (TCID₅₀ of 10^{6.6}/ ml) and again immediately after challenge (TCID₅₀ of 10^{6.2} / ml). The challenge virus was kept chilled throughout the challenge process.

Blood samples were collected the day of vaccination, the day of challenge and 14 days post-challenge. Bovine Respiratory Syncytial Virus antibody titers were determined by the constant virus-decreasing serum neutralization method.

Nasal swabs were collected the day of vaccination, the day of challenge and for 14 consecutive days post-challenge.

Rectal temperatures were recorded daily for each calf for two days prior to challenge, the day of challenge and for 14 consecutive days post-challenge.

Clinical observations were made. The observers were blinded due to both the vaccinate and control groups being commingled with the only differentiating feature being the ear tag number. At no time throughout the study were the observers given knowledge of the vaccination status of an individual test animal. Laboratory personnel did not have knowledge of the vaccination status of calves.

**BRSV Detection**: Nasal swabs were collected from calves by swabbing both nares with a BBL Culture Swab® in Liquid Stuart Media. Visual observation of the BRSV specific CPE along with Cell culture-enhanced PCR techniques were utilized to detect BRSV in collected samples. Briefly, the samples were processed by sterile filtration (0.2µm) on to TVL-BK cells in culture. The cultures incubated for 5 days at 37°C ± 2 °C and 5 ± 1% CO₂. The cultures were observed for BRSV specific CPE and results were recorded. The supernatant from all culture samples (both + and - CPE) were individually assayed for the presence/absence of BRSV using the probe/primer set described by M. Boxus *et.al.* (Real Time RT-PCR for the detection and quantitation of bovine respiratory syncytial virus, M. Boxus, C. Letellier, P. Kerkhofs, Journal of Virological Methods, 125 (2005) 125-130).

The specificity of the probe/primer set was confirmed in house and did not cross react with BHV, PI3, BVDV1a, BVDV1b or BVDV2. The RT qPCR assays were performed using Taqman® based assays on an Applied Biosystems 7500.

All samples that were BRSV specific CPE positive were also PCR positive. There were 10 samples that were negative for BRSV specific CPE and positive for BRSV by the PCR assay. This is consistent with in house studies indicating that PCR based assays are more sensitive than assays based on the observation of BRSV specific CPE. Samples that were negative for BRSV after the primary culture were subcultured and the supernatant from subcultured fluids were assayed for the presence/absence of BRSV according to the same method.

**Statistical Analysis:** The efficacy of the BRSV fraction of this vaccine was primarily based on the proportion of vaccinates that shed BRSV as compared to the proportion of controls that shed BRSV post-challenge. The preventable fraction was calculated as described by Tanner and Morgan (1993). The number of days post-challenge that BRSV was isolated from individual calves in the study was evaluated. Groups were compared to determine if the frequency of the BRSV isolation (shedding) was significantly reduced in the vaccinate group as compared to the control group after BRSV challenge.

Group comparisons of the ordinally scaled titer data was compared by the Mann-Whitney U Test. Rectal temperatures were analyzed using the pre-challenge temperature average as a covariate. Analysis of post-challenge temperature for day 1-14 was conducted with a repeated measures analysis of variance (ANOVA). All statistical analysis was performed using SAS for Windows.

### RESULTS

**Serum neutralization antibody titers**: All calves in this study had a pre-vaccination serum neutralization antibody titer of <1:2. Post vaccination serum neutralization antibody titers were significantly (p≤.05) greater in the vaccinate group as compared to those of the control group. Twenty one of 22 vaccinated calves showed an increase in BRSV titers following vaccination, whereas, all eleven placebo-vaccinated controls remained negative throughout the vaccination period prior to challenge. The average antibody titer of the vaccinate group was 8.5 while the average titer of the control group was <2.

**BRSV Isolation**: All eleven control calves in this study shed BRSV during the post-challenge phase of this study. Only 3 of the 22 vaccinates shed BRSV during this time. This results in a Preventable Fraction of 0.8636. There was also a significant difference (p<0.05) in the frequency of shedding. The control group shed BRSV for a mean of 6.00 days while the vaccinates shed for only 0.227 days. This is a difference of 5.773 days.

**Rectal Temperatures**: The post-challenge rectal temperature analysis using the pre-challenge average temperature as a covariate revealed no statistically significant differences between groups. No further analysis of this data was pursued.

**Clinical Signs of BRSV**: Each animal was examined for clinical signs of respiratory disease for two consecutive days prior to challenge, the day of challenge and for fourteen consecutive days post-challenge. No clinical signs that could be definitively attributed to BRSV infection were observed. However, excessive clear nasal discharge was recorded as a clinical sign for 7 of the 11 controls during the same time period the calves were actively shedding BRSV. No clinical signs were recorded for the vaccinate group during the study. No adverse post-vaccination reactions were observed in any of the calves. CONCLUSIONS

The preceding report demonstrates the efficacy of the BRSV fraction of the Bovine Rhinotracheitis - Virus Diarrhea - Parainfluenza₃ - Respiratory Syncytial Virus Vaccine, Modified Live Virus, as an aid in the prevention of shedding of BRSV in stocker/feeder calves.

The efficacy and the antigenicity of the BRSV fraction of this combined product is attested to by the following:
1) Virus (BRSV) isolation studies revealed that the administration of one dose vaccine provided 86% (19/22) protection in vaccinated calves as compared to 100% (11/11) susceptibility in the controls (Preventable Fraction = 0.8636).
2) A significant decrease in the frequency of shedding amongst the vaccinate group as compared to the control group. Control calves shed BRSV for 5.773 days longer than did the vaccinates.
3) A significant increase in antibody titers to BRSV of the vaccinate group as compared to the control group.

### EXAMPLE 7 - IBR (HBV-1) EFFICACY PROTOCOL

This example demonstrates that the BHV-1 viral fraction of the hexavalent MLV vaccine aids in the prevention of IBR.

### MATERIALS AND METHODS

The hexavalent modified, live virus vaccine is prepared as described above. Briefly, BHV-1 (Cooper strain), BVDV-la (Singer strain), BVDV-lb (TGAC Strain), BVDV-2 (125), PI₃ (Reisinger SF-4), and BRSV (N375) are propagated in the TVL-BK cell line (20^{th} pass). Individual fractions are harvested at the 10^{th} passage and blended together into the six-way product.

A BHV-1-delete placebo is made alongside the efficacy serial. The BHV-1 delete placebo contains the same BVDV-1a, BVDV-1b, BVDV-2, PI₃ and BRSV harvest batches as the efficacy serial. Culture media is substituted for the BHV-1 component to maintain equivalent volume between efficacy serial and BHV-1 delete placebo. Sterile diluent is used to rehydrate both vaccine and the BHV-1 delete placebo.

Commercial stocker/feeder calves approximately 3-4 months of age are randomized as described above, then de-wormed and vaccinated against pasteurellosis, mycoplasmosis and blackleg. All calves are given free-choice access to water, coastal Bermuda hay and a mixed feed ration containing a coccidiostat, but no antibiotic. Hay availability may subsequently be restricted after the calves are on feed.

Approximately twenty or more vaccinate and ten or more control calves are used in this single level study. The two groups of calves are maintained in separate but equivalent pens prior to challenge. Calves are then commingled in one pen, two days prior to challenge (day -2).

### RANDOMIZATION

A matched randomization scheme of the calves may be used to allocate calves into two treatment groups (vaccinate or placebo-vaccinated control). For example, a sequence of three calves entering the chute may be randomized into the two treatment groups in a 2:1 ratio. Ear tags, randomly drawn from a bucket at the time of initial blood draw for serological evaluation, are used to identify calves.

### BLINDING

Blinded personnel do not have knowledge of the identity of the controls or vaccinates, and all field and laboratory personnel (with the exception of the record keeper), are blinded for the duration of the study.

### OUTCOME

The primary outcome for this study is a difference in nasal lesions associated with IBR.

This is defined as the presence or absence of nasal lesions. Other clinical signs of IBR including duration and severity of nasal lesions, isolation of BHV-1 from the nares, and body temperature, are all considered as secondary outcomes.

The nasal lesion scores themselves are estimated as ordinal categories. The duration and severity of the lesions in the visible nasal mucosa characteristic of IBR both serve as criteria for evaluation. Clinical signs of respiratory disease and nasal shedding also may be used as criteria for determining resistance (unaffected) or susceptibility (affected) to challenge. Individual daily rectal temperatures are routinely analyzed using the pre-challenge average temperature (baseline) as a covariate.

Experimental units are excluded from the study if they have a BHV-1 SN titer of ≥2 at the initiation of the study.

Blood samples are collected from calves immediately prior to vaccination (day -28 to day -21) with efficacy serial or BHV-1 deleted placebo. Blood samples are also taken immediately prior to challenge (Day 0) and again 14 days post challenge (Day 14).

### SAMPLING FRAMES, DATA COLLECTION, AND CLINICAL OBSERVATIONS

Nasal samples are collected, and calves observed for lesions in the visible nasal mucosa and clinical signs of IBR on Day 0 through Day 14. Rectal temperatures are determined for each calf on Day -2 through Day 14.

Outcome variables include lesions of the visible nasal mucosa consistent with IBR, rectal temperatures, virus isolation, antibody titers and clinical signs of IBR.

Gross nasal lesions of IBR have been described by Rosner (1968). Typical lesions observed include inflammation and edema of the nasal passages with hemorrhages and fibronecrotic exudate adherent to the mucus lining. It has been reported by Rosner that necrotic exudate at times is so extensive in the nasal passages that it forms a pseudomembranous exudate that is detached from the underlying tissue. Rosner also reports that these pathologic findings give a high degree of confidence in making a presumptive diagnosis of IBR. Nasal lesion scores may be assigned according to established criteria, and the duration data analyzed to demonstrate whether vaccination had a mitigating effect on the duration that lesions were observed.

Rectal temperature assessment, virus isolation, antibody titers, and statistical methods are as described in previous examples. Clinical signs of IBR have been described by Rosner (1968), and include, but are not limited to, rapid breathing, anorexia, pyrexia, coughing, nasal discharge, a loss of weight and condition. Clinical signs are recorded two days prior to challenge, the day of challenge and for fourteen days post challenge. The observation of clinical signs of IBR may be taken into consideration during the analysis of the data but it is not considered as a primary outcome, because these signs can also be indicative of many other respiratory diseases commonly found in commercial cattle.

***Statistical Analyses:*** Group comparisons of the antibody titers were analyzed with the Mann-Whitney U Test. All statistical analyses were performed using SAS Learning Edition v2.0 for MS-Windows® as described above.

### Example 7a - IBR (HBV-1) Efficacy study

The results of this study demonstrate that the modified live Bovine Herpes Virus- 1(BHV-1) fraction of Bovine Rhinotracheitis - Virus Diarrhea - Parainfluenza₃ - Respiratory Syncytial Virus Vaccine, Modified Live Virus, is antigenic and efficacious as an aid in the reduction of Infectious Bovine Rhinotracheitis (IBR). Two doses of the vaccine were administered to twenty-two BHV-1 sero-negative calves. Twelve sero-negative control calves were vaccinated with two doses of a product matched placebo vaccine. All calves were challenged intra-nasally with BHV-1. Vaccination resulted in an increased BHV-1 antibody titer and a reduction in both the duration and severity of nasal lesions associated with IBR. Vaccination also decreased the duration of shedding and febrile response that typically follows a BHV challenge in calves. Consequently, a minimum BHV-1 TCID₅₀ of 5 X 10³ per dose has been established for the BHV-1 fraction of this vaccine.

### MATERIALS AND METHODS

**Vaccination and Challenge of Calves**: Thirty-four BHV-1 negative calves were commingled in a sorting ally. Each calf was gate cut as they were randomly herded, three calves at a time, through the alley into one of two groups (Group A - vaccinates or Group B - placebo vaccinated controls) by using a matched-set randomization scheme provided by CVB-Biometrics.

Twenty-two calves were vaccinated with one dose of Bovine Rhinotracheitis - Virus Diarrhea - Parainfluenza₃ - Respiratory Syncytial Vaccine, Modified Live Virus. Each vaccinate was given a 2 ml dose of the product by subcutaneous injection in the left side of the neck on day 0. Twelve calves were vaccinated with a product matched placebo vaccine (BHV-1 delete). Nasal swabs were collected for BHV-1 virus isolation and blood was drawn from all calves for serological confirmation of susceptibility to BHV-1. On day 13 the vaccinate and control calves received a second 2 ml dose of the Efficacy Serial and the product matched placebo, subcutaneously, in the right side of the neck. Two days prior to challenge both vaccinate and control groups were commingled for the purpose of pre-challenge clinical observation and to acquire baseline body temperature readings.

The vaccinate and control calves were challenged 15 days following the second vaccination with 4 ml (2ml per nostril) of BHV-1 Challenge Virus provided by the USDA-APHIS-CVB, Coopers strain, Lot Number 05-08, Fill Date- October 26, 2005.

The challenge virus titer was determined by CVB to be 10^{8.2} TCID₅₀/2mL. The challenge virus was titered immediately prior to challenge and determined to be 10^{8.0} TCID₅₀ / 4 mL and again immediately after challenge and determined to be 10^{7.4} TCID₅₀ / 4mL. The challenge virus was kept chilled throughout the challenge process.

Blood samples were collected the day of vaccination, the day of challenge and 14 days post-challenge. Bovine Herpesvirus antibody titers were determined by the constant virus-decreasing serum neutralization method.

Nasal swabs were collected the day of vaccination, the day of challenge and for 14 consecutive days post-challenge.

Rectal temperatures were recorded daily for each calf for two days prior to challenge, the day of challenge and for 14 consecutive days post-challenge.

Clinical observations were made and nasal lesion scores were assigned. Gross nasal lesions caused by BHV have been described by S.F. Rosner, in JAVMA Vol 153, No.12, December 1968, p. 1631-1638. Typical lesions observed include inflammation and edema of the nasal passages with hemorrhages and fibronectrotic exudate adherent to the mucus lining. It has been reported by Rosner that necrotic exudate at times is so extensive in the nasal passages that it forms a pseudomembranous exudate which is detached from the underlying tissue. Rosner also reports that these pathologic findings give a high degree of confidence in making a presumptive diagnosis of IBR.

Nasal lesion scores were assigned according to the criteria listed below:

| Score | Description |
|---|---|
| 0. | Absence of definitive lesions of IBR virus disease. |
| 1. | The presence of lesions characteristic of IBR disease not to exceed 10% of the visible nasal mucus membrane. |
| 2. | The presence of lesions characteristic of IBR disease effecting 11-25% of the visible nasal mucus membrane. |
| 3. | The presence of lesions characteristic of IBR disease effecting 26-50% of the visible nasal mucus membrane. |
| 4. | The presence of lesions characteristic of IBR disease effecting greater than 50% of the visible nasal mucus membrane. |

The observers were blinded due to both the vaccinate and control groups being commingled with the only differentiating feature being the ear tag number. At no time throughout the study were the observers given knowledge of the vaccination status of an individual test animal. Laboratory personnel did not have knowledge of the vaccination status of calves.

**BHV-1 Detection**: Nasal swabs were collected from calves by swabbing both nares with a BBL Culture Swab® in Liquid Stuart Media. Visual observation of the BHV-1 specific CPE along with Cell culture-enhanced PCR techniques were utilized to detect BHV-1 in collected samples. Briefly, the samples were processed by sterile filtration (0.2µm) on to TVL-BK cells in culture. The cultures incubated for 4 days at 37°C ± 2°C and 5 ± 1% CO₂. The cultures were observed for BHV-1 specific CPE and results were recorded. The supernatant from all culture samples (both + and - CPE) were individually assayed for the presence/absence of BHV-1 using conventional RT qPCR methodology and the following probe/primer set:
Forward Primer : CCATGTTAGCGCTCTGGAACC (SEQ ID NO:16)
Reverse Primer: CGTCTTTACGGTCGACGACTCC (SEQ ID NO:17)
TaqMan MGB Probe: ACGGACGTGCGCGAA (SEQ ID NO:18)

The specificity of the probe/primer set was confirmed in house and did not cross react with PI3, BRSV, BVDV1a, BVDV1b or BVDV2. The RT qPCR assays were performed using Taqman® based assays on an Applied Biosystems 7500. All samples that were BHV-1 specific CPE positive were also PCR positive. There were 8 samples that were negative for BHV-1 specific CPE and positive for BHV by the PCR assay. This is consistent with in house studies indicating that PCR based assays are slightly more sensitive than assays based on the observation of BHV-1 specific CPE. Samples that were negative for BHV-1 after the initial culture were subcultured and observed for CPE.

The supernatant from subcultured fluids were assayed for the presence/absence of BHV-1 according to the same method.

**Nasal Lesions**: The primary outcome of the study was differences in nasal lesions associated with BHV-1. The prevented fraction was calculated as described by Tanner and Morgan (1993) based on the presence or absence of lesions in an individual calf. An estimated conditional mitigated fraction of the severity of the lesions in affected calves was determined according to B.J. Fergen, *Estimating an Intervention Effect on Outcome Severity*, USDA, CVB. The duration of lesions (the number of days from the first to the last day that lesions were observed) was determined for each affected calf and the difference in the duration of lesions between the two groups was estimated.

**Body Temperature**: A secondary outcome of the study was differences in body temperature between the vaccinate and control group calves. Analysis of rectal temperatures for days 1-14 were conducted with a repeated measures analysis of variance (ANOVA) including terms for Group, Day and Group*Day interactions and using the baseline temperatures as a covariate. Since Group*Day interactions were significant (p<0.5), the vaccinate group was compared to the control through the simple effect of Group for each time point. These simple effect comparisons were obtained from the Group*Day interactions.

**Virus Shedding**: Another secondary outcome of the study was the difference in post-challenge BHV shedding between the vaccinate and control calves. The prevented fraction was calculated as described by Tanner and Morgan (1993) based on the isolation and identification of BHV in nasal samples from an individual calf. The duration of shedding (the number of days from the first to the last day that BHV-1 was detected) was determined for each affected calf and the difference in the duration of shedding between the two groups was estimated.

All statistical analysis was performed using SPSS version 17 for Windows.

### RESULTS

**Serum neutralization antibody titers**: All calves in this study had a pre-vaccination BHV-1 serum neutralization antibody titer of <1:2. All 12 calves in the control group were still sero-negative on the day of challenge (antibody titer of <1:2). Twenty-one of the 22 calves in the vaccinate group had sero-converted with titers of ≥ 1:2 by the day of challenge (group median titer of 1:4). By 14 days post-challenge all calves had sero-converted to BHV-1 (median vaccinate titer >256, median control titer 128).

**Nasal Lesions**: Nasal lesions were observed in all 12 control calves (100%) and 20 of 22 vaccinated calves (91%). The estimated prevented fraction for observed nasal lesions is 9%. An estimated mitigated fraction on the severity of affected calves was 90% (95% CI: 72%, 100%). The lesions among the affected controls were more severe than among affected vaccinates. The median duration that nasal lesions were observed was 6 days for the vaccinates and 10.5 days for the controls. The estimated shift in duration between the two groups was 4.5 days.

**Rectal Temperatures**: The post-challenge rectal temperature analysis using the pre-challenge average temperature as a covariate revealed a statistically significant (p<0.05) group and group by day effect. The analysis revealed that the temperature of the control group was significantly (p<0.05) increase from baseline as compared to the vaccinate group on days 3 through 9 and day 11 post-challenge.

**BHV-1 Isolation**: All of the control and vaccinate calves shed BHV-1 during the post-challenge phase of this study. The median duration of BHV-1 isolation from the vaccinate group was 6 days and 10.5 days for the controls. The estimated shift in duration of shedding was 4.5 days. All samples exhibiting BHV specific CPE were confirmed by PCR. However, there were 8 samples in which the CPE was not observed yet they had weak positive PCR signals. These 8 samples were not included as positive for this analysis..

**Clinical Signs of BHV-1**: Each animal was examined for clinical signs of respiratory disease for two consecutive days prior to challenge, the day of challenge and for fourteen consecutive days post-challenge. Clinical signs that could be attributed to BHV-1 infection were observed and recorded. No adverse post-vaccination reactions were observed in any of the calves.

### CONCLUSIONS

The preceding report demonstrates the efficacy of the BHV-1 fraction Bovine Rhinotracheitis - Virus Diarrhea - Parainfluenza₃ - Respiratory Syncytial Virus Vaccine, Modified Live Virus as an aid in the reduction of disease due to BHV-1 in stocker/feeder calves. The efficacy and the antigenicity of the BHV-1 fraction of this combined product is attested to by the following:
1) A decrease in the severity and duration of nasal lesions in the vaccinate group when compared to the control group.
2) A decrease in pyrexia in the vaccinate group as compared to the control group.
3) Sero-conversion to BHV-1 of the vaccinate group as compared to the control group.

### EXAMPLE 8 - PI₃ EFFICACY PROTOCOL

This example demonstrates that the PI₃ fraction of the hexavalent MLV vaccine aids in the prevention of disease caused by PI₃.

### MATERIALS AND METHODS

The hexavalent modified, live virus vaccine is prepared as described above. A PI₃-delete placebo is made alongside the efficacy serial. The PI₃ delete placebo contains the same BHV-1, BVDV-1b, BVDV-2, BVDV-1a, and BRSV harvest batches as the efficacy serial. Culture media is substituted for the PI₃ component to maintain equivalent volume between efficacy serial and PI₃ delete placebo. Sterile diluent is used to rehydrate both vaccine and the PI₃ delete placebo.

Commercial stocker/feeder calves approximately 3-4 months of age are randomized as described above, then de-wormed and vaccinated against pasteurellosis, mycoplasmosis and blackleg. All calves are given free-choice access to water, coastal Bermuda hay and a mixed feed ration containing a coccidiostat, but no antibiotic. Hay availability may subsequently be restricted after the calves are on feed.

Approximately twenty or more vaccinate and ten or more control calves is used in this single level study. The two groups of calves are maintained in separate but equivalent pens prior to challenge. Calves are then commingled in one pen, two days prior to challenge (day -2).

### RANDOMIZATION

A matched randomization scheme of the calves may be used to allocate calves into two treatment groups (vaccinate or placebo-vaccinated control). For example, a sequence of three calves entering the chute may be randomized into the two treatment groups in a 2:1 ratio. Ear tags, randomly drawn from a bucket at the time of initial blood draw for serological evaluation, are used to identify calves.

### BLINDING

Blinded personnel do not have knowledge of the identity of the controls or vaccinates. All field and laboratory personnel, with the exception of the record keeper, are blinded for the duration of the study.

### OUTCOME

The primary outcome for this study is the prevention of nasal shedding of PI₃ virus after challenge. This is determined by the presence or absence of PI₃ in nasal samples collected daily from the nares of all calves during the course of the study. Other anticipated secondary outcomes include decreased clinical signs of PI₃ infection, reduction of pyrexia and reduction of the duration of shedding in the calves shedding PI₃. Experimental units are excluded from the study if they have a PI₃ titer of ≥2 at the initiation of the study.

### SAMPLING FRAMES, DATA COLLECTION, AND CLINICAL OBSERVATIONS

Blood samples are collected from calves immediately prior to vaccination (day -28 to -21) with efficacy serial or PI₃ deleted placebo. Blood samples are also collected immediately prior to challenge (Day 0) and again 14 days post challenge (Day 14). Blood samples for differential WBC counts are collected on day -2 through at least day 14. All samples are collected in EDTA Vacutainer® tubes, and counts performed using a Drew Scientific, Hemavet 950 differential WBC counter. Nasal swabs are taken for virus isolation on day -2 through at least Day 14.

The shedding of PI₃ by the vaccinate and control groups is determined daily for 14 days post-challenge. The detection of PI₃ serves as the primary criteria for determining resistance (unaffected) or susceptibility (affected) to challenge. The affected and unaffected groups are compared to determine if vaccination prevented the shedding of PI₃ after a PI₃ challenge exposure. Clinical signs of respiratory disease serve as a secondary criterion for determining susceptibility or resistance to challenge. The duration of shedding (number of days PI₃ was detected from an individual calf) is analyzed to determine if the vaccination had a mitigating effect on this parameter. All individual daily rectal temperatures are analyzed using the pre-challenge average temperature (baseline) as a covariate.

***PI₃ detection:*** Nasal swabs are taken from each calf on the day of challenge and for 14 consecutive days post-challenge by swabbing both nares with a BBL Culture Swab® with Liquid Stuart Media. The media from the samples are assayed for the presence of PI₃ by Real Time PCR using the PI₃-specific probe/primer set described herein.

***Duration of shedding:*** Duration of shedding is defined as the number of days that PI₃ is detected in the nasal swabs of an individual calf. The collected duration data is analyzed to determine if vaccination had a mitigating effect on the duration of time PI₃ was detected in nasal samples.

***Rectal Temperature:*** Daily rectal temperatures for each animal are recorded between Day -2 and Day 14 (2 days prior to challenge, the day of challenge, and 14 days' post-challenge). A pre-challenge mean is calculated for each animal by averaging temperature values for Days -2 through Day 0. The daily temperatures are analyzed using baseline as a covariate.

***Antibody Titers:*** Serum neutralization antibody titers are determined by the constant virus decreasing serum method for each animal on the day of vaccination, Day 0 and Day 14 of the study.

***Clinical Signs:*** Clinical signs of PI₃ have been described by Bryson *et al.* (1989). Clinical Signs include but are not limited to dullness, rapid breathing, pyrexia, coughing, adventitious and/or harsh lung sounds. Clinical signs are recorded two days prior to challenge, the day of challenge and for fourteen days' post-challenge. The observation of clinical signs of PI₃ is taken into consideration during the analysis of the data but is not considered as a primary outcome due to the fact that these signs can be indicative of many respiratory diseases commonly found in commercial cattle.

### Example 8a - PI₃ Efficacy Study

The results of this study demonstrate that the modified live Bovine Parainfluenza₃ Virus (PI3) fraction of Bovine Rhinotracheitis - Virus Diarrhea - Parainfluenza₃ - Respiratory Syncytial Virus Vaccine, Modified Live Virus, is antigenic and efficacious as an aid in the prevention of shedding of Bovine Parainfluenza₃ Virus in stocker/feeder calves. Two doses of vaccine were administered to twenty PI3 sero-negative calves. Ten sero-negative control calves were vaccinated with two doses of a product matched-placebo vaccine. All calves were challenged intra-nasally with PI3. Vaccination resulted in an increased PI3 antibody titer and prevented PI3 shedding after challenge. Consequently, a minimum PI3-TCID₅₀ of 6 X 10⁴per dose has been established for the PI3 fraction of this vaccine.

### MATERIALS AND METHODS

**Vaccination and Challenge of Calves**: Thirty (30) PI3 negative calves were commingled in a sorting ally. Each calf was gate cut as they were randomly herded, three calves at a time, through the alley into one of two groups (Group A - vaccinates or Group B - placebo vaccinated controls) by using a matched-set randomization scheme provided by CVB-Biometrics.

Twenty calves were vaccinated with one dose of Bovine Rhinotracheitis - Virus Diarrhea - Parainfluenza₃ - Respiratory Syncytial Vaccine, Modified Live Virus. Each vaccinate was given a 2 ml dose of the product by subcutaneous injection in the left side of the neck on day 0. Ten calves were vaccinated with a product matched placebo vaccine (PI3 delete). Nasal swabs were collected for PI3 virus isolation and blood was drawn from all calves for serological confirmation of susceptibility to PI3. On day 25 the vaccinate and control calves received a second 2 ml dose of the Efficacy Serial and the product matched placebo, subcutaneously, in the right side of the neck. Two days prior to challenge both vaccinate and control groups were commingled for the purpose of pre-challenge clinical observation and to acquire baseline body temperature readings. The vaccinate and control calves were challenged 15 days following the second vaccination with 4 ml (2ml per nostril) of PI3 Challenge Virus provided by the USDA-APHIS-CVB, Reisinger strain, Lot Number 05-07, Fill Date- July 26, 2005. The challenge virus titer was determined by CVB to be 10^{8.2} TCID₅₀/2mL. The challenge virus was titered immediately prior to challenge and determined to be 10^{8.3} TCID₅₀ / 4 mL and again immediately after challenge and determined to be 10^{8.2} TCID₅₀ / 4 mL. The challenge virus was kept chilled throughout the challenge process.

Blood samples were collected the day of vaccination, six days after vaccination, the day of challenge, six days after challenge and 14 days post-challenge. Bovine Parainfluenza₃ Virus antibody titers were determined by the constant virus-decreasing serum neutralization method.

Nasal swabs were collected the day of vaccination, the day of challenge and for 10 consecutive days post-challenge.

Rectal temperatures were recorded daily for each calf for two days prior to challenge, the day of challenge and for 14 consecutive days post-challenge.

Clinical observations were made. The observers were blinded due to both the vaccinate and control groups being commingled with the only differentiating feature being the ear tag number. At no time throughout the study were the observers given knowledge of the vaccination status of an individual test animal. Laboratory personnel did not have knowledge of the vaccination status of calves.

***PI3 Detection**:* Nasal swabs were collected from calves by swabbing both nares with a BBL Culture Swab® in Liquid Stuart Media. Visual observation of the PI3 specific CPE along with Cell culture-enhanced PCR techniques were utilized to detect PI3 in collected samples. Briefly, the samples were processed by sterile filtration (0.2µm) on to TVL-BK cells in culture. The cultures incubated for 4 days at 37°C ± 2 °C and 5 ± 1% CO₂. The cultures were observed for PI3 specific CPE and results were recorded. The supernatant from all culture samples (both + and - CPE) were individually assayed for the presence/absence of PI3 using conventional RT qPCR methodology and the following probe/primer set:
Forward Primer : GGAGACCAAGACCAAGGAGATG (SEQ ID NO:13)
Reverse Primer: CGTCTGCCCATGCATAAGG (SEQ ID NO:14)
TaqMan MGB Probe: ACCTCGGTCATCCATAG (SEQ ID NO:15)

This method was developed based on the work of A. Hu *et.al.* (Development of a real-time RT-PCR assay for detection and quantitation of parainfluenza virus 3. A. Hu, M. Colella, P. Zhao, F. Li, J.S. Tam, R. Rappaport, S.M. Cheng. Journal of Virological Methods, 130 (2005) 145-148). The specificity of the probe/primer set was confirmed in house and did not cross react with BHV, BRSV, BVDV1a, BVDV1b or BVDV2. The RT qPCR assays were performed using Taqman® based assays on an Applied Biosystems 7500.

All samples that were PI3 specific CPE positive were also PCR positive. There were 17 samples that were negative for PI3 specific CPE and positive for PI3 by the PCR assay.

This is consistent with in house studies indicating that PCR based assays are more sensitive than assays based on the observation of PI3 specific CPE. Samples that were negative for PI3 after the initial culture were subcultured and observed for CPE. The supernatant from subcultured fluids were assayed for the presence/absence of PI3 according to the same method.

***Statistical Analysis***: The efficacy of the PI3 fraction of this vaccine was primarily based on the proportion of vaccinates that shed PI3 as compared to the proportion of controls that shed PI3 post-challenge. The preventable fraction was calculated as described by Tanner and Morgan (1993). Group comparisons of the ordinally scaled titer data was compared by the Mann- Whitney U Test. Rectal temperatures were analyzed using the pre-challenge temperature average as a covariate. Analysis of post-challenge temperature for day 1-14 was conducted with a repeated measures analysis of variance (ANOVA). All statistical analysis was performed using SAS for Windows.

### RESULTS

**Serum neutralization antibody titers:** All calves in this study had a pre-vaccination PI3 serum neutralization antibody titer of <1:2. None of the vaccinated calves demonstrated an anamnestic immune response to the vaccine. By the day of challenge, the vaccinate group had an average PI3 titer of 1:16 which was significantly (p≤.05) greater than that of the control group at 1:3. One calf in the control group, calf 7, developed a titer of 1:16 during the course of the study. This calf also demonstrated an anamnestic response to the challenge and had a tier of 1: 128 six days after challenge. The other nine control calves were sero- negative on the day of challenge and did not demonstrate an anamnestic response to the challenge.

**PI3 Isolation**: All ten control calves in this study shed PI3 during the post-challenge phase of this study. Only 4 of the 20 vaccinates shed PI3 during this time. This results in a Preventable Fraction of 0.80. All samples exhibiting PI3 specific CPE were confirmed by PCR. However, there were 17 samples in which the CPE was not observed yet they had weak positive PCR signals. These 17 samples have been included as PI3 positive for this analysis.

**Rectal Temperatures**: The post-challenge rectal temperature analysis using the pre-challenge average temperature as a covariate revealed no statistically significant differences between groups. No further analysis of this data was pursued.

**Clinical Signs of PI3**: Each animal was examined for clinical signs of respiratory disease for two consecutive days prior to challenge, the day of challenge and for fourteen consecutive days post-challenge. No clinical signs that could be definitively attributed to PI3 infection were observed. No adverse post-vaccination reactions were observed in any of the calves.

### CONCLUSIONS

The preceding report demonstrates the efficacy of the PI3 fraction Bovine Rhinotracheitis - Virus Diarrhea - Parainfluenza₃ - Respiratory Syncytial Virus Vaccine, Modified Live Virus as an aid in the prevention of shedding of PI3 in stocker/feeder calves.

The efficacy and the antigenicity of the PI3 fraction of this combined product is attested to by the following:
1) Virus (PI3) isolation studies revealed that the administration of two doses of vaccine provided 80% (16/20) protection in vaccinated calves as compared to 100% (10/10) susceptibility in the controls (Preventable Fraction = 0.80).
2) A significant increase in antibody titers to PI3 of the vaccinate group as compared to the control group.

### EXAMPLE 9-BVDV-2 EFFICACY PROTOCOL

This example demonstrates that the BVDV type 2 fraction of the hexavalent MLV vaccine aids in the prevention of disease caused by BVDV-2.

### MATERIALS AND METHODS

The hexavalent modified, live virus vaccine is prepared as described above. A BVDV-2 delete placebo is made alongside the efficacy serial. The BVDV-2 delete placebo contains the same BHV-1, BVDV-lb, BVDV-la, PI₃ and BRSV harvest batches as the efficacy serial. Culture media is substituted for the BVDV-2 component to maintain equivalent volume between efficacy serial and BVDV-2 delete placebo. Sterile diluent is used to rehydrate both vaccine and the BVDV-2 delete placebo.

Commercial stocker/feeder calves approximately 3-4 months of age are randomized as described above, then de-wormed and vaccinated against pasteurellosis, mycoplasmosis and blackleg. All calves are given free-choice access to water, coastal Bermuda hay and a mixed feed ration containing a coccidiostat, but no antibiotic. Hay availability may subsequently be restricted after the calves are on feed.

Approximately twenty or more vaccinate and ten or more control calves is used in this single level study. The two groups of calves are maintained in separate but equivalent pens prior to challenge. Calves are then commingled in one pen, two days prior to challenge (day -2).

### RANDOMIZATION, BLINDING, AND OUTCOME

Randominzation, blinding, and outcome, are as described in Example 10.

### SAMPLING FRAMES, DATA COLLECTION, AND CLINICAL OBSERVATIONS

Blood samples are collected from calves immediately prior to vaccination (day -28 to -21) with efficacy serial or BVDV-2 delete placebo. Blood samples are also collected immediately prior to challenge (Day 0) and again 14 days post challenge (Day 14).

Blood samples for differential WBC counts are collected on day -2 through at least day 14. All samples are collected in EDTA Vacutainer® tubes, and counts performed using a Drew Scientific, Hemavet 950 differential WBC counter. Nasal swabs are taken for virus isolation on day -2 through at least Day 14.

Outcome variables include leukopenia, pyrexia, nasal virus shedding, viremia, neutralizing antibody production and clinical signs. The primary outcome is the prevention of BVDV-2 induced leukopenia in the vaccinate group as compared to the control group following BVDV-2 challenge.

**Leukopenia:** The WBC count data is analyzed to determine if there is a reduction in the post-challenge count. A pre-challenge mean is calculated for each animal by averaging WBC counts for Days -2 through 0. The ratio of daily WBC counts relative to the pre-challenge mean is calculated (WBC count as a proportion of pre-challenge) for each post-challenge day 1-14 or longer at the discretion of the observers. If the proportion decreases by 25% the individual is classified as leukopenic.

**Shedding:** Nasal swabs are taken from each calf on the day of challenge and for 14 consecutive days post-challenge by swabbing both nares with a BBL Culture Swab® with Liquid Stuart Media. On the day of collection, samples are processed by sterile filtration through a 0.2-µm filter. Each well of a 12-well plate containing TVL-MDBK (∼80% confluent) is then inoculated with a sample. One well per plate remains uninoculated and serves as the negative control. One well of the plate is inoculated with a dilution of the challenge virus and serves as the positive control. The plates are incubated at 3-7% CO₂ at a temperature of 35-39°C for 2 to 4 days. Media from each well is harvested and stored at -18 to -22°C. Media from wells that exhibit typical BVDV-la cytopathic morphology is further processed to determine the identity of the infectious agent. Individuals that are culture positive are classified as "shedding".

**Viremia:** Buffy coats from blood samples are used to inoculate each well of a 12-well plate containing TVL-MDBK (∼80% confluent). One well per plate remains uninoculated and serves as the negative control. One well of the plate is inoculated with a dilution of the challenge virus and serves as the positive control. The plates are incubated at 3-7% CO₂ at a temperature of 35-39°C for 2 to 4 days. Media from each well is harvested and stored at -18 to -22°C. Media from wells that exhibit typical BVDV-2 cytopathic morphology are further processed to determine the identity of the infectious agent. Individuals that are culture positive for BVDV-2 are considered "viremic".

**Antibody Titers:** Serum neutralization antibody titers against BVDV-2 is determined by the constant virus decreasing serum method for each animal on the day of initial vaccination (day -21), the day of challenge (day 0), and at the conclusion of the study. Antibody titers are determined according to Special Outline A-17, Titration of Bovine Viral Diarrhea Virus Type 2 Neutralizing Antibody. Animals that develop an antibody titer ≥8 are considered as seroconverted to BVDV-2.

**Clinical Signs:** Clinical signs of BVDV-2 may include, but are not limited to pyrexia, leukopenia, dyspnea, nasal discharge and loose stools. All relevant clinical signs of BVDV infection are recorded two days prior to challenge, the day of challenge and for 14 days' post-challenge. The observation of clinical signs of BVDV-2 is taken into consideration during the analysis of the data but is not considered as a primary outcome because these signs may be indicative of other diseases commonly encountered in this class of cattle.

### EXAMPLE 9A-BVDV-2 EFFICACY STUDY

The results of this study demonstrate that the modified live Bovine Viral Diarrhea Virus - Type 2 (BVDV2) fraction of Bovine Rhinotracheitis - Virus Diarrhea - Parainfluenza₃ - Respiratory Syncytial Virus Vaccine, Modified Live Virus, is antigenic and efficacious as an aid in the prevention of disease caused by BVDV2. One dose was administered to twenty BVDV sero-negative calves. Ten sero-negative control calves were vaccinated with one doses of a product matched-placebo vaccine. All calves were challenged intra-nasally with BVDV2 (strain 1373). Vaccination resulted in an increased BVDV2 antibody titer (from an average of <2 to 24), reduced post challenge death rate (7/10 controls dead, 0/20 vaccinates dead). Consequently, a minimum BVDV2-TCID₅₀ of 3 X 10³ TCID₅₀ per dose has been established for the BVDV2 fraction of this vaccine.

### Example 10-BVDV-1A Efficacy Protocol

This example demonstrates that the BVDV Type 1a fraction of the hexavalent MLV vaccine aids in the prevention of disease caused by BVDV-1a.

### MATERIALS AND METHODS

The hexavalent modified, live virus vaccine is prepared as described above.

A BVDV-la-delete placebo is made alongside the efficacy serial. The BVDV-la delete placebo contains the same BHV-1, BVDV-lb, BVDV-2, PI₃ and BRSV harvest batches as the efficacy serial. Culture media is substituted for the BVDV-la component to maintain equivalent volume between efficacy serial and BVDV-la delete placebo. Sterile diluent is used to rehydrate both vaccine and the BVDV-la delete placebo.

Commercial stocker/feeder calves approximately 3-4 months of age are randomized as described above, then de-wormed and vaccinated against pasteurellosis, mycoplasmosis and blackleg. All calves are given free-choice access to water, coastal Bermuda hay and a mixed feed ration containing a coccidiostat, but no antibiotic. Hay availability may subsequently be restricted after the calves are on feed.

Approximately twenty or more vaccinate and ten or more control calves is used in this single level study. The two groups of calves are maintained in separate but equivalent pens prior to challenge. Calves are then commingled in one pen, two days prior to challenge (day -2).

### RANDOMIZATION

A matched randomization scheme of the calves may be used to allocate calves into two treatment groups (vaccinate or placebo-vaccinated control). For example, a sequence of three calves entering the chute may be randomized into the two treatment groups in a 2:1 ratio. Ear tags, randomly drawn from a bucket at the time of initial blood draw for serological evaluation, are used to identify calves.

### BLINDING

Blinded personnel do not have knowledge of the identity of the controls or vaccinates; all field and laboratory personnel (with the exception of the record keeper), are blinded for the duration of the study.

### OUTCOME

The primary outcome is leukopenia after challenge. Leukopenia is defined as a 25% decrease from baseline WBC counts. Other outcomes that would further support the efficacy of the vaccine include nasal shedding, viremia, pyrexia, antibody production and clinical signs of BVDV.

Experimental units are excluded from the study if they have a BVDV-1 titer of ≥2 at the initiation of the study.

### SAMPLING FRAMES, DATA COLLECTION, AND CLINICAL OBSERVATIONS

Blood samples are collected from calves immediately prior to vaccination (day -28 to -21) with efficacy serial or BVDV delete placebo. Blood samples are also collected immediately prior to challenge (Day 0) and again 14 days post challenge (Day 14). Blood samples for differential WBC counts are collected on day -2 through at least day 14. All samples are collected in EDTA Vacutainer® tubes, and counts performed using a Drew Scientific, Hemavet 950 differential WBC counter. Nasal swabs are taken for virus isolation on day -2 through at least Day 14.

Outcome variables include leukopenia, pyrexia, nasal virus shedding, viremia, neutralizing antibody production and clinical signs. The primary outcome is the prevention of BVDV induced leukopenia in the vaccinate group as compared to the control group following BVDV-la challenge.

**Leukopenia, Shedding, and Viremia:** WBC count data is analyzed as described in Example 9; nasal swabs are taken from each calf on the day of challenge and for 14 consecutive days post-challenge by swabbing both nares with a BBL Culture Swab® with Liquid Stuart Media to determine shedding, also as described in Example 9. Buffy coats from blood samples are used to inoculate each well of a 12-well plate containing TVL-MDBK (∼80% confluent) and assayed as described in Example 9.

**Antibody Titers:** Serum neutralization antibody titers against BVDV-la are determined by the "constant virus decreasing serum" method for each animal on the day of initial vaccination (day -21), the day of challenge (day 0), and at the conclusion of the study. Antibody titers are determined according to Special Outline A-17, Titration of Bovine Viral Diarrhea Virus Type 1a Neutralizing Antibody. Animals that develop an antibody titer ≥8 are considered as seroconverted to BVDV-la.

**Clinical Signs:** Clinical signs of BVDV-la may include, but are not limited to pyrexia, leukopenia, dyspnea, nasal discharge and loose stools. All relevant clinical signs of BVDV infection are recorded two days prior to challenge, the day of challenge and for 14 days' post-challenge. The observation of clinical signs of BVDV-1 is taken into consideration during the analysis of the data but is not considered as a primary outcome because these signs may be indicative of other diseases commonly encountered in this class of cattle.

### EXAMPLE 10A-BVDV-1A EFFICACY STUDY

The results of this study demonstrate that the modified live Bovine Viral Diarrhea Virus - Type 1a (BVDV1a) fraction of Bovine Rhinotracheitis - Virus Diarrhea - Parainfluenza₃ - Respiratory Syncytial Virus Vaccine, Modified Live Virus, is antigenic and efficacious as an aid in the prevention of infection caused by BVDV1a. One doses of vaccine was administered to twenty BVDV sero-negative calves. Ten sero-negative control calves were vaccinated with one doses of a product matched-placebo vaccine. All calves were challenged intra-nasally with BVDV1a. Vaccination resulted in an increased BVDV1a antibody titer and either prevented or reduced BVDV1a induced viremia, nasal shedding, pyrexia, lymphopenia and leukopenia. Consequently, a minimum BVDV1a-TCID₅₀ of 5 X 10³ TCID₅₀ per dose has been established for the BVDV1a fraction of this vaccine.

### MATERIALS AND METHODS

**Vaccination, Challenge and Sample Collection:** Thirty BVDV negative calves were commingled in a sorting ally. Each calf was gate cut as they were randomly herded, three calves at a time, through the alley into one of two groups (Group A - vaccinates or Group B - placebo vaccinated controls) by using a matched-set randomization scheme provided by CVB-Biometrics.

Twenty calves were vaccinated with one dose of Bovine Rhinotracheitis - Virus Diarrhea - Parainfluenza₃ - Respiratory Syncytial Vaccine, Modified Live Virus,. Each vaccinate was given a 2 ml dose of the product by subcutaneous injection in the left side of the neck on day 0. Ten calves were vaccinated with a product matched placebo vaccine (BVDV delete). Two days prior to challenge both vaccinate and control groups were commingled for the purpose of pre-challenge clinical observation and to acquire baseline body temperature and WBC readings. The vaccinate and control calves were challenged 21 days following vaccination with a virulent isolate of BVDV1a obtained from the lungs of a pen dead calf at Ruff Farms in Hanston, Kansas. The virus had been passed *in vitro* on TVL-BK cells using Dulbecco's Modified Eagle Medium with 10% Equine Sera. The challenge virus was titered immediately prior to challenge and determined to be 10^{7.8} TCID₅₀ / 4mls and immediately after challenge and determined to be 10^{7.3} TCID₅₀ / 4mls. Two mls of challenge virus was administered into each nasal passage during inspiration.

The challenge virus was kept chilled throughout the challenge process.

Blood samples for determining antibody titers were collected prior to vaccination, the day of challenge (21 days post-vaccination), and 14 days post-challenge. Bovine Viral Diarrhea Virus antibody titers were determined by the constant virus-decreasing serum neutralization method.

Nasal swabs for virus isolation were collected from calves by swabbing both nares with a BBL Culture Swab® in Liquid Stuart Media on the day of challenge and for 14 consecutive days post-challenge.

Blood samples for buffy coat virus isolation were collected from each calf by venapuncture into an EDTA vacutainer tube on the day of challenge and for 14 consecutive days post-challenge.

Rectal temperatures were recorded daily for each calf for two consecutive days prior to challenge, the day of challenge and for 14 consecutive days post-challenge.

Clinical observations for each calf were made and recorded. The observers were blinded due to both the vaccinate and the control groups being commingled with the only differentiating feature being the ear tag number. At no time throughout the study were the observers given knowledge of the vaccination status of an individual test animal. Laboratory personnel did not have knowledge of the vaccination status of calves.

Blood samples for determining differential WBC counts were collected for two consecutive days prior to challenge, the day of challenge, and 14 consecutive days post-challenge. Samples were collected in EDTA vacutainer tubes and counts performed using a Drew Scientific, Hemavet 950 differential WBC counter.

*BVDV Detection:* Observation of CPE and cell culture-enhanced PCR techniques were utilized to detect BVDV1a in collected samples. Briefly, the nasal samples were processed by sterile filtration (0.2µm) on to TVL-BK cells in culture. Buffy coats were placed directly onto TVL-BK cells in culture for 45 - 75 minutes prior to being washed off the cells. The cultures incubated for 4 days at 37°C ± 2 °C and 5 ± 1% CO₂. Each culture was observed for the presence/absence of BVDV specific CPE and the supernatant from each culture was individually assayed for the presence/absence of BVDV1a using conventional RT qPCR methodology and the following probe/primer set:
Forward Primer : GGTCGCCCAGGTAAAAGCA (SEQ ID NO:7)
Reverse Primer: GCCTCTGCAGCACCCTATCA (SEQ ID NO:8)
TaqMan MGB Probe: AACCGACTGTTACGAATAC (SEQ ID NO:9)

This method was developed based on (Differentiation of types 1a, 1b and 2 bovine viral diarrhea virus (BVDV) by PCR, Julia F. Ridpath and Steven R. Bolin, Molecular and Cellular probes, Journal of Virological Methods, 130 (2005) 145-148). This method is also based on the Center for Veterinary Biologics and National Veterinary Services Laboratories Test Protocol - Genotyping Bovine Viral Diarrhea Virus, Number BPPR02010.01. Both of these methods of differentiating genotypes and subgenotypes of BVDV exploit the genetic differences that exists in the 5' Untranslated Region (UTR) of the genome. This probe/primer set was specifically designed to amplify a section of the 5' UTR in which a uniquely BVDV1a sequence can be detected by the highly specific probe. The specificity of the probe/primer set was confirmed in house and did not cross react with BHV,PI3, BRSV, BVDV1b, or BVDV2. The RT qPCR assays were performed using Taqman® based assays on an Applied Biosystems 7500.

**Statistical Analysis:** Antibody Titers: Group comparisons of the ordinally scaled titers were analyzed with the Mann-Whitney U Test.

**Nasal Shedding**: Virus isolation data from nasal swab analysis is based on the proportion of vaccinates that BVDV1a was isolated from as compared to the proportion of controls that BVDV1a was isolated from post-challenge. Preventable fractions were calculated based on observation of CPE and on detection by PCR independently. The preventable fraction for BVDV1a shedding was calculated as described by Tanner and Morgan (1993).

**Viremia**: Virus isolation data from buffy coats of blood samples was analyzed based on the proportion of vaccinates that BVDV1a was isolated from as compared to the proportion of controls that BVDV1a was isolated from post-challenge. Preventable fractions were calculated based on observation of CPE and on detection by PCR independently. The preventable fraction for BVDV1a viremia was calculated as described by Tanner and Morgan (1993).

**Rectal Temperature**: A pre-challenge average rectal temperature was determined for each animal. This average was used as a covariate in a repeated measures analysis of variance (ANOVA) which included terms for Group, Day and Group*Day interactions.

**Clinical Signs of Disease**: Clinical signs of disease were recorded daily by each observer independently.

**Lymphopenia**: A pre-challenge lymphocyte mean was calculated for each animal by averaging lymphocyte counts for Days -2 through 0. The ratio of daily lymphocyte counts relative to the pre-challenge mean was calculated (lymphocyte count as a proportion of pre-challenge) for each post-challenge day 1-14. This proportion data was analyzed to determine if individual animals developed a lymphopenia as defined by a 25% decrease in counts from baseline. The preventable fraction was calculated as described by Tanner and Morgan (1993) to determine if vaccination prevented the development of lymphopenia in this study

**Leukopenia**: A pre-challenge leukocyte mean was calculated for each animal by averaging leukocyte counts for Days -2 through 0. The ratio of daily leukocyte counts relative to the pre-challenge mean was calculated (leukocyte count as a proportion of pre-challenge) for each post-challenge day 1-14. This proportion data was analyzed to determine if individual animals developed a leukopenia as defined by a 25% decrease in counts from baseline. The preventable fraction was calculated as described by Tanner and Morgan (1993) to determine if vaccination prevented the development of leukopenia in this study.

All statistical analysis was performed using SPSS version 16 for Windows.

### RESULTS

**Serum neutralization antibody titers:** All calves in this study had a pre-vaccination BVDV (BVDV1a, BVDV1b and BVDV2) serum neutralization antibody titer of <1:2. By the day of challenge (Day 21), the vaccinate group had an average BVDV1a titer of 1:21 which was significantly (p≤.05) greater than that of the control group < 1:2. The BVDV1a antibody titers increased 14 days post-challenge in both the control and vaccinate calves indicating that the calves had been challenged with BVDV1a.

**BVDV1a Isolation from Nasal Swabs:** Based on the observation of BVDV specific CPE , all ten control calves in this study shed BVDV1a during the post-challenge phase of this study. Only 3 of the 20 vaccinates shed BVDV 1a during the post challenge period. A prevented fraction was calculated using a ratio of 0/10 controls naturally protected and 17/20 vaccinates protected. This results in a Preventable Fraction of 0.85.

Based on the cell culture enhanced PCR detection of BVDV1a, all ten control calves in this study shed BVDV1b during the post-challenge phase of this study. Only 5 of the 20 vaccinates shed BVDV 1a during the post challenge period. A prevented fraction was calculated using a ratio of 0/10 controls naturally protected and 15/20 vaccinates protected. This results in a Preventable Fraction of 0.75.

**BVDV1a Isolation from Buffy Coats:** Based on the observation of BVDV specific CPE, six of the ten control calves in this study were viremic during the post-challenge phase of this study. None of the 20 vaccinates were viremic during the post challenge period. A prevented fraction was calculated using a ratio of 4/10 controls naturally protected and 20/20 vaccinates protected. This results in a Preventable Fraction of 1.0.

Based on the cell culture enhanced PCR detection of BVDV1a, seven of the ten controls in this study were viremic during the post-challenge phase of this study. Two of the 20 vaccinates were viremic during the post challenge period. A prevented fraction was calculated using a ration of 3/10 controls naturally protected and 18/20 vaccinates protected. This results in a Preventable Fraction of 0.86.

**Rectal Temperatures**: Post-challenge rectal temperature analysis using the pre-challenge average temperature as a covariate revealed a statistically significant (p<0.05) group and group by day effects. The analysis revealed that the temperature of the control group was significantly greater than that of the vaccinate group on days 6, 8 and 14 and approached significance on days 5 and 11.

**Clinical Signs of BVDV1a**: One calf in the control group demonstrated clinical signs that could be contributed to BVDV1a infection. The clinical sign observed from calf 21 was a copious amounts of mucopurulent nasal discharge. None of the calves in the vaccinate group demonstrated any clinical signs of BVDV1a infection during the post-challenge phase of the study. No adverse post-vaccination reactions were observed in any of the calves.

**Lymphopenia:** Lymphopenia was detected in two of the 20 vaccinates and five of the ten controls resulting in a preventable fraction of 80%. Daily averages of the proportion data reveal a typical response of the control calves to BVDV challenge (lymphopenia followed with a rebound spike). The vaccinated calves did not respond in like manner.

**Leukopenia:** Leukopenia was detected in five of the 20 vaccinates and four of the ten controls resulting in a preventable fraction of 37%. Daily averages of the proportion data are represented graphically in Figure 3.

### CONCLUSIONS

The preceding report demonstrates the antigenicity and efficacy of the BVDV1a fraction Bovine Rhinotracheitis - Virus Diarrhea - Parainfluenza₃ - Respiratory Syncytial Virus Vaccine, Modified Live Virus as an aid in the prevention of infection caused by BVDV1a.

The antigenicity and the efficacy of the BVDV1a fraction of this combined product is attested to by the following:
1) A significant increase in antibody titers to BVDV1a in the vaccinate group as compared to the control group. Nineteen of the 20 calves in the vaccinate group developed BVDV1a titers ≥ 1:8 after administration of one 2ml dose of vaccine, meeting the requirements defined in 9CFR 113.311 (c) (5).
2) Virus isolation studies revealed that the administration of one dose decreased BVDV1a induced nasal shedding and viremia in post-challenge calves.
3) Vaccinated calves exhibited significantly less fever when compared to control calves.
4) Vaccination also decreased BVDV1a induced lymphopenia and leukopenia in post-challenge calves.

The study's findings are both statistically significant and clinically relevant, therefore, demonstrating the efficacy of the BVDV1a fraction contained in Bovine Rhinotracheitis - Virus Diarrhea - Parainfluenza₃ - Respiratory Syncytial Virus Vaccine, Modified Live Virus.

### EXAMPLE 11-POTENCY ASSAY PROTOCOL FOR SHIPPING FEVER VACCINES

In co-pending United States Provisional Patent Application Number 61/427,404, entitled "Compositions and Methods for Identifying and Differentiating Viral Components of Multivalent Shipping Fever Vaccines" (filed concurrently herewith on December 27, 2010, and specifically incorporated herein in its entirety by express reference thereto), the present inventors reported the development of a modified genetically-based assay protocol useful in obtaining direct viral quantitation (*i.e*., "potency") of each individual viral component in a multivalent vaccine. The invention replaces conventional FA/IFA assays (such as those described in the current SAM 101 assay) with an RT-qPCR assay to determine the presence or absence of particular virus species, types, or subtypes in individual assay wells (*i.e*., dilutions).

The potency tests for the individual components of a multivalent vaccine, such as the hexavalent MLV vaccine described herein can also be made more objective by substituting RT-qPCR/qPCR analysis of individual wells for visual observation of CPE. For example, the observation of CPE in viral titration assays can be rather subjective, and is often considered to be the source of differences in MLV vaccine titers between testing facilities. By increasing the objectivity of the assay, however, the reproducibility between laboratories should also increase.

Potency testing for PI3 may be conducted using a modified Supplemental Assay Method for the Titration of Parainfluenza 3 Virus Vaccines (MVSAM102.01). Potency testing for BRSV may be conducted using a modified Supplemental Assay Method for Titration of Bovine Respiratory Syncytial Virus in Vaccines (MVSAM0129.01). These assays are modified by substituting real-time qPCR analysis of individual wells for visual observation of CPE. Titration of the BHV fraction is conducted using a modified Supplemental Assay Method for the Titration of Infectious Bovine Rhinotracheitis Virus in Vaccines (MVSAM0105.01). In this assay, a 96-well format can be used instead of the standard 6-well format, in order to standardize the assays for multivalent vaccine analysis. Importantly, this protocol includes substituting qPCR analysis of individual wells for visual observation and counting of BHV plaques to provide a more accurate and robust assay.

The present example demonstrates use of the quantitative (real-time) polymerase chain reaction (qPCR) techniques set forth in the inventors' co-pending U.S. Provisional Patent Application No. 61/427,404, to successfully determine the potency of each of the six individual viral components of the hexavalent MLV vaccine described herein. The inventors show that the assay is particularly advantageous over existing methodologies in determining the individual potencies of genetically-related strains in a multivalent example. Using the hexavalent MLV BRDC vaccine as a model, the assay is demonstrated to effectively quantitate the individual potencies of the three genetic subgenotypes of BVDV-1a, BVDV-1b, and BVDV-2 contained therein.

### MATERIALS AND METHODS

### CELL CULTURE

All cultures are prepared using conventional techniques and supplies as described by Freshney (1987). The TVL-Bovine Kidney Cell Line, which exhibit a typical epithelial like morphology in culture, is used for all culture assays.

### SPECIFICITY OF PRIMER/PROBE SETS

RNA is extracted from viral fluids from each of the following United States Department of Agriculture (USDA)-approved viral seeds: BVDV-la (Singer strain), BVDV-lb (TGAC Strain), BVDV-2 (125), PI₃ (Reisinger SF-4), and BRSV (N375) (no extraction process is performed on BHV-1 [Cooper strain] since it is a DNA virus). Extractions are performed using RNAqueous®-4PCR (Ambion; Austin, TX, USA).

Each of the RNA viral samples is used as template (sample) in separate RT-qPCR reactions for each of the six primer/probe sets using TaqMan® one-step RT-PCR chemistry. The DNA virus (BHV-1) is also used as template (sample) in separate qPCR reactions with each of the six primer/probe sets described above. Analysis of the BRSV primer/probe set against all six viral fractions of the hexavalent vaccine indicate there is no cross-reactivity for each of the primer/probe sets with any of the other viral fractions.

### QPCR ASSAYS

qPCR assays are conducted using an Applied Biosystems 7500 Real-Time PCR System.

### OLIONUCLEOTIDE PRIMERS AND LABELED MOLECULAR PROBES

The following custom TaqMan® Probes and virus-specific forward and reverse primer pairs were fabricated by Applied Biosystems (Foster City, CA, USA).

### BVDV-lb (first):

Forward primer: 5'-CACCCTATCAGGCTGTATTCATAGC-3' (SEQ ID NO:1);
Reverse primer: 5'-TGCCCACAGCACATCTTAACC-3' (SEQ ID NO:2); and
BVDV-lb TaqMan® MGB probe: 5'-TCACCTGGACGACCC-3' (SEQ ID NO:3).

### BVDV-lb (second):

Second Forward primer: 5'-GTCGTCCAGGTGAAAACGGT-3' (SEQ ID NO:19);
Second Reverse primer: 5'-GTCGTCCAGGTGAAAACGGT-3' (SEQ ID NO:20); and
Second BVDV-lb Detection probe: 5'-GTCGTCCAGGTGAAAACGGT-3' (SEQ ID NO:21).

### BRSV:

Forward primer: 5'-GCAATGCTGCAGGACTAGGTATAAT-3' (SEQ ID NO:4);
Reverse primer: 5'-ACACTGTAATTGATGACCCCATTCT-3' (SEQ ID NO:5); and
BRSV TaqMan® MGB probe: 5'-AAGACTTGTATGATGCTGCCAA-3' (SEQ ID NO:6).

### BVDV-1a:

Forward primer: 5'-GGTCGCCCAGGTAAAAGCA-3' (SEQ ID NO:7);
Reverse primer: 5'-GCCTCTGCAGCACCCTATCA-3' (SEQ ID NO:8); and
BVDV-la TaqMan® MGB probe: 5'-AACCGACTGTTACGAATAC-3' (SEQ ID NO:9).

### BVDV-2:

Forward primer: 5'-GCTAGCCATGCCCTTAGTAGGAC-3' (SEQ ID NO:10);
Reverse primer: 5'-GACGAGTCCCCTGTACTCAGG-3' (SEQ ID NO:11); and
BVDV-2 TaqMan® MGB probe: 5'-CAGTGAGTCCATTGGATGG-3' (SEQ ID NO:12).

### PI₃:

Forward primer: 5'-GGAGACCAAGACCAAGGAGATG-3' (SEQ ID NO:13);
Reverse primer: 5'-CGTCTGCCCATGCATAAGG-3' (SEQ ID NO:14); and
PI₃ TaqMan® MGB probe: 5'-ACCTCGGTCATCCATAG-3' (SEQ ID NO:15).

### BHV-1:

Forward primer: 5'-CCATGTTAGCGCTCTGGAACC-3' (SEQ ID NO:16);
Reverse primer: 5'-CGTCTTTACGGTCGACGACTCC-3' (SEQ ID NO:17); and
BHV-1 TaqMan® MGB probe: 5'-ACGGACGTGCGCGAA-3' (SEQ ID NO:18).

### POTENCY ASSAY FOR MONOVALENT VACCINES

Protocols for each of the following assays utilize Applied Biosystems 7500 Comparative Threshold Cycle (C_{T}) methodology to determine if an individual well of a titration plate contains a greater amount of virus than the equivalent reference well. Most viral samples contain some non-viable viral particles that could be detected by the PCR assay, thus giving potentially false-positive results. This issue is resolved by the use of a reference plate without cells. The sample is diluted in the reference plate exactly as it is in the assay plate but without cells as to eliminate the possibility for viral replication. The reference plate is used to generate as baseline or background C_{T} value for each of the wells in each assay. If a well on the assay plate has a higher C_{T} value than the corresponding background well, viral replication has occurred and the well is considered positive. If no C_{T} value is determined for a well, or the C_{T} value is equivalent to the background C_{T} value, then the well is considered negative.

### POTENCY ASSAY FOR MULTIVALENT VACCINES

A pilot serial of the IBR/BVDV-1a, -1b, -2/PI₃/RSV hexavalent vaccine, modified live virus, was prepared as described herein. Briefly, BHV-1 (Cooper strain), BVDV-la (Singer strain), BVDV-lb (TGAC Strain), BVDV-2 (125), PI₃ (Reisinger SF-4), and BRSV (N375) were propagated in the TVL-BK cell line (20^{th} pass). Individual fractions were harvested at the 10^{th} passage and blended together into the six-way product. A potency test of for each fraction of the 6-way vaccine may be conducted as described herein with the addition of appropriate neutralizing antisera. The TCID₅₀ of each viral fraction is determined based on CPE/FA/IFA results and compared to those based on RT-qPCR/qPCR.

### BVDV-IA, -1B AND -2 POTENCY TESTS

Monovalent BVDV-la, BVDV-lb and BVDV-2 samples may be titered separately according to Supplemental Assay Method for the Titration of Bovine Viral Diarrhea Virus in Vaccine (SAM 101). Briefly, the assay is conducted in duplicate with one of the plates being used for titer calculation based on CPE and/or FA/IFA as described. The other plate is used to determine a titer based on RT-qPCR. A cell-deficient reference plate is included as described above for each of the viruses.

### PI₃ POTENCY TEST

Potency testing for PI₃ is conducted in duplicate with one of the plates being used for titer calculation based on CPE. The other plate is used to determine a titer based on the comparative C_{T} values of PI₃ as determined by RT-qPCR. A cell-deficient reference plate is included as described above.

### BRSV POTENCY TEST

Potency testing for BRSV is conducted in duplicate with one of the plates being used for titer calculation based on CPE as described above, with the remaining plate being used to determine a titer based on the comparative C_{T} values of BRSV as determined by RT-qPCR. As discussed above, a cell-deficient reference plate is also included in the assay.

### BHV POTENCY TEST

Potency testing for BHV is conducted in a 96-well format, by substituting qPCR analysis of individual wells for conventional visual observation and counting of plaques. The assay is performed in duplicate, with one of the plates being used for titer calculation based on presence or absence of CPE in an individual well (dilution), while the remaining plate is used to calculate a titer based on the comparative C_{T} values of BHV as determined by QPCR. A cell-deficient reference plate is included as described above.

### REFERENCES

The following references, to the extent that they provide exemplary procedural or other details supplementary to those set forth herein, are specifically incorporated herein in their entirety by express reference thereto:
United States Patent No. 4,415,732 to Caruthers et al.
United States Patent No. 4,458,066 to Caruthers et al.
United States Patent No. 4,683,195 to Mullis et al.
United States Patent No. 4,683,202 to Mullis et al.
United States Patent No. 4,725,677 to Koster et al.
United States Patent No. 4,973,679 to Caruthers et al.
United States Patent No. 4,980,460 to Molko et al.
United States Patent No. 5,614,388 to Picone et al.
Agrawal et al., Nucl. Acids Res., 18:5419-5423, 1990
Baker, J.C., "The clinical manifestations of bovine viral diarrhea infection," Vet. Clin. N. Am. Food Anim. Pract., 11:425-445, 1995.
Baker, J.C., Werdin, R.E., Ames, T.R., Markham, R.J., and Larson, V.L., "Study on the etiologic role of bovine respiratory syncytial virus in pneumonia of dairy calves," J. Am. Vet. Med. Assoc., 189(1):66-70, 1986.
Barber, D.M., Nettleton, P.F., and Herring, J.A., "Disease in a dairy herd associated with the introduction and spread of bovine diarrhoea virus," Vet. Rec., 117(18):459-464, 1985.
Beaucage and Iyer, Tetrahedron, 48:2223-2311, 1992.
Becher, P., Konig, M., Paton, D.J., and Thiel, H.J., "Further characterization of border disease virus isolates: evidence for the presence of more than three species within the genus pestivirus," Virology, 209(1):200-206, 1995.
Collett, M.S., R. Larson, S.K. Belzer, and E. Retzel, "Proteins encoded by bovine viral diarrhea virus: the gehomic organization of a pestivirus," Virology, 165(1):200-208, 1988.
Elbers, K., N. Tautz, P. Becher, D. Stoll, T. Rumenapf, and H.J. Thiel, "Processing in the pestivirus E2-NS2 region: identification of proteins p7 and E2p7," J. Virol., 70(6):4131-4135, 1996.
Fergen, B.J., "Estimating an Intervention Effect on Outcome Severity," USDA Center for Veterinary Biologics, Ames, IA, USA; Personal communication, 2004.
Finney, D.J., "Statistical method in biological assay," 3rd Ed., Charles Griffin and Company Ltd., London, 1978.
Flores, E.F., Ridpath, J.F., Weiblen, R. et al., "Phylogenetic analysis of Brazilian bovine viral diarrhea virus type 2 (BVDB-2) isolates: evidence of subgenotype within BVDV-2," Virus Res., 87:51-60, 2002.
Freshney, R.I., "Culture of Animal Cells, A Manual of Basic Technique," Second Edition, Dept of Medical Oncology, University of Glasgow, Alan R. Liss, Inc., Publisher, New York, NY, USA, 1987.
Fulton, R.W., Hessmand, B, Johnson, B.J. et al. "Evaluation of diagnostic test used for detection of bovine viral diarrhea virus and prevalence of subtypes 1a, 1b and 2a in persistently infected cattle entering a feedlot," J. Am. Vet. Med. Assoc., 228(4):578-584, 2006.
Gillespie, J.H., J.A. Baker, and K. McEntee, "A cytopathogenic strain of virus diarrhea virus," Cornell Vet., 50:73-79, 1960.
Hofmann, M.A., Brechtbuhl, K., and Stauber, N., "Rapid characterization of new pestivirus strains by direct sequencing of PCR-amplified cDNA from the 5' non-coding region," Arch. Virol., 139:217-229, 1994.
Holland et al., Proc. Natl. Acad. Sci. USA, 88:7276-7280, 1991.
Liess, B., H.R. Frey, H. Kittsteiner, F. Baumann, and W. Neumann, "Bovine mucosal disease, an immunobiological explainable late stage of BVD-MD virus infection with criteria of a 'slow virus infection,'" Dtsch. Tieraerzti. Wschr., 81(2):481-487, 1974.
Malmquist, W.A., "Bovine viral diarrhea mucosal disease: etiology, pathogenesis, and applied immunity," J. Am. Vet. Med. Assoc., 152:763-768, 1968.
McNulty M.S., Allan G.M., "Application of immunofluorescence in veterinary viral diagnosis," In: Recent advances in virus diagnosis, McNulty MS, McFerran JB (Eds.), pp. 15-26. Martinus Nijhoff, The Hague, Netherlands, 1984.
Olafson, P., A.D., MacCallum, and F.H. Fox, "An apparently new transmissible disease of cattle," Cornell Vet., 36:205-213, 1946.
Ozaki et al., Nucl. Acids Res., 20:5205-5214, 1992.
Paton, D.J., "Pestivirus diversity," J. Comp. Pathol., 112(3):215-236, 1995.
Pellerin, C., J. van den Hurk, J. Lecomte, and P. Tussen, "Identification of a new group of bovine viral diarrhea virus strains associated with severe outbreaks and high mortalities," Virology, 203(2):260-268, 1994.
Ramsey, F.K., and W.H. Chivers, "Mucosal disease of cattle," North Am. Vet., 34:629-633, 1953.
Ranheim, T, Mathis, P.K., Joelsson, D.B. et al., "Development and application of a quantitative RT-PCR potency assay for a pentavalent rotavirus vaccine (Rota Teq®)," J. Virol. Meth., 131:193-201, 2006.
Ridpath, J.F., and Bolin, S.R., "Differentiation of types 1a, 1b, and 2 bovine virus diarrhea virus by PCR," Molec. Cell. Probes, 12:101-106, 1998.
Ridpath, J.F., S.R. Bolin, and E.J. Dubovi, "Segregation of bovine viral diarrhea virus into genotypes," Virology, 205(1):66-74, 1994.
Ridpath, J.F., Bolin, S.R., and Dubovi, E.J., "Segregation of bovine viral diarrhea virus into genotypes," Virology, 205:66-74, 1994.
Ross, C.E., Dubovi, E.J., and Donis, R.O., "Herd problems of abortions and malformed calves attributed to bovine viral diarrhea," J. Am. Vet. Med. Assoc., 188(6):618-619, 1986.
Sambrook et al., Molecular cloning: a laboratory manual, 2nd Edition, Cold Spring Harbor Laboratory, Cold Spring Harbor, NY, USA, 1989.
Sambrook and Russell, Molecular cloning: a laboratory manual, 3rd Edition, Cold Spring Harbor Laboratory, Cold Spring Harbor, NY, USA, 2001.
Schalk, J.A.C., de Vries, C.G.J.C.A., and Jongen, P.M.J.M., "Potency estimation of measles, mumps and rubella trivalent vaccines with quantitative PCR infectivity assay," Biologicals, 33(2):71-79, 2005.
Schalk, J.A.C., Elzen ,C.V.D., Ovelgonne, H, et al., "Estimation of the number of infectious measles viruses in live virus vaccines using quantitative real-time PCR," J. Virol. Meth., 117:179-187, 2004.
Schefers, J., Munoz-Zanzi, C., Collins, J.E., Goyal, S.M., and Ames, T.R., "Serological evaluation of precolostral serum samples to detect Bovine viral diarrhea virus infections in large commercial dairy herds," J. Vet. Diagn. Invest., 20:625-628, 2008.
Tanner, J.E., and A. P. Morgan, "Design and analysis of veterinary vaccine efficacy trials," Vet. Microbiol., 37(3-4):221-230, 1993.
Tautz, N., Elbers, K., Stoll, D., Meyers, G., and Thiel, H.J., "Serine protease of pestiviruses: determination of cleavage sites," J. Virol., 71(7):5415-5422, 1997.
Thiel et al., "The pestiviruses," In Virology, Fields et al., (eds.) (Lippincott-Raven, Philadelphia, PA, USA), pp.1059-1073, 1996.
Vilcek, S, Paton, D.J., Durkovic, B, et al. "Bovine viral diarrhea virus genotype 1 can be separated into at least eleven genetic groups." Arch. Virol., 146:99-115, 2001.
Wang, F, Puddy, A.C., Mathis, B.C., et al., "Using QPCR to assign infectious potencies to adenovirus based vaccines and vectors for gene therapy: toward a universal method for the facile quantitation of virus and vector potency," Vaccine, 23:4500-4508, 2005.
Wiskerchen, M., and M.S. Collett, "Pestivirus gene expression: protein p80 of bovine viral diarrhea virus is a proteinase involved in polyprotein processing," Virology, 184(1):341-350, 1991.
Wren, G., "New Thinking on BRSV: Research into BRSV and vaccines reveals new information about how the virus behaves and how it may interact with killed vaccines," Bovine Veterinarian, (February) pp. 16-19, 2001.
Xu, J., E. Mendez, P.R. Caron, C. Lin, M.A. Murcko, M.S. Collett, and C.M. Rice, "Bovine viral diarrhea virus NS3 serine proteinase: polyprotein cleavage sites, cofactor requirements, and molecular model of an enzyme essential for pestivirus replication," J. Virol., 71(7):5312-5322, 1997.
Xue, W., D. Mattick, L. Smith, J. Umbaugh, and E. Trigo, "Vaccination with a modified-live bovine viral diarrhea virus (BVDV) type 1a vaccine completely protected calves against challenge with BVDV type 1 b strains," , Vaccine, 29(1):70-76, Dec. 10, 2010, [epub ahead of print Oct. 27, 2010].

## Claims

1. An immunogenic composition comprising: a modified, live, attenuated bovine viral diarrhea virus type 1b (BVDV1b), wherein the modified, live BVDV1b is a strain deposited under the ATCC accession number PTA-11553.

2. The immunogenic composition of claim 1, further comprising an immunologically effective amount of at least one additional antigen.

3. The immunogenic composition of claim 2, wherein the at least one additional antigen is specific for an organism selected from the group consisting of *Actinobacillus, Actinomyces, Bacillus, Bordetella, Brachyspira, Campylobacter, Clostridium, Ehrlichia, Erysipelothrix, Escherichia, Histophilus, Leptospira, Listeria, Mannheimia, Moraxella, Mycobacterium, Mycoplasma, Pasteurella, Salmonella,* and *Streptococcus,* or a combination thereof

4. The immunogenic composition of claim 2, wherein the at least one additional antigen is inactivated.

5. The immunogenic composition of claim 1, further comprising one or more of:
a modified, live parainfluenza virus Type 3 (PI₃),
a modified, live bovine respiratory syncytial virus (BRSV),
a modified, live bovine herpes virus (BHV-1),
a modified, live bovine viral diarrhea virus Type 1a (BVDV1a), or
a modified, live bovine viral diarrhea virus Type 2 (BVDV2).

6. A vaccine comprising a prophylactically-effective amount of the immunogenic composition of any one of claims 1 to 5, and an adjuvant.

7. The vaccine of claim 6, further comprising an immunologically-effective amount of a least one microbial pathogen-specific antigen.

8. A prophylactically-effective amount of a composition according to any one of claims 1 to 7 for use in preventing shipping fever, bovine respiratory disease complex or bovine viral diarrhea, in an animal.

9. The composition for use according to claim 8, wherein the animal is infected by, or susceptible to infection from, a pestivirus.

10. The composition for use according to claim 8 or claim 9, wherein the animal is a bovid.

11. A vaccine comprising the immunogenic composition Claim 1, and an adjuvant; wherein the immunogenic composition comprises at least 2x10³ TCID₅₀ of the modified, live BVDV1b per dose.

12. The vaccine of claim 11, further comprising one or more of:
at least 5x10³ TCID₅₀ of a modified, live BHV-1 per dose;
at least 5x10³ TCID₅₀ of a modified, live BVDV1a per dose;
at least 3x10³ TCID₅₀ of a modified, live BVDV2 per dose;
at least 6x10⁴ TCID₅₀ of a modified, live PI₃ per dose; and
at least 7x10² TCID₅₀ of a modified, live BRSV per dose.

## Patentansprüche

1. Immunogene Zusammensetzung, umfassend: einen modifizierten lebenden abgeschwächten bovinen Virusdiarrhoe-Virus vom Typ 1b (BVDV1b), wobei der modifizierte lebende BVDF1b ein unter der ATCC-Zugangsnummer PTA-11553 hinterlegter Stamm ist.

2. Immunogene Zusammensetzung nach Anspruch 1, weiter umfassend eine immunologisch wirksame Menge mindestens eines zusätzlichen Antigens.

3. Immunogene Zusammensetzung nach Anspruch 2, wobei das wenigstens eine zusätzliche Antigen für einen Organismus spezifisch ist, welcher ausgewählt ist aus der Gruppe, bestehend aus *Actinobacillus, Actinomyces, Bacillus, Bordetella, Brachyspira, Campylobacter, Clostridium, Ehrlichia, Erysipelothrix, Escherichia, Histophilus, Leptospira, Listeria, Mannheimia, Moraxella, Mycobacterium, Mycoplasma, Pasteurella, Salmonella* und *Streptococcus,* oder eine Kombination davon.

4. Immunogene Zusammensetzung nach Anspruch 2, wobei das wenigstens eine zusätzliche Antigen inaktiviert ist.

5. Immunogene Zusammensetzung nach Anspruch 1, weiter umfassend eines oder mehrere von:
einem modifizierten lebenden Parainfluenza-Virus vom Typ 3 (PI3),
einem modifizierten lebenden bovinen respiratorischen Syncytialvirus (BRSV),
einem modifizierten lebenden bovinen Herpes-Virus (BV-1),
einem modifizierten lebenden bovinen Virusdiarrhoe-Virus vom Typ 1a (BVDV1a) oder
einem modifizierten lebende bovinen Virusdiarrhoe-Virus vom Typ 2 (BVDV2).

6. Impfstoff, umfassend eine prophylaktisch wirksame Menge der immunogenen Zusammensetzung nach einem der Ansprüche 1 bis 5 und einen Hilfsstoff.

7. Impfstoff nach Anspruch 6, weiter umfassend eine immunologisch wirksame Menge wenigstens eines mikrobiellen pathogen-spezifischen Antigens.

8. Eine prophylaktisch wirksame Menge einer Zusammensetzung nach einem der Ansprüche 1 bis 7 zur Verwendung bei der Vermeidung von Transportfieber, Rinder-Atemwegserkrankungskomplex oder boviner Virusdiarrhoe in einem Tier.

9. Zusammensetzung zur Verwendung nach Anspruch 8, wobei das Tier durch einen Pestivirus infiziert ist oder für eine derartige Infizierung anfällig ist.

10. Zusammensetzung zur Verwendung nach Anspruch 8 oder Anspruch 9, wobei das Tier ein Bovide ist.

11. Impfstoff, umfassend die immunogene Zusammensetzung nach Anspruch 1 und einen Hilfsstoff, wobei die immunogene Zusammensetzung wenigstens 2x10³ TCID50 des modifizierten lebenden BVDV1b pro Dosis umfasst.

12. Impfstoff nach Anspruch 11, weiter umfassend eines oder mehrere von:
wenigstens 5x10³ TCID₅₀ eines modifizierten lebenden BHV-1 pro Dosis;
wenigstens 5x10³ TCID₅₀ eines modifizierten lebenden BHDV1a pro Dosis;
wenigstens 3x10³ TCID₅₀ eines modifizierten lebenden BHDV2 pro Dosis;
wenigstens 6x10⁴ TCID₅₀ eines modifizierten lebenden PI₃ pro Dosis;
wenigstens 7x10² TCID₅₀ eines modifizierten lebenden BRSV pro Dosis.

## Revendications

1. Composition immunogène comprenant : un virus de la diarrhée virale bovine de type 1b (BVDV1b), atténué, vivant, modifié, dans laquelle le BVDV1b vivant modifié est une souche déposée auprès de l'ATCC sous le numéro d'accès PTA-11553.

2. Composition immunogène selon la revendication 1, comprenant en outre une quantité efficace sur le plan immunologique d'au moins un antigène supplémentaire.

3. Composition immunogène selon la revendication 2, dans laquelle l'au moins un antigène supplémentaire est spécifique d'un organisme sélectionné dans le groupe constitué par *Actinobacillus, Actinomyces, Bacillus, Bordetella, Brachyspira, Campylobacter, Clostridium, Ehrlichia, Erysipelothrix, Escherichia, Histophilus, Leptospira, Listeria, Mannheimia, Moraxella, Mycobacterium, Mycoplasma, Pasteurella, Salmonella,* et *Streptococcus,* ou une combinaison de ceux-ci.

4. Composition immunogène selon la revendication 2, dans laquelle l'au moins un antigène supplémentaire est inactivé.

5. Composition immunogène selon la revendication 1, comprenant en outre un ou plusieurs éléments parmi : un virus para-influenza de type 3 (PI₃) vivant, modifié,
un virus respiratoire syncytial bovin (BRSV) vivant, modifié,
un virus de l'herpès bovin (BHV-1) vivant, modifié,
un virus de la diarrhée virale bovine de type 1a (BVDV1a) vivant, modifié, ou
un virus de la diarrhée virale bovine de type 2 (BVDV2) vivant, modifié.

6. Vaccin comprenant une quantité efficace sur le plan prophylactique de la composition immunogène selon l'une quelconque des revendications 1 à 5, et un adjuvant.

7. Vaccin selon la revendication 6, comprenant en outre une quantité efficace sur le plan immunologique d'au moins un antigène spécifique d'un pathogène microbien.

8. Quantité efficace sur le plan prophylactique d'une composition selon l'une quelconque des revendications 1 à 7 destinée à être utilisée dans la prévention de la fièvre des transports, du complexe de maladies respiratoires bovines ou de la diarrhée virale bovine, chez un animal.

9. Composition destinée à être utilisée selon la revendication 8, dans laquelle l'animal est infecté par, ou est prédisposé à l'infection par, un pestivirus.

10. Composition destinée à être utilisée selon la revendication 8 ou la revendication 9, dans laquelle l'animal est un bovidé.

11. Vaccin comprenant la composition immunogène selon la revendication 1, et un adjuvant ; dans lequel la composition immunogène comprend au moins 2 x 10³ TCID₅₀ du BVDV1b vivant, modifié, par dose.

12. Vaccin selon la revendication 11, comprenant en outre un ou plusieurs éléments parmi :
au moins 5 x 10³ TCID₅₀ d'un BHV-1 vivant, modifié, par dose ;
au moins 5 x 10³ TCID₅₀ d'un BVDV1a vivant, modifié, par dose ;
au moins 3 x 10³ TCID₅₀ d'un BVDV2 vivant, modifié, par dose ;
au moins 6 x 10⁴ TCID₅₀ d'un PI₃ vivant, modifié, par dose ; et
au moins 7 x 10² TCID₅₀ d'un BRSV vivant, modifié par dose.


### SEQUENCE LISTING

<110> Eli Lilly and Company
   Weise, Dale W
   Harris, James R
<120> BOVINE VIRAL DIARRHEA VIRUS TYPE 1B VACCINE COMPOSITIONS AND METHODS
<130> X19557
<150> 61/427361
   <151> 2010-12-27
<160> 21
<170> PatentIn version 3.5
<210> 1
   <211> 25
   <212> DNA
   <213> Artificial Sequence
<220>
   <223> synthetic construct - primer
<400> 1
   caccctatca ggctgtattc atagc 25
<210> 2
   <211> 21
   <212> DNA
   <213> Artificial Sequence
<220>
   <223> synthetic construct - primer
<400> 2
   tgcccacagc acatcttaac c 21
<210> 3
   <211> 15
   <212> DNA
   <213> Artificial Sequence
<220>
   <223> synthetic construct - primer
<400> 3
   tcacctggac gaccc 15
<210> 4
   <211> 25
   <212> DNA
   <213> Artificial Sequence
<220>
   <223> synthetic construct - primer
<400> 4
   gcaatgctgc aggactaggt ataat 25
<210> 5
   <211> 25
   <212> DNA
   <213> Artificial Sequence
<220>
   <223> synthetic construct - primer
<400> 5
   acactgtaat tgatgacccc attct 25
<210> 6
   <211> 22
   <212> DNA
   <213> Artificial Sequence
<220>
   <223> synthetic construct - primer
<400> 6
   aagacttgta tgatgctgcc aa 22
<210> 7
   <211> 19
   <212> DNA
   <213> Artificial Sequence
<220>
   <223> synthetic construct - primer
<400> 7
   ggtcgcccag gtaaaagca 19
<210> 8
   <211> 20
   <212> DNA
   <213> Artificial Sequence
<220>
   <223> synthetic construct - primer
<400> 8
   gcctctgcag caccctatca 20
<210> 9
   <211> 19
   <212> DNA
   <213> Artificial Sequence
<220>
   <223> synthetic construct - primer
<400> 9
   aaccgactgt tacgaatac 19
<210> 10
   <211> 23
   <212> DNA
   <213> Artificial Sequence
<220>
   <223> synthetic construct - primer
<400> 10
   gctagccatg cccttagtag gac 23
<210> 11
   <211> 21
   <212> DNA
   <213> Artificial Sequence
<220>
   <223> synthetic construct - primer
<400> 11
   gacgagtccc ctgtactcag g 21
<210> 12
   <211> 19
   <212> DNA
   <213> Artificial Sequence
<220>
   <223> synthetic construct - primer
<400> 12
   cagtgagtcc attggatgg 19
<210> 13
   <211> 22
   <212> DNA
   <213> Artificial Sequence
<220>
   <223> synthetic construct - primer
<400> 13
   ggagaccaag accaaggaga tg 22
<210> 14
   <211> 19
   <212> DNA
   <213> Artificial Sequence
<220>
   <223> synthetic construct - primer
<400> 14
   cgtctgccca tgcataagg 19
<210> 15
   <211> 17
   <212> DNA
   <213> Artificial Sequence
<220>
   <223> synthetic construct - primer
<400> 15
   acctcggtca tccatag 17
<210> 16
   <211> 21
   <212> DNA
   <213> Artificial Sequence
<220>
   <223> synthetic construct - primer
<400> 16
   ccatgttagc gctctggaac c 21
<210> 17
   <211> 22
   <212> DNA
   <213> Artificial Sequence
<220>
   <223> synthetic construct - primer
<400> 17
   cgtctttacg gtcgacgact cc 22
<210> 18
   <211> 15
   <212> DNA
   <213> Artificial Sequence
<220>
   <223> synthetic construct - primer
<400> 18
   acggacgtgc gcgaa 15
<210> 19
   <211> 20
   <212> DNA
   <213> Artificial Sequence
<220>
   <223> synthetic construct - primer
<400> 19
   gtcgtccagg tgaaaacggt 20
<210> 20
   <211> 20
   <212> DNA
   <213> Artificial Sequence
<220>
   <223> synthetic construct - primer
<400> 20
   gtcgtccagg tgaaaacggt 20
<210> 21
   <211> 20
   <212> DNA
   <213> Artificial Sequence
<220>
   <223> synthetic construct - primer
<400> 21
   gtcgtccagg tgaaaacggt 20